# FASCICULE DE BREVET EUROPEEN

(11) **EP 1 798 242 B1**
(45) Date de publication et mention de la délivrance du brevet: **23.01.2013**
(21) Numéro de dépôt: 06126429.7
(22) Date de dépôt: 20.01.2003
(51) Int. Cl.: C07K 16/28, A61K 39/395, G01N 33/68, A61P 35/00, A61P 17/06, A01K 67/027, C12N 15/00, C12N 15/20, C12N 5/12

(54) **Anticorps anti-IGF-IR et leurs applications**
Anti-IGF-IF-Antikörper und ihre Anwendungen
Anti-IGF-IR antibodies and their applications

(30) Priorité: 18.01.2002 FR 0200654; 18.01.2002 FR 0200653; 07.05.2002 FR 0205753
(43) Date de publication de la demande: 20.06.2007
(62) Demande divisionnaire de: 03712270.2
(73) Titulaire: PIERRE FABRE MEDICAMENT, 92100 Boulogne-Billancourt (FR)
(72) Inventeur: Goetsch, Liliane, 74130 Ayse (FR); Corvaia, Nathalie, 74160 Collonges sous Salève (FR); Leger, Olivier, 74800 Saint Sixt (FR)
(74) Mandataire: Regimbeau

(56) Documents cités:
- WO-A-01/88138
- WO-A-02/053596
- WRAIGHT C J ET AL: "Reversal of epidermal hyperproliferation in psoriasis by insulin-like growth factor I receptor antisense oligonucleotides." NATURE BIOTECHNOLOGY MAY 2000 LNKD- PUBMED:10802619, vol. 18, no. 5, mai 2000 (2000-05), pages 521-526, XP001172823 ISSN: 1087-0156
- LI SHU-LIAN ET AL: "Single-chain antibodies against human insulin-like growth factor I receptor: Expression, purification, and effect on tumor growth." CANCER IMMUNOLOGY IMMUNOTHERAPY, vol. 49, no. 4-5, juillet 2000 (2000-07), pages 243-252, XP001113064 ISSN: 0340-7004
- BARTUCCI MONICA ET AL: "Differential insulin-like growth factor I receptor signaling and function in estrogen receptor (ER)-positive MCF-7 and ER-negative MDA-MB-231 breast cancer cells." CANCER RESEARCH, vol. 61, no. 18, 15 septembre 2001 (2001-09-15), pages 6747-6754, XP002218289 ISSN: 0008-5472
- IIHARA K ET AL: "Growth-regulatory mechanism of two human esophageal cancer cell lines in protein-free conditions." INTERNATIONAL JOURNAL OF CANCER, vol. 55, no. 3, 1993, pages 364-370, XP008009313 ISSN: 0020-7136

### LISTE DE SEQUENCES

<110> Pierre Fabre Médicament
<120> Nouveaux anticorps anti-IGF-IR et leurs applications
<130> D19919
<140> EP 06126429.7
   <141> 2006-12-18
<150> FR 02/00 653
   <151> 2002-01-18
<150> FR 02/00 654
   <151> 2002-01-18
<150> FR 02/05 753
   <151> 2002-05-07
<150> EP 03712270.2
   <151> 2003-01-20
<160> 156
<170> PatentIn Ver. 2.1
<210> 1
   <211> 48
   <212> ADN
   <213> Mus musculus
<220>
   <221> CDS
   <222> (1)..(48)
<400> 1
   aga tct agt cag agc att gta cat agt aat gga aac acc tat tta caa    48
   Arg Ser Ser Gln Ser Ile Val His Ser Asn Gly Asn Thr Tyr Leu Gln
     1               5                  10                  15
<210> 2
   <211> 16
   <212> PRT
   <213> Mus musculus
<400> 2
   Arg Ser Ser Gln Ser Ile Val His Ser Asn Gly Asn Thr Tyr Leu Gln
     1               5                  10                  15
<210> 3
   <211> 21
   <212> ADN
   <213> Mus musculus
<220>
   <221> CDS
   <222> (1)..(21)
<400> 3
   aaa gtt tcc aac cga ctt tat                                         21
   Lys Val Ser Asn Arg Leu Tyr
     1               5
<210> 4
   <211> 7
   <212> PRT
   <213> Mus musculus
<400> 4
   Lys Val Ser Asn Arg Leu Tyr
     1               5
<210> 5
   <211> 27
   <212> ADN
   <213> Mus musculus
<220>
   <221> CDS
   <222> (1)..(27)
<400> 5
   ttt caa ggt tca cat gtt ccg tgg acg                               27
   Phe Gln Gly Ser His Val Pro Trp Thr
     1               5
<210> 6
   <211> 9
   <212> PRT
   <213> Mus musculus
<400> 6
   Phe Gln Gly Ser His Val Pro Trp Thr
     1               5
<210> 7
   <211> 18
   <212> ADN
   <213> Mus musculus
<220>
   <221> CDS
   <222> (1)..(18)
<400> 7
   ggt ggt tat tta tgg aac                                           18
   Gly Gly Tyr Leu Trp Asn
     1               5
<210> 8
   <211> 6
   <212> PRT
   <213> Mus musculus
<400> 8
   Gly Gly Tyr Leu Trp Asn
     1               5
<210> 9
   <211> 48
   <212> ADN
   <213> Mus musculus
<220>
   <221> CDS
   <222> (1)..(48)
<400> 9
   tac ata agc tac gac ggt acc aat aac tac aaa cca tct ctc aaa gat    48
   Tyr Ile Ser Tyr Asp Gly Thr Asn Asn Tyr Lys Pro Ser Leu Lys Asp
    1               5                  10                  15
<210> 10
   <211> 16
   <212> PRT
   <213> Mus musculus
<400> 10
   Tyr Ile Ser Tyr Asp Gly Thr Asn Asn Tyr Lys Pro Ser Leu Lys Asp
    1               5                  10                  15
<210> 11
   <211> 24
   <212> ADN
   <213> Mus musculus
<220>
   <221> CDS
   <222> (1)..(24)
<400> 11
   tac ggt agg gtc ttc ttt gac tac                                    24
   Tyr Gly Arg Val Phe Phe Asp Tyr
    1               5
<210> 12
   <211> 8
   <212> PRT
   <213> Mus musculus
<400> 12
   Tyr Gly Arg Val Phe Phe Asp Tyr
    1               5
<210> 13
   <211> 26
   <212> ADN
   <213> Mus musculus
<400> 13
   atgaaatgca gctgggtcat sttctt 26
<210> 14
   <211> 26
   <212> ADN
   <213> Mus musculus
<400> 14
   atgggatgga gctrtatcat sytctt 26
<210> 15 v
   <211> 26
   <212> ADN
   <213> Mus musculus
<400> 15
   atgaagwtgt ggttaaactg ggtttt 26
<210> 16
   <211> 23
   <212> ADN
   <213> Mus musculus
<400> 16
   atgractttg ggytcagctt grt 23
<210> 17
   <211> 26 v
   <212> ADN
   <213> Musv musculus
<400> 17
   atggactcca ggctcaattt agtttt 26
<210> 18
   <211> 26
   <212> ADN
   <213> Mus musculus
<400> 18
   atggctgtcy trgsgctrct cttctg 26
<210> 19
   <211> 26
   <212> ADN
   <213> Mus musculus
<400> 19
   atggratgga gckggrtctt tmtctt 26
<210> 20
   <211> 23
   <212> ADN
   <213> Mus musculus
<400> 20
   atgagagtgc tgattctttt gtg 23
<210> 21
   <211> 26
   <212> ADN
   <213> Mus musculus
<400> 21
   atggmttggg tgtggamctt gctatt 26
<210> 22
   <211> 26
   <212> ADN
   <213> Mus musculus
<400> 22
   atgggcagac ttacattctc attcct 26
<210> 23
   <211> 28
   <212> ADN
   <213> Mus musculus
<400> 23
   atggattttg ggctgatttt ttttattg 28
<210> 24
   <211> 26
   <212> ADN
   <213> Mus musculus
<400> 24
   atgatggtgt taagtcttct gtacct 26
<210> 25
   <211> 29
   <212> ADN
   <213> Mus musculus
<400> 25
   atgaagttgc ctgttaggct gttggtgct 29
<210> 26
   <211> 29
   <212> ADN
   <213> Mus musculus
<400> 26
   atggagwcag acacactcct gytatgggt 29
<210> 27
   <211> 23
   <212> ADN
   <213> Mus musculus
<400> 27
   atgagtgtgc tcactcaggt cct 23
<210> 28
   <211> 26
   <212> ADN
   <213> Mus musculus
<400> 28
   atgaggrccc ctgctcagwt tyttgg 26
<210> 29
   <211> 29
   <212> ADN
   <213> Mus musculus
<400> 29
   atggatttwc aggtgcagat twtcagctt 29
<210> 30
   <211> 29
   <212> ADN
   <213> Mus musculus
<400> 30
   atggatttwc argtgcagat twtcagctt 29
<210> 31
   <211> 26
   <212> ADN
   <213> Mus musculus
<400> 31
   atgaggtkcy ytgytsagyt yctgrg 26
<210> 32
   <211> 23
   <212> ADN
   <213> Mus musculus
<400> 32
   atgggcwtca agatggagtc aca 23
<210> 33
   <211> 29
   <212> ADN
   <213> Mus musculus
<400> 33
   atgtggggay ctktttycmm tttttcaat 29
<210> 34
   <211> 24
   <212> ADN
   <213> Mus musculus
<400> 34
   atggtrtccw casctcagtt cctt 24
<210> 35
   <211> 26
   <212> ADN
   <213> Mus musculus
<400> 35
   atgtatatat gtttgttgtc tatttc 26
<210> 36
   <211> 26
   <212> ADN
   <213> Mus musculus
<400> 36
   atggaagccc cagctcagct tctctt 26
<210> 37
   <211> 26
   <212> ADN
   <213> Mus musculus
<400> 37
   atgragtywc agacccaggt cttyrt 26
<210> 38
   <211> 26
   <212> ADN
   <213> Mus musculus
<400> 38
   atggagacac attctcaggt ctttgt 26
<210> 39
   <211> 26
   <212> ADN
   <213> Mus musculus
<400> 39
   atggattcac aggcccaggt tcttat 26
<210> 40
   <211> 20
   <212> ADN
   <213> Mus musculus
<400> 40
   actggatggt gggaagatgg 20
<210> 41
   <211> 42
   <212> ADN
   <213> Mus musculus
<220>
   <221> CDS
   <222> (1)..(42)
<400> 41
   gct gat gct gca cca act gta tcc atc ttc cca cca tcc agt        42
   Ala Asp Ala Ala Pro Thr Val Ser Ile Phe Pro Pro Ser Ser
     1               5                  10
<210> 42
   <211> 14
   <212> PRT
   <213> Mus musculus
<400> 42
   Ala Asp Ala Ala Pro Thr Val Ser Ile Phe Pro Pro Ser Ser
     1               5                  10
<210> 43
   <211> 20
   <212> ADN
   <213> Mus musculus
<400> 43
   ccatcttccc accatccagt 20
<210> 44
   <211> 18
   <212> ADN
   <213> Mus musculus
<400> 44
   ccagtggata gacagatg 18
<210> 45
   <211> 33
   <212> ADN
   <213> Mus musculus
<220>
   <221> CDS
   <222> (1)..(33)
<400> 45
   gcc aaa acg aca ccc cca tct gtc tat cca ctg          33
   Ala Lys Thr Thr Pro Pro Ser Val Tyr Pro Leu
   1               5                   10
<210> 46
   <211> 11
   <212> PRT
   <213> Mus musculus
<400> 46
   Ala Lys Thr Thr Pro Pro Ser Val Tyr Pro Leu
   1               5                   10
<210> 47
   <211> 21
   <212> ADN
   <213> Mus musculus
<400> 47 21
   cccccatctg tctatccact g 21
<210> 48
   <211> 438
   <212> ADN
   <213> Mus musculus
<220>
   <221> CDS
   <222> (28)..(393)
<400> 48
   atgaagttgc ctgttaggct gttggtg ctg atg ttc tgg att cct gct tcc aga 54
                                 Leu Met Phe Trp Ile Pro Ala Ser Arg
                                  1               5
   agt gat gtt ttg atg acc caa att cca ctc tcc ctg cct gtc agt ctt   102
   Ser Asp Val Leu Met Thr Gln Ile Pro Leu Ser Leu Pro Val Ser Leu
    10                  15                  20                  25
   gga gat caa gcc tcc atc tct tgc aga tct agt cag agc att gta cat   150
   Gly Asp Gln Ala Ser Ile Ser Cys Arg Ser Ser Gln Ser Ile Val His
                   30                  35                  40
   agt aat gga aac acc tat tta caa tgg tac ctg cag aaa cca ggt cag   198
   Ser Asn Gly Asn Thr Tyr Leu Gln Trp Tyr Leu Gln Lys Pro Gly Gln
               45                  50                  55
   tct cca aag ctc ctg atc tac aaa gtt tcc aac cga ctt tat ggg gtc   246
   Ser Pro Lys Leu Leu Ile Tyr Lys Val Ser Asn Arg Leu Tyr Gly Val
           60                  65                  70
   cca gac agg ttc agt ggc agt gga tca ggg aca gat ttc aca ctc aag   294
   Pro Asp Arg Phe Ser Gly Ser Gly Ser Gly Thr Asp Phe Thr Leu Lys
       75                  80                  85
   atc agc agc gtg gag gct gag gat ctg gga gtt tat tac tgc ttt caa   342
   Ile Ser Ser Val Glu Ala Glu Asp Leu Gly Val Tyr Tyr Cys Phe Gln
    90                  95                 100                 105
   ggt tca cat gtt ccg tgg acg ttc ggt gga ggc acc aag ctg gaa atc   390
   Gly Ser His Val Pro Trp Thr Phe Gly Gly Gly Thr Lys Leu Glu Ile
                   110                 115                 120
   aaa cgg gctgatgctg caccaactgt atccatcttc ccaccatcca gt            438
   Lys
<210> 49
   <211> 122
   <212> PRT
   <213> Mus musculus
<400> 49
   Leu Met Phe Trp Ile Pro Ala Ser Arg Ser Asp Val Leu Met Thr Gln
    1               5                  10                  15
   Ile Pro Leu Ser Leu Pro Val Ser Leu Gly Asp Gln Ala Ser Ile Ser
               20                  25                  30
   Cys Arg Ser Ser Gln Ser Ile Val His Ser Asn Gly Asn Thr Tyr Leu
           35                  40                  45
   Gln Trp Tyr Leu Gln Lys Pro Gly Gln Ser Pro Lys Leu Leu Ile Tyr
       50                  55                  60
   Lys Val Ser Asn Arg Leu Tyr Gly Val Pro Asp Arg Phe Ser Gly Ser
   65                  70                  75                  80
   Gly Ser Gly Thr Asp Phe Thr Leu Lys Ile Ser Ser Val Glu Ala Glu
                   85                  90                  95
   Asp Leu Gly Val Tyr Tyr Cys Phe Gln Gly Ser His Val Pro Trp Thr
               100                 105                 110
   Phe Gly Gly Gly Thr Lys Leu Glu Ile Lys
           115                 120
<210> 50
   <211> 438
   <212> ADN
   <213> Mus musculus
<400> 50
   tacttcaacg gacaatccga caaccacgac tacaagacct aaggacgaag gtcttcacta 60
   caaaactact gggtttaagg tgagagggac ggacagtcag aacctctagt tcggaggtag 120
   agaacgtcta gatcagtctc gtaacatgta tcattacctt tgtggataaa tgttaccatg 180
   gacgtctttg gtccagtcag aggtttcgag gactagatgt ttcaaaggtt ggctgaaata 240
   ccccagggtc tgtccaagtc accgtcacct agtccctgtc taaagtgtga gttctagtcg 300
   tcgcacctcc gactcctaga ccctcaaata atgacgaaag ttccaagtgt acaaggcacc 360
   tgcaagccac ctccgtggtt cgacctttag tttgcccgac tacgacgtgg ttgacatagg 420
   tagaagggtg gtaggtca                                               438
<210> 51
   <211> 438
   <212> ADN
   <213> Mus musculus
<220>
   <221> CDS
   <222> (25)..(405)
<400> 51
   atgatggtgt taagtcttct gtac ctc ttg aca gcc att cct ggt atc ctg    51
                             Leu Leu Thr Ala Ile Pro Gly Ile Leu
                              1               5
   tct gat gta cag ctt cag gag tca gga cct ggc ctc gtg aaa cct tct   99
   Ser Asp Val Gln Leu Gln Glu Ser Gly Pro Gly Leu Val Lys Pro Ser
    10                  15                  20                  25
   cag tct ctg tct ctc acc tgc tct gtc acc ggc tac tcc atc acc ggt   147
   Gln Ser Leu Ser Leu Thr Cys Ser Val Thr Gly Tyr Ser Ile Thr Gly
                    30                  35                  40
   ggt tat tta tgg aac tgg atc cgg cag ttt cca gga aac aaa ctg gag   195
   Gly Tyr Leu Trp Asn Trp Ile Arg Gln Phe Pro Gly Asn Lys Leu Glu
                45                  50                  55
   tgg atg ggc tac ata agc tac gac ggt acc aat aac tac aaa cca tct   243
   Trp Met Gly Tyr Ile Ser Tyr Asp Gly Thr Asn Asn Tyr Lys Pro Ser
            60                  65                  70
   ctc aaa gat cga atc tcc atc act cgt gac aca tct aag aac cag ttt   291
   Leu Lys Asp Arg Ile Ser Ile Thr Arg Asp Thr Ser Lys Asn Gln Phe
        75                  80                  85
   ttc ctg aag ttg aat tct gtg act aat gaa gac aca gct aca tat tac   339
   Phe Leu Lys Leu Asn Ser Val Thr Asn Glu Asp Thr Ala Thr Tyr Tyr
    90                  95                 100                 105
   tgt gca aga tac ggt agg gtc ttc ttt gac tac tgg ggc caa ggc acc   387
   Cys Ala Arg Tyr Gly Arg Val Phe Phe Asp Tyr Trp Gly Gln Gly Thr
                   110                 115                 120
   act ctc aca gtc tcc tca gccaaaacga cacccccatc tgtctatcca ctg      438
   Thr Leu Thr Val Ser Ser
               125
<210> 52
   <211> 127
   <212> PRT
   <213> Mus musculus
<400> 52
   Leu Leu Thr Ala Ile Pro Gly Ile Leu Ser Asp Val Gln Leu Gln Glu
   1               5                   10                  15
   Ser Gly Pro Gly Leu Val Lys Pro Ser Gln Ser Leu Ser Leu Thr Cys
               20                  25                  30
   Ser Val Thr Gly Tyr Ser Ile Thr Gly Gly Tyr Leu Trp Asn Trp Ile
           35                  40                  45
   Arg Gln Phe Pro Gly Asn Lys Leu Glu Trp Met Gly Tyr Ile Ser Tyr
       50                  55                  60
   Asp Gly Thr Asn Asn Tyr Lys Pro Ser Leu Lys Asp Arg Ile Ser Ile
   65                  70                  75                  80
   Thr Arg Asp Thr Ser Lys Asn Gln Phe Phe Leu Lys Leu Asn Ser Val
                   85                  90                  95
   Thr Asn Glu Asp Thr Ala Thr Tyr Tyr Cys Ala Arg Tyr Gly Arg Val
               100                 105                 110
   Phe Phe Asp Tyr Trp Gly Gln Gly Thr Thr Leu Thr Val Ser Ser
           115                 120                 125
<210> 53
   <211> 438
   <212> ADN
   <213> Mus musculus
<400> 53
   tactaccaca attcagaaga catggacaac tgtcggtaag gaccatagga cagactacat 60
   gtcgaagtcc tcagtcctgg accggagcac tttggaagag tcagagacag agagtggacg 120
   agacagtggc cgatgaggta gtggccacca ataaatacct tgacctaggc cgtcaaaggt 180
   cctttgtttg acctcaccta cccgatgtat tcgatgctgc catggttatt gatgtttggt 240
   agagagtttc tagcttagag gtagtgagca ctgtgtagat tcttggtcaa aaaggacttc 300
   aacttaagac actgattact tctgtgtcga tgtataatga cacgttctat gccatcccag 360
   aagaaactga tgaccccggt tccgtggtga gagtgtcaga ggagtcggtt ttgctgtggg 420
   ggtagacaga taggtgac                                                438
<210> 54
   <211> 112
   <212> PRT
   <213> Mus musculus
<400> 54
   Asp Val Leu Met Thr Gln Ile Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Gln Trp Tyr Leu Gln Lys Pro Gly Gln Ser
           35                  40                  45
   Pro Lys Leu Leu Ile Tyr Lys Val Ser Asn Arg Leu Tyr Gly Val Pro
       50                  55                  60
   Asp Arg Phe Ser Gly Ser Gly Ser Gly Thr Asp Phe Thr Leu Lys Ile
   65                  70                  75                  80
   Ser Ser Val Glu Ala Glu Asp Leu Gly Val Tyr Tyr Cys Phe Gln Gly
                   85                  90                  95
   Ser His Val Pro Trp Thr Phe Gly Gly Gly Thr Lys Leu Glu Ile Lys
               100                 105                 110
<210> 55
   <211> 112
   <212> PRT
   <213> Mus musculus
<400> 55
   Asp Val Leu Met Thr Gln Thr Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Phe Thr Phe Gly Ser Gly Thr Lys Leu Asp Ile Lys
               100                 105                 110
<210> 56
   <211> 112
   <212> PRT
   <213> Mus musculus
<400> 56
   Asp Val Leu Met Thr Gln Thr Pro Leu Ser Leu Pro Val Ser Leu Gly
    1               5                  10                  15
   Asp Gln Ala Ser Ile Ser Cys Arg Ser Ser Gln Ser Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Trp Thr Phe Gly Gly Gly Thr Lys Leu Glu Ile Lys
               100                 105                 110
<210> 57
   <211> 112
   <212> PRT
   <213> Mus musculus
<400> 57
   Asp Val Val Met Thr Gln Thr Pro Leu Ser Leu Pro Val Ser Leu Gly
    1               5                  10                  15
   Asp Gln Ala Ser Ile Ser Cys Arg Ser Ser Gln Ser Leu Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Thr His Val Pro Tyr Thr Phe Gly Gly Gly Thr Lys Leu Glu Ile Lys
               100                 105                 110
<210> 58
   <211> 112
   <212> PRT
   <213> Homo sapiens
<400> 58
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                      80
   Ser Arg Val Glu Ala Glu Asp Val Gly Val Tyr Tyr Cys Met Gln Ala
                   85                  90                  95
   Leu Gln Thr Pro Gln Thr Phe Gly Gln Gly Thr Lys Val Glu Ile Lys
               100                 105                 110
<210> 59
   <211> 100
   <212> PRT
   <213> Homo sapiens
<400> 59
   Asp Ile Val Met Thr Gln Ser Pro Leu Ser Leu Pro Val Thr Pro Gly
    1               5                  10                  15
   Glu Pro Ala Ser Ile Ser Cys Arg Ser Ser Gln Ser Leu Leu His Ser
               20                  25                  30
   Asn Gly Tyr Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                      80
   Ser Arg Val Glu Ala Glu Asp Val Gly Val Tyr Tyr Cys Met Gln Ala
                   85                  90                  95
   Leu Gln Thr Pro
               100
<210> 60
   <211> 112
   <212> PRT
   <213> Homo sapiens
<220>
   <221> VARIANT
   <222> (35)..(36)
   <223> Xaa correspond à n'importe quel acide aminé
<220>
   <221> VARIANT
   <222> (39)
   <223> Xaa correspond à n'importe quel acide aminé
<220>
   <221> VARIANT
   <222> (99)
   <223> Xaa correspond à n'importe quel acide aminé
<400> 60
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asp Gly Xaa Xaa Tyr Leu Xaa Trp Tyr Leu Gln Lys Pro Gly Gln Ser
           35                  40                  45
   Pro Gln Leu Leu Ile Tyr Leu Val Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ala
                   85                  90                  95
   Leu Gln Xaa Pro Arg Thr Phe Gly Gln Gly Thr Lys Val Glu Ile Lys
               100                 105                 110
<210> 61
   <211> 112
   <212> PRT
   <213> Homo sapiens
<400> 61
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Ile Val His Ser
               20                  25                  30
   Asn Gly Asn Thr Tyr Leu Gln Trp Tyr Leu Gln Lys Pro Gly Gln Ser
           35                  40                  45
   Pro Gln Leu Leu Ile Tyr Lys Val Ser Asn Arg Leu Tyr Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Phe Gln Gly
                   85                  90                  95
   Ser His Val Pro Trp Thr Phe Gly Gln Gly Thr Lys Val Glu Ile Lys
               100                 105                 110
<210> 62
   <211> 433
   <212> ADN
   <213> Homo sapiens
<220>
   <221> CDS
   <222> (22)..(414)
<400> 62
   gtcagaacgc gtgccgccac c atg aag ttg cct gtt agg ctg ttg gtg ctg   51
                           Met Lys Leu Pro Val Arg Leu Leu Val Leu
                           1               5                   10
   atg ttc tgg ttt cct gct tcc agc agt gat gtt gtg atg act cag tct   99
   Met Phe Trp Phe Pro Ala Ser Ser Ser Asp Val Val Met Thr Gln Ser
                   15                  20                  25
   cca ctc tcc ctg ccc gtc acc cct gga gag ccg gcc tcc atc tcc tgc   147
   Pro Leu Ser Leu Pro Val Thr Pro Gly Glu Pro Ala Ser Ile Ser Cys
               30                  35                  40
   agg tct agt cag agc att gta cat agt aat gga aac acc tat ttg caa   195
   Arg Ser Ser Gln Ser Ile Val His Ser Asn Gly Asn Thr Tyr Leu Gln
           45                  50                  55
   tgg tac ctg cag aag cca ggg cag tct cca cag ctc ctg atc tat aaa   243
   Trp Tyr Leu Gln Lys Pro Gly Gln Ser Pro Gln Leu Leu Ile Tyr Lys
       60                  65                  70
   gtt tct aat cgg ctt tat ggg gtc cct gac agg ttc agt ggc agt gga   291
   Val Ser Asn Arg Leu Tyr Gly Val Pro Asp Arg Phe Ser Gly Ser Gly
   75                  80                  85                  90
   tca ggc aca gat ttt aca ctg aaa atc agc aga gtg gag gct gag gat   339
   Ser Gly Thr Asp Phe Thr Leu Lys Ile Ser Arg Val Glu Ala Glu Asp
                   95                  100                 105
   gtt ggg gtt tat tac tgc ttt caa ggt tca cat gtt ccg tgg acg ttc   387
   Val Gly Val Tyr Tyr Cys Phe Gln Gly Ser His Val Pro Trp Thr Phe
               110                 115                 120
   ggc caa ggg acc aag gtg gaa atc aaa cgt gagtggatcc tctgcg         433
   Gly Gln Gly Thr Lys Val Glu Ile Lys
           125                 130
<210> 63
   <211> 131
   <212> PRT
   <213> Homo sapiens
<400> 63
   Met Lys Leu Pro Val Arg Leu Leu Val Leu Met Phe Trp Phe Pro Ala
   1               5                   10                  15
   Ser Ser Ser Asp Val Val Met Thr Gln Ser Pro Leu Ser Leu Pro Val
               20                  25                  30
   Thr Pro Gly Glu Pro Ala Ser Ile Ser Cys Arg Ser Ser Gln Ser Ile
           35                  40                  45
   Val His Ser Asn Gly Asn Thr Tyr Leu Gln Trp Tyr Leu Gln Lys Pro
       50                  55                  60
   Gly Gln Ser Pro Gln Leu Leu Ile Tyr Lys Val Ser Asn Arg Leu Tyr
   65                  70                  75                  80
   Gly Val Pro Asp Arg Phe Ser Gly Ser Gly Ser Gly Thr Asp Phe Thr
                   85                  90                  95
   Leu Lys Ile Ser Arg Val Glu Ala Glu Asp Val Gly Val Tyr Tyr Cys
               100                 105                 110
   Phe Gln Gly Ser His Val Pro Trp Thr Phe Gly Gln Gly Thr Lys Val
           115                 120                 125
   Glu Ile Lys
       130
<210> 64
   <211> 433
   <212> ADN
   <213> Homo sapiens
<400> 64
   cagtcttgcg cacggcggtg gtacttcaac ggacaatccg acaaccacga ctacaagacc 60
   aaaggacgaa ggtcgtcact acaacactac tgagtcagag gtgagaggga cgggcagtgg 120
   ggacctctcg gccggaggta gaggacgtcc agatcagtct cgtaacatgt atcattacct 180
   ttgtggataa acgttaccat ggacgtcttc ggtcccgtca gaggtgtcga ggactagata 240
   tttcaaagat tagccgaaat accccaggga ctgtccaagt caccgtcacc tagtccgtgt 300
   ctaaaatgtg acttttagtc gtctcacctc cgactcctac aaccccaaat aatgacgaaa 360
   gttccaagtg tacaaggcac ctgcaagccg gttccctggt tccaccttta gtttgcactc 420
   acctaggaga cgc                                                    433
<210> 65
   <211> 112
   <212> PRT
   <213> Homo sapiens
<400> 65
<210> 66
   <211> 433
   <212> ADN
   <213> Homo sapiens
<220>
   <221> CDS
   <222> (22)..(414)
<400> 66
   gtcagaacgc gtgccgccac c atg aag ttg cct gtt agg ctg ttg gtg ctg    51
                           Met Lys Leu Pro Val Arg Leu Leu Val Leu
                           1               5                   10
   atg ttc tgg ttt cct gct tcc agc agt gat att gtg atg act cag tct    99
   Met Phe Trp Phe Pro Ala Ser Ser Ser Asp Ile Val Met Thr Gln Ser
                   15                  20                  25
   cca ctc tcc ctg ccc gtc acc cct gga gag ccg gcc tcc atc tcc tgc    147
   Pro Leu Ser Leu Pro Val Thr Pro Gly Glu Pro Ala Ser Ile Ser Cys
               30                  35                  40
   agg tct agt cag agc att gta cat agt aat gga aac acc tat ttg caa    195
   Arg Ser Ser Gln Ser Ile Val His Ser Asn Gly Asn Thr Tyr Leu Gln
           45                  50                  55
   tgg tac ctg cag aag cca ggg cag tct cca cag ctc ctg atc tat aaa    243
   Trp Tyr Leu Gln Lys Pro Gly Gln Ser Pro Gln Leu Leu Ile Tyr Lys
       60                  65                  70
   gtt tct aat cgg ctt tat ggg gtc cct gac agg ttc agt ggc agt gga    291
   Val Ser Asn Arg Leu Tyr Gly Val Pro Asp Arg Phe Ser Gly Ser Gly
   75                  80                  85                  90
   tca ggc aca gat ttt aca ctg aaa atc agc aga gtg gag gct gag gat    339
   Ser Gly Thr Asp Phe Thr Leu Lys Ile Ser Arg Val Glu Ala Glu Asp
                   95                  100                 105
   gtt ggg gtt tat tac tgc ttt caa ggt tca cat gtt ccg tgg acg ttc    387
   Val Gly Val Tyr Tyr Cys Phe Gln Gly Ser His Val Pro Trp Thr Phe
               110                 115                 120
   ggc caa ggg acc aag gtg gaa atc aaa cgt gagtggatcc tctgcg          433
   Gly Gln Gly Thr Lys Val Glu Ile Lys
           125                 130
<210> 67
   <211> 131
   <212> PRT
   <213> Homo sapiens
<400> 67
   Met Lys Leu Pro Val Arg Leu Leu Val Leu Met Phe Trp Phe Pro Ala
    1               5                  10                  15
   Ser Ser Ser Asp Ile Val Met Thr Gln Ser Pro Leu Ser Leu Pro Val
               20                  25                  30
   Thr Pro Gly Glu Pro Ala Ser Ile Ser Cys Arg Ser Ser Gln Ser Ile
           35                  40                  45
   Val His Ser Asn Gly Asn Thr Tyr Leu Gln Trp Tyr Leu Gln Lys Pro
       50                  55                  60
   Gly Gln Ser Pro Gln Leu Leu Ile Tyr Lys Val Ser Asn Arg Leu Tyr
   65                  70                  75                  80
   Gly Val Pro Asp Arg Phe Ser Gly Ser Gly Ser Gly Thr Asp Phe Thr
                   85                  90                  95
   Leu Lys Ile Ser Arg Val Glu Ala Glu Asp Val Gly Val Tyr Tyr Cys
               100                 105                 110
   Phe Gln Gly Ser His Val Pro Trp Thr Phe Gly Gln Gly Thr Lys Val
           115                 120                 125
   Glu Ile Lys
       130
<210> 68
   <211> 433
   <212> ADN
   <213> Homo sapiens
<400> 68
   cagtcttgcg cacggcggtg gtacttcaac ggacaatccg acaaccacga ctacaagacc 60
   aaaggacgaa ggtcgtcact acaacactac tgagtcagag gtgagaggga cgggcagtgg 120
   ggacctctcg gccggaggta gaggacgtcc agatcagtct cgtaacatgt atcattacct 180
   ttgtggataa acgttaccat ggacgtcttc ggtcccgtca gaggtgtcga ggactagata 240
   tttcaaagat tagccgaaat accccaggga ctgtccaagt caccgtcacc tagtccgtgt 300
   ctaaaatgtg acttttagtc gtctcacctc cgactcctac aaccccaaat aatgacgaaa 360
   gttccaagtg tacaaggcac ctgcaagccg gttccctggt tccaccttta gtttgcactc 420
   acctaggaga cgc                                                    433
<210> 69
   <211> 117
   <212> PRT
   <213> Mus musculus
<400> 69
   Asp Val Gln Leu Gln Glu Ser Gly Pro Gly Leu Val Lys Pro Ser Gln
   1 5 10 15
   Ser Leu Ser Leu Thr Cys Ser Val Thr Gly Tyr Ser Ile Thr Gly Gly
   20 25 30
   Tyr Leu Trp Asn Trp Ile Arg Gln Phe Pro Gly Asn Lys Leu Glu Trp
   35 40 45
   Met Gly Tyr Ile Ser Tyr Asp Gly Thr Asn Asn Tyr Lys Pro Ser Leu
   50 55 60
   Lys Asp Arg Ile Ser Ile Thr Arg Asp Thr Ser Lys Asn Gln Phe Phe
   65 70 75 80
   Leu Lys Leu Asn Ser Val Thr Asn Glu Asp Thr Ala Thr Tyr Tyr Cys
   85 90 95
   Ala Arg Tyr Gly Arg Val Phe Phe Asp Tyr Trp Gly Gln Gly Thr Thr
   100 105 110
   Leu Thr Val Ser Ser
   115
<210> 70
   <211> 118
   <212> PRT
   <213> Mus musculus
<400> 70
   Asp Val Gln Leu Gln Glu Ser Gly Pro Gly Leu Val Lys Pro Ser Gln
   1 5 10 15
   Ser Leu Ser Leu Thr Cys Ser Val Thr Gly Tyr Ser Ile Thr Ser Gly
   20 25 30
   Tyr Tyr Trp Asn Trp Ile Arg Gln Phe Pro Gly Asn Lys Leu Glu Trp
   35 40 45
   Met Gly Tyr Ile Asn Tyr Asp Gly Asn Asn Asn Tyr Asn Pro Ser Leu
   50 55 60
   Lys Asn Arg Ile Ser Ile Thr Arg Asp Thr Ser Lys Asn Gln Phe Phe
   65 70 75 80
   Leu Lys Leu Asn Ser Val Thr Thr Glu Asp Thr Ala Thr Tyr Tyr Cys
   85 90 95
   Ala Arg Glu Gly Tyr Gly Tyr Phe Phe Asp Tyr Trp Gly Gln Gly Thr
   100 105 110
   Thr Leu Thr Val Ser Ser
   115
<210> 71
   <211> 118
   <212> PRT
   <213> Mus musculus
<400> 71
   Glu Val Gln Leu Gln Glu Ser Gly Pro Ser Leu Val Lys Pro Ser Gln
    1               5                  10                  15
   Thr Leu Ser Leu Thr Cys Ser Val Thr Gly Asp Ser Ile Thr Ser Gly
               20                  25                  30
   Tyr Trp Asn Asn Trp Ile Arg Gln Phe Pro Gly Asn Lys Leu Glu Trp
           35                  40                  45
   Met Gly Tyr Ile Ser Tyr Ser Gly Ser Thr Tyr Tyr Asn Pro Ser Leu
       50                  55                  60
   Lys Ser Arg Ile Ser Ile Thr Arg Asp Thr Ser Lys Asn Gln Tyr Phe
   65                  70                  75                  80
   Leu Gln Leu Asn Ser Val Thr Thr Glu Asp Thr Ala Thr Tyr Tyr Cys
                   85                  90                  95
   Ala Arg Gly Gly Tyr Gly Tyr Gly Phe Asp Tyr Trp Gly Gln Gly Thr
               100                 105                 110
   Thr Val Thr Val Ser Ser
           115
<210> 72
   <211> 117
   <212> PRT
   <213> Homo sapiens
<220>
   <221> VARIANT
   <222> (59)
   <223> Xaa corespondant à n'importe quel acide aminé
<400> 72
   Gln Val Gln Leu Gln Glu Ser Gly Pro Gly Leu Val Lys Pro Ser Gln
    1               5                  10                  15
   Thr Leu Ser Leu Thr Cys Thr Val Ser Gly Gly Ser Val Ser Ser Tyr
               20                  25                  30
   Trp Ser Trp Asn Trp Ile Arg Gln Pro Pro Gly Lys Gly Leu Glu Trp
           35                  40                  45
   Ile Gly Arg Ile Tyr Tyr Ser Gly Ser Thr Xaa Tyr Asn Pro Ser Leu
       50                  55                  60
   Lys Ser Arg Val Thr Ile Ser Val Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Glu Leu Pro Gly Gly Tyr Asp Val Trp Gly Gln Gly Thr Leu
               100                 105                 110
   Val Thr Val Ser Ser
   115
<210> 73
   <211> 123
   <212> PRT
   <213> Homo sapiens
<400> 73
   Gln Val Gln Leu Gln Glu Ser Gly Pro Gly Leu Val Lys Pro Ser Glu
   1               5                   10                  15
   Thr Leu Ser Leu Thr Cys Thr Val Ser Gly Tyr Ser Ile Ser Ser Gly
               20                  25                  30
   Tyr Tyr Trp Ser Trp Ile Arg Gln Pro Pro Gly Lys Gly Leu Glu Trp
           35                  40                  45
   Ile Gly Ser Met Phe His Ser Gly Ser Ser Tyr Tyr Asn Pro Ser Leu
       50                  55                  60
   Lys Ser Arg Val Thr Ile Ser Val Asp Thr Ser Lys Asn Gln Phe Ser
   65                  70                  75                  80
   Leu Gln Leu Arg Ser Val Thr Ala Ala Asp Thr Ala Val Tyr Tyr Cys
                   85                  90                  95
   Ala Arg Gly Arg Tyr Cys Ser Ser Thr Ser Cys Asn Trp Phe Asp Pro
               100                 105                 110
   Trp Gly Gln Gly Thr Leu Val Thr Val Ser Ser
           115                 120
<210> 74
   <211> 98
   <212> PRT
   <213> Homo sapiens
<400> 74
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Tyr Ser Ile Ser Ser Gly
               20                  25                  30
   Tyr Tyr Trp Ser Trp Ile Arg Gln Pro Pro Gly Lys Gly Leu Glu Trp
           35                  40                  45
   Ile Gly Ser Ile Tyr His Ser Gly Ser Thr Tyr Tyr Asn Pro Ser Leu
       50                  55                  60
   Lys Ser Arg Val Thr Ile Ser Val Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg
<210> 75
   <211> 117
   <212> PRT
   <213> Homo sapiens
<400> 75
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Tyr Ser Ile Thr Gly Gly
               20                  25                  30
   Tyr Leu Trp Asn Trp Ile Arg Gln Pro Pro Gly Lys Gly Leu Glu Trp
           35                  40                  45
   Met Gly Tyr Ile Ser Tyr Asp Gly Thr Asn Asn Tyr Lys Pro Ser Leu
       50                  55                  60
   Lys Asp Arg Ile Thr Ile Ser Arg Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Tyr Gly Arg Val Phe Phe Asp Tyr Trp Gly Gln Gly Thr Leu
               100                 105                 110
   Val Thr Val Ser Ser
           115
<210> 76
   <211> 445
   <212> ADN
   <213> Homo sapiens
<220>
   <221> CDS
   <222> (22)..(426)
<400> 76
   gtcagaacgc gtgccgccac c atg aaa gtg ttg agt ctg ttg tac ctc ttg    51
                           Met Lys Val Leu Ser Leu Leu Tyr Leu Leu
                           1               5                   10
   aca gcc att cct ggt atc ctg tct cag gtg cag ctt cag gag tcg ggc    99
   Thr Ala Ile Pro Gly Ile Leu Ser Gln Val Gln Leu Gln Glu Ser Gly
                   15                  20                  25
   cca gga ctg gtg aag cct tcg gag acc ctg tcc ctc acc tgc act gtc    147
   Pro Gly Leu Val Lys Pro Ser Glu Thr Leu Ser Leu Thr Cys Thr Val
               30                  35                  40
   tct ggt tac tcc atc acc ggt ggt tat tta tgg aac tgg ata cgg cag    195
   Ser Gly Tyr Ser Ile Thr Gly Gly Tyr Leu Trp Asn Trp Ile Arg Gln
           45                  50                  55
   ccc cca ggg aag gga ctg gag tgg atg ggg tat atc agc tac gac ggt    243
   Pro Pro Gly Lys Gly Leu Glu Trp Met Gly Tyr Ile Ser Tyr Asp Gly
       60                  65                  70
   acc aat aac tac aaa ccc tcc ctc aag gat cga atc acc ata tca cgt    291
   Thr Asn Asn Tyr Lys Pro Ser Leu Lys Asp Arg Ile Thr Ile Ser Arg
   75                  80                  85                  90
   gac acg tcc aag aac cag ttc tcc ctg aag ctg agc tct gtg acc gct    339
   Asp Thr Ser Lys Asn Gln Phe Ser Leu Lys Leu Ser Ser Val Thr Ala
                   95                  100                 105
   gcg gac act gca gtg tat tac tgt gcg aga tac ggt agg gtc ttc ttt    387
   Ala Asp Thr Ala Val Tyr Tyr Cys Ala Arg Tyr Gly Arg Val Phe Phe
               110                 115                 120
   gac tac tgg ggc cag gga acc ctg gtc acc gtc tcc tca ggtgagtgga     436
   Asp Tyr Trp Gly Gln Gly Thr Leu Val Thr Val Ser Ser
           125                 130
   tcctctgcg                                                          445
<210> 77
   <211> 135
   <212> PRT
   <213> Homo sapiens
<400> 77
   Met Lys Val Leu Ser Leu Leu Tyr Leu Leu Thr Ala Ile Pro Gly Ile
    1               5                  10                  15
   Leu Ser Gln Val Gln Leu Gln Glu Ser Gly Pro Gly Leu Val Lys Pro
               20                  25                  30
   Ser Glu Thr Leu Ser Leu Thr Cys Thr Val Ser Gly Tyr Ser Ile Thr
           35                  40                  45
   Gly Gly Tyr Leu Trp Asn Trp Ile Arg Gln Pro Pro Gly Lys Gly Leu
       50                  55                  60
   Glu Trp Met Gly Tyr Ile Ser Tyr Asp Gly Thr Asn Asn Tyr Lys Pro
   65                  70                  75                  80
   Ser Leu Lys Asp Arg Ile Thr Ile Ser Arg Asp Thr Ser Lys Asn Gln
                   85                  90                  95
   Phe Ser Leu Lys Leu Ser Ser Val Thr Ala Ala Asp Thr Ala Val Tyr
               100                 105                 110
   Tyr Cys Ala Arg Tyr Gly Arg Val Phe Phe Asp Tyr Trp Gly Gln Gly
           115                 120                 125
   Thr Leu Val Thr Val Ser Ser
       130
<210> 78
   <211> 445
   <212> ADN
   <213> Homo sapiens
<400> 78
   cagtcttgcg cacggcggtg gtactttcac aactcagaca acatggagaa ctgtcggtaa 60
   ggaccatagg acagagtcca cgtcgaagtc ctcagcccgg gtcctgacca cttcggaagc 120
   ctctgggaca gggagtggac gtgacagaga ccaatgaggt agtggccacc aataaatacc 180
   ttgacctatg ccgtcggggg tcccttccct gacctcacct accccatata gtcgatgctg 240
   ccatggttat tgatgtttgg gagggagttc ctagcttagt ggtatagtgc actgtgcagg 300
   ttcttggtca agagggactt cgactcgaga cactggcgac gcctgtgacg tcacataatg 360
   acacgctcta tgccatccca gaagaaactg atgaccccgg tcccttggga ccagtggcag 420
   aggagtccac tcacctagga gacgc                                       445
<210> 79
   <211> 117
   <212> PRT
   <213> Homo sapiens
<400> 79
<210> 80
   <211> 445
   <212> ADN
   <213> Homo sapiens
<220>
   <221> CDS
   <222> (22)..(426)
<400> 80
   gtcagaacgc gtgccgccac c atg aaa gtg ttg agt ctg ttg tac ctc ttg    51
                           Met Lys Val Leu Ser Leu Leu Tyr Leu Leu
                           1               5                   10
   aca gcc att cct ggt atc ctg tct cag gtg cag ctt cag gag tcg ggc    99
   Thr Ala Ile Pro Gly Ile Leu Ser Gln Val Gln Leu Gln Glu Ser Gly
               15                  20                  25
   cca gga ctg gtg aag cct tcg gag acc ctg tcc ctc acc tgc act gtc   147
   Pro Gly Leu Val Lys Pro Ser Glu Thr Leu Ser Leu Thr Cys Thr Val
               30                  35                  40
   tct ggt tac tcc atc acc ggt ggt tat tta tgg aac tgg ata cgg cag     195
   Ser Gly Tyr Ser Ile Thr Gly Gly Tyr Leu Trp Asn Trp Ile Arg Gln
           45                  50                  55
   ccc cca ggg aag gga ctg gag tgg atc ggg tat atc agc tac gac ggt     243
   Pro Pro Gly Lys Gly Leu Glu Trp Ile Gly Tyr Ile Ser Tyr Asp Gly
       60                  65                  70
   acc aat aac tac aaa ccc tcc ctc aag gat cga gtc acc ata tca cgt     291
   Thr Asn Asn Tyr Lys Pro Ser Leu Lys Asp Arg Val Thr Ile Ser Arg
   75                  80                  85                  90
   gac acg tcc aag aac cag ttc tcc ctg aag ctg agc tct gtg acc gct     339
   Asp Thr Ser Lys Asn Gln Phe Ser Leu Lys Leu Ser Ser Val Thr Ala
                   95                  100                 105
   gcg gac act gca gtg tat tac tgt gcg aga tac ggt agg gtc ttc ttt     387
   Ala Asp Thr Ala Val Tyr Tyr Cys Ala Arg Tyr Gly Arg Val Phe Phe
               110                 115                 120
   gac tac tgg ggc cag gga acc ctg gtc acc gtc tcc tca ggtgagtgga      436
   Asp Tyr Trp Gly Gln Gly Thr Leu Val Thr Val Ser Ser
           125                 130
   tcctctgcg                                                           445
<210> 81
   <211> 135
   <212> PRT
   <213> Homo sapiens
<400> 81
   Met Lys Val Leu Ser Leu Leu Tyr Leu Leu Thr Ala Ile Pro Gly Ile
   1               5                   10                  15
   Leu Ser Gln Val Gln Leu Gln Glu Ser Gly Pro Gly Leu Val Lys Pro
               20                  25                  30
   Ser Glu Thr Leu Ser Leu Thr Cys Thr Val Ser Gly Tyr Ser Ile Thr
           35                  40                  45
   Gly Gly Tyr Leu Trp Asn Trp Ile Arg Gln Pro Pro Gly Lys Gly Leu
       50                  55                  60
   Glu Trp Ile Gly Tyr Ile Ser Tyr Asp Gly Thr Asn Asn Tyr Lys Pro
   65                  70                  75                  80
   Ser Leu Lys Asp Arg Val Thr Ile Ser Arg Asp Thr Ser Lys Asn Gln
                   85                  90                  95
   Phe Ser Leu Lys Leu Ser Ser Val Thr Ala Ala Asp Thr Ala Val Tyr
               100                 105                 110
   Tyr Cys Ala Arg Tyr Gly Arg Val Phe Phe Asp Tyr Trp Gly Gln Gly
           115                 120                 125
   Thr Leu Val Thr Val Ser Ser
                           130
<210> 82
   <211> 445
   <212> ADN
   <213> Homo sapiens
<400> 82
   cagtcttgcg cacggcggtg gtactttcac aactcagaca acatggagaa ctgtcggtaa 60
   ggaccatagg acagagtcca cgtcgaagtc ctcagcccgg gtcctgacca cttcggaagc 120
   ctctgggaca gggagtggac gtgacagaga ccaatgaggt agtcgccacc aataaatacc 180
   ttgacctatg ccgtcggggg tcccttccct gacctcacct agcccatata gtcgatgctg 240
   ccatggttat tgatgtttgg gagggagttc ctagctcagt ggtatagtgc actgtgcagg 300
   ttcttggtca agagggactt cgactcgaga cactggcgac gcctgtgacg tcacataatg 360
   acacgctcta tgccatccca gaagaaactg atgaccccgg tcccttggga ccagtggcag 420
   aggagtccac tcacctagga gacgc                                       445
<210> 83
   <211> 117
   <212> PRT
   <213> Homo sapiens
<400> 83
   Gln Val Gln Leu Gln Glu Ser Gly Pro Gly Leu Val Lys Pro Ser Glu
    1               5                  10                  15
   Thr Leu Ser Leu Thr Cys Thr Val Ser Gly Tyr Ser Ile Ser Gly Gly
               20                  25                  30
   Tyr Leu Trp Asn Trp Ile Arg Gln Pro Pro Gly Lys Gly Leu Glu Trp
           35                  40                  45
   Ile Gly Tyr Ile Ser Tyr Asp Gly Thr Asn Asn Tyr Lys Pro Ser Leu
       50                  55                  60
   Lys Asp Arg Val Thr Ile Ser Val Asp Thr Ser Lys Asn Gln Phe Ser
   65                  70                  75                  80
   Leu Lys Leu Ser Ser Val Thr Ala Ala Asp Thr Ala Val Tyr Tyr Cys
                   85                  90                  95
   Ala Arg Tyr Gly Arg Val Phe Phe Asp Tyr Trp Gly Gln Gly Thr Leu
               100                 105                 110
   Val Thr Val Ser Ser
           115
<210> 84
   <211> 445
   <212> ADN
   <213> Homo sapiens
<220>
   <221> CDS
   <222> (22)..(426)
<400> 84
   gtcagaacgc gtgccgccac c atg aaa gtg ttg agt ctg ttg tac ctc ttg   51
                                 Met Lys Val Leu Ser Leu Leu Tyr Leu Leu
                                  1               5                  10
   aca gcc att cct ggt atc ctg tct cag gtg cag ctt cag gag tcg ggc    99
   Thr Ala Ile Pro Gly Ile Leu Ser Gln Val Gln Leu Gln Glu Ser Gly
                   15                  20                  25
   cca gga ctg gtg aag cct tcg gag acc ctg tcc ctc acc tgc act gtc   147
   Pro Gly Leu Val Lys Pro Ser Glu Thr Leu Ser Leu Thr Cys Thr Val
               30                  35                  40
   tct ggt tac tcc atc agc ggt ggt tat tta tgg aac tgg ata cgg cag   195
   Ser Gly Tyr Ser Ile Ser Gly Gly Tyr Leu Trp Asn Trp Ile Arg Gln
           45                  50                  55
   ccc cca ggg aag gga ctg gag tgg atc ggg tat atc agc tac gac ggt   243
   Pro Pro Gly Lys Gly Leu Glu Trp Ile Gly Tyr Ile Ser Tyr Asp Gly
       60                  65                  70
   acc aat aac tac aaa ccc tcc ctc aag gat cga gtc acc ata tca gtg   291
   Thr Asn Asn Tyr Lys Pro Ser Leu Lys Asp Arg Val Thr Ile Ser Val
   75                  80                  85                  90
   gac acg tcc aag aac cag ttc tcc ctg aag ctg agc tct gtg acc gct   339
   Asp Thr Ser Lys Asn Gln Phe Ser Leu Lys Leu Ser Ser Val Thr Ala
                   95                  100                 105
   gcg gac act gca gtg tat tac tgt gcg aga tac ggt agg gtc ttc ttt   387
   Ala Asp Thr Ala Val Tyr Tyr Cys Ala Arg Tyr Gly Arg Val Phe Phe
               110                 115                 120
   gac tac tgg ggc cag gga acc ctg gtc acc gtc tcc tca ggtgagtgga   436
   Asp Tyr Trp Gly Gln Gly Thr Leu Val Thr Val Ser Ser
           125                 130
   tcctctgcg                                                        445
<210> 85
   <211> 135
   <212> PRT
   <213> Homo sapiens
<400> 85
   Met Lys Val Leu Ser Leu Leu Tyr Leu Leu Thr Ala Ile Pro Gly Ile
    1               5                  10                  15
   Leu Ser Gln Val Gln Leu Gln Glu Ser Gly Pro Gly Leu Val Lys Pro
               20                  25                  30
   Ser Glu Thr Leu Ser Leu Thr Cys Thr Val Ser Gly Tyr Ser Ile Ser
           35                  40                  45
   Gly Gly Tyr Leu Trp Asn Trp Ile Arg Gln Pro Pro Gly Lys Gly Leu
       50                  55                  60
   Glu Trp Ile Gly Tyr Ile Ser Tyr Asp Gly Thr Asn Asn Tyr Lys Pro
   65                  70                  75                  80
   Ser Leu Lys Asp Arg Val Thr Ile Ser Val Asp Thr Ser Lys Asn Gln
                   85                  90                  95
   Phe Ser Leu Lys Leu Ser Ser Val Thr Ala Ala Asp Thr Ala Val Tyr
               100                 105                 110
   Tyr Cys Ala Arg Tyr Gly Arg Val Phe Phe Asp Tyr Trp Gly Gln Gly
           115                 120                 125
   Thr Leu Val Thr Val Ser Ser
       130
<210> 86
   <211> 445
   <212> ADN
   <213> Homo sapiens
<400> 86
   cagtcttgcg cacggcggtg gtactttcac aactcagaca acatggagaa ctgtcggtaa 60
   ggaccatagg acagagtcca cgtcgaagtc ctcagcccgg gtcctgacca cttcggaagc 120
   ctctgggaca gggagtggac gtgacagaga ccaatgaggt agtcgccacc aataaatacc 180
   ttgacctatg ccgtcggggg tcccttccct gacctcacct agcccatata gtcgatgctg 240
   ccatggttat tgatgtttgg gagggagttc ctagctcagt ggtatagtca cctgtgcagg 300
   ttcttggtca agagggactt cgactcgaga cactggcgac gcctgtgacg tcacataatg 360
   acacgctcta tgccatccca gaagaaactg atgaccccgg tcccttggga ccagtggcag 420
   aggagtccac tcacctagga gacgc                                      445
<210> 87
   <211> 18
   <212> ADN
   <213> Séquence artificielle
<220>
   <223> Description de la séquence artificielle: Oligonucléotide
<400> 87
   gtcagaacgc gtgccgcc 18
<210> 88
   <211> 32
   <212> ADN
   <213> Séquence artificielle
<220>
   <223> Description de la séquence artificielle: Oligonucléotide
<400> 88
   accatgaagt tgcctgttag gctgttggtg ct 32
<210> 89
   <211> 32
   <212> ADN
   <213> Séquence artificielle
<220>
   <223> Description de la séquence artificielle: Oligonucléotide
<400> 89
   gatgttctgg tttcctgctt ccagcagtga tg 32
<210> 90
   <211> 32
   <212> ADN
   <213> Séquence artificielle
<220>
   <223> Description de la séquence artificielle: Oligonucléotide
<400> 90
   ttgtgatgac tcagtctcca ctctccctgc cc 32
<210> 91
   <211> 32
   <212> ADN
   <213> Séquence artificielle
<220>
   <223> Description de la séquence artificielle: Oligonucléotide
<400> 91
   gtcacccctg gagagccggc ctccatctcc tg 32
<210> 92
   <211> 32
   <212> ADN
   <213> Séquence artificielle
<220>
   <223> Description de la séquence artificielle: Oligonucléotide
<400> 92
   caggtctagt cagaccatta tacatagtaa tg 32
<210> 93
   <211> 30
   <212> ADN
   <213> Séquence artificielle
<220>
   <223> Description de la séquence artificielle: Oligonucléotide
<400> 93
   gaaacaccta tttggaatgg tacctgcaga 30
<210> 94
   <211> 32
   <212> ADN
   <213> Séquence artificielle
<220>
   <223> Description de la séquence artificielle: Oligonucléotide
<400> 94
   ggcaacttca tggtggcggc acgcgttctg ac 32
<210> 95
   <211> 32
   <212> ADN
   <213> Séquence artificielle
<220>
   <223> Description de la séquence artificielle: Oligonucléotide
<400> 95
   gaaaccagaa catcagcacc aacagcctaa ca 32
<210> 96
   <211> 32
   <212> ADN
   <213> Séquence artificielle
<220>
   <223> Description de la séquence artificielle: Oligonucléotide
<400> 96
   ctgagtcatc acaacatcac tgctggaagc ag 32
<210> 97
   <211> 32
   <212> ADN
   <213> Séquence artificielle
<220>
   <223> Description de la séquence artificielle: Oligonucléotide
<400> 97
   tctccagggg tgacgggcag ggagagtgga ga 32
<210> 98
   <211> 32
   <212> ADN
   <213> Séquence artificielle
<220>
   <223> Description de la séquence artificielle: Oligonucléotide
<400> 98
   tctgactaga cctgcaggag atggaggccg gc 32
<210> 99
   <211> 31
   <212> ADN
   <213> Séquence artificielle
<220>
   <223> Description de la séquence artificielle: Oligonucléotide
<400> 99
   aaataggtgt ttccattact atgtacaatg c 31
<210> 100
   <211> 32
   <212> ADN
   <213> Séquence artificielle
<220>
   <223> Description de la séquence artificielle: Oligonucléotide
<400> 100
   cagggcagtc tccacagctc ctgatctata aa 32
<210> 101
   <211> 32
   <212> ADN
   <213> Séquence artificielle
<220>
   <223> Description de la séquence artificielle: Oligonucléotide
<400> 101
   gtttctaatc ggctttatgg ggtccctgac ag 32
<210> 102
   <211> 32
   <212> ADN
   <213> Séquence artificielle
<220>
   <223> Description de la séquence artificielle: Oligonucléotide
<400> 102
   gttcagtggc agtggatcag gcacagattt ta 32
<210> 103
   <211> 32
   <212> ADN
   <213> Séquence artificielle
<220>
   <223> Description de la séquence artificielle: Oligonucléotide
<400> 103
   cactgaaaat cagcagagtg gaggctgagg at 32
<210> 104
   <211> 32
   <212> ADN
   <213> Séquence artificielle
<220>
   <223> Description de la séquence artificielle: Oligonucléotide
<400> 104
   gttggggttt attactgctt tcaaggttca ca 32
<210> 105
   <211> 32
   <212> ADN
   <213> Séquence artificielle
<220>
   <223> Description de la séquence artificielle: Oligonucléotide
<400> 105
   tgttccgtgg acgttcggcc aagggaccaa gg 32
<210> 106
   <211> 30
   <212> ADN
   <213> Séquence artificielle
<220>
   <223> Description de la séquence artificielle: Oligonucléotide
<400> 106
   tggaaatcaa acgtgagtgg atcctctgcg 30
<210> 107
   <211> 17
   <212> ADN
   <213> Séquence artificielle
<220>
   <223> Description de la séquence artificielle: Oligonucléotide
<400> 107
   tctgcaggta ccattgc 17
<210> 108
   <211> 21
   <212> ADN
   <213> Séquence artificielle
<220>
   <223> Description de la séquence artificielle: Oligonucléotide
<400> 108
   tgcaatggta cctgcagaag c 21
<210> 109
   <211> 32
   <212> ADN
   <213> Séquence artificielle
<220>
   <223> Description de la séquence artificielle: Oligonucléotide
<400> 109
   agactgccct ggcttctgca ggtaccattg ca 32
<210> 110
   <211> 32
   <212> ADN
   <213> Séquence artificielle
<220>
   <223> Description de la séquence artificielle: Oligonucléotide
<400> 110
   cgattagaaa ctttatagat caggagctgt gg 32
<210> 111
   <211> 32
   <212> ADN
   <213> Séquence artificielle
<220>
   <223> Description de la séquence artificielle: Oligonucléotide
<400> 111
   tgccactgaa cctgtcaggg accccataaa gc 32
<210> 112
   <211> 32
   <212> ADN
   <213> Séquence artificielle
<220>
   <223> Description de la séquence artificielle: Oligonucléotide
<400> 112
   gattttcagt gtaaaatctg tgcctgatcc ac 32
<210> 113
   <211> 32
   <212> ADN
   <213> Séquence artificielle
<220>
   <223> Description de la séquence artificielle: Oligonucléotide
<400> 113
   taaaccccaa catcctcagc ctccactctg ct 32
<210> 114
   <211> 32
   <212> ADN
   <213> Séquence artificielle
<220>
   <223> Description de la séquence artificielle: Oligonucléotide
<400> 114
   tccacggaac atgtgaacct tgaaagcagt aa 32
<210> 115
   <211> 31
   <212> ADN
   <213> Séquence artificielle
<220>
   <223> Description de la séquence artificielle: Oligonucléotide
<400> 115
   tttgatttcc accttggtcc cttggccgaa c 31
<210> 116
   <211> 19
   <212> ADN
   <213> Séquence artificielle
<220>
   <223> Description de la séquence artificielle: Oligonucléotide
<400> 116
   cgcagaggat ccactcacg 19
<210> 117
   <211> 18
   <212> ADN
   <213> Séquence artificielle
<220>
   <223> Description de la séquence artificielle: Oligonucléotide
<400> 117
   gtcagaacgc gtgccgcc 18
<210> 118
   <211> 34
   <212> ADN
   <213> Séquence artificielle
<220>
   <223> Description de la séquence artificielle: Oligonucléotide
<400> 118
   accatgaaag tgttgagtct gttgtacctc ttga 34
<210> 119
   <211> 34
   <212> ADN
   <213> Séquence artificielle
<220>
   <223> Description de la séquence artificielle: Oligonucléotide
<400> 119
   cagccattcc tggtatcctg tctcaggtgc agct 34
<210> 120
   <211> 34
   <212> ADN
   <213> Séquence artificielle
<220>
   <223> Description de la séquence artificielle: Oligonucléotide
<400> 120
   tcaggagtcg ggcccaggac tggtgaagcc ttcg 34
<210> 121
   <211> 33
   <212> ADN
   <213> Séquence artificielle
<220>
   <223> Description de la séquence artificielle: Oligonucléotide
<400> 121
   gagaccctgt ccctcacctg cactgtctct ggt 33
<210> 122
   <211> 33
   <212> ADN
   <213> Séquence artificielle
<220>
   <223> Description de la séquence artificielle: Oligonucléotide
<400> 122
   tactccatca ccggtggtta tttatggaac tgg 33
<210> 123
   <211> 33
   <212> ADN
   <213> Séquence artificielle
<220>
   <223> Description de la séquence artificielle: Oligonucléotide
<400> 123
   atacggcagc ccccagggaa gggactggag tgg 33
<210> 124
   <211> 33
   <212> ADN
   <213> Séquence artificielle
<220>
   <223> Description de la séquence artificielle: Oligonucléotide
<400> 124
   atggggtata tcagctacga cggtaccaat aac 33
<210> 125
   <211> 34
   <212> ADN
   <213> Séquence artificielle
<220>
   <223> Description de la séquence artificielle: Oligonucléotide
<400> 125
   tcaacacttt catggtggcg gcacgcgttc tgac 34
<210> 126
   <211> 34
   <212> ADN
   <213> Séquence artificielle
<220>
   <223> Description de la séquence artificielle: Oligonucléotide
<400> 126
   ataccaggaa tggctgtcaa gaggtacaac agac 34
<210> 127
   <211> 34
   <212> ADN
   <213> Séquence artificielle
<220>
   <223> Description de la séquence artificielle: Oligonucléotide
<400> 127
   tgggcccgac tcctgaagct gcacctgaga cagg 34
<210> 128
   <211> 34
   <212> ADN
   <213> Séquence artificielle
<220>
   <223> Description de la séquence artificielle: Oligonucléotide
<400> 128
   tgagggacag ggtctccgaa ggcttcacca gtcc 34
<210> 129
   <211> 34
   <212> ADN
   <213> Séquence artificielle
<220>
   <223> Description de la séquence artificielle: Oligonucléotide
<400> 129
   ccaccggtga tggagtaacc agagacagtg cagg 34
<210> 130
   <211> 34
   <212> ADN
   <213> Séquence artificielle
<220>
   <223> Description de la séquence artificielle: Oligonucléotide
<400> 130
   ccctgggggc tgccgtatcc agttccataa ataa 34
<210> 131
   <211> 32
   <212> ADN
   <213> Séquence artificielle
<220>
   <223> Description de la séquence artificielle:
   Oligonucléotide
<400> 131
   tagctgatat accccatcca ctccagtccc tt 32
<210> 132
   <211> 16
   <212> ADN
   <213> Séquence artificielle
<220>
   <223> Description de la séquence artificielle: Oligonucléotide
<400> 132
   gttattggta ccgtcg 16
<210> 133
   <211> 21
   <212> ADN
   <213> Séquence artificielle
<220>
   <223> Description de la séquence artificielle: Oligonucléotide
<400> 133
   tacgacggta ccaataacta c 21
<210> 134
   <211> 32
   <212> ADN
   <213> Séquence artificielle
<220>
   <223> Description de la séquence artificielle: Oligonucléotide
<400> 134
   aaaccctccc tcaaggatcg aatcaccata tc 32
<210> 135
   <211> 32
   <212> ADN
   <213> Séquence artificielle
<220>
   <223> Description de la séquence artificielle: Oligonucléotide
<400> 135
   acgtgacacg tccaagaacc agttctccct ga 32
<210> 136
   <211> 32
   <212> ADN
   <213> Séquence artificielle
<220>
   <223> Description de la séquence artificielle: Oligonucléotide
<400> 136
   agctgagctc tgtgaccgct gcggacactg ca 32
<210> 137
   <211> 32
   <212> ADN
   <213> Séquence artificielle
<220>
   <223> Description de la séquence artificielle: Oligonucléotide
<400> 137
   gtgtattact gtgcgagata cggtagggtc tt 32
<210> 138
   <211> 32
   <212> ADN
   <213> Séquence artificielle
<220>
   <223> Description de la séquence artificielle: Oligonucléotide
<400> 138
   ctttgactac tggggccagg gaaccctggt ca 32
<210> 139
   <211> 30
   <212> ADN
   <213> Séquence artificielle
<220>
   <223> Description de la séquence artificielle: Oligonucléotide
<400> 139
   ccgtctcctc aggtgagtgg atcctctgcg 30
<210> 140
   <211> 32
   <212> ADN
   <213> Séquence artificielle
<220>
   <223> Description de la séquence artificielle: Oligonucléotide
<400> 140
   agggagggtt tgtagttatt ggtaccgtcg ta 32
<210> 141
   <211> 32
   <212> ADN
   <213> Séquence artificielle
<220>
   <223> Description de la séquence artificielle: Oligonucléotide
<400> 141
   acgtgtcacg tgatatggtg attcgatcct tg 32
<210> 142
   <211> 32
   <212> ADN
   <213> Séquence artificielle
<220>
   <223> Description de la séquence artificielle: Oligonucléotide
<400> 142
   agagctcagc ttcagggaga actggttctt gg 32
<210> 143
   <211> 32
   <212> ADN
   <213> Séquence artificielle
<220>
   <223> Description de la séquence artificielle: Oligonucléotide
<400> 143
   cagtaataca ctgcagtgtc cgcagcggtc ac 32
<210> 144
   <211> 32
   <212> ADN
   <213> Séquence artificielle
<220>
   <223> Description de la séquence artificielle: Oligonucléotide
<400> 144
   agtagtcaaa gaagacccta ccgtatctcg ca 32
<210> 145
   <211> 33
   <212> ADN
   <213> Séquence artificielle
<220>
   <223> Description de la séquence artificielle: Oligonucléotide
<400> 145
   ctgaggagac ggtgaccagg gttccctggc ccc 33
<210> 146
   <211> 18
   <212> ADN
   <213> Séquence artificielle
<220>
   <223> Description de la séquence artificielle: Oligonucléotide
<400> 146
   cgcagaggat ccactcac 18
<210> 147
   <211> 31
   <212> ADN
   <213> Homo sapiens
<400> 147
   ctggttactc catcagcggt ggttatttat g 31
<210> 148
   <211> 31
   <212> ADN
   <213> Homo sapiens
<400> 148
   cataaataac caccgctgat ggagtaacca g 31
<210> 149
   <211> 31
   <212> ADN
   <213> Homo sapiens
<400> 149
   gggactggag tggatcgggt atatcagcta c 31
<210> 150
   <211> 31
   <212> ADN
   <213> Homo sapiens
<400> 150
   gtagctgata tacccgatcc actccagtcc c 31
<210> 151
   <211> 31
   <212> ADN
   <213> Homo sapiens
<400> 151
   tccctcaagg atcgagtcac catatcacgt g 31
<210> 152
   <211> 31
   <212> ADN
   <213> Homo sapiens
<400> 152
   cacgtgatat ggtgactcga tccttgaggg a 31
<210> 153
   <211> 39
   <212> ADN
   <213> Homo sapiens
<400> 153
   gatcgagtca ccatatcagt ggacacgtcc aagaaccag 39
<210> 154
   <211> 39
   <212> ADN
   <213> Homo sapiens
<400> 154
   ctggttcttg gacgtgtcca ctgatatggt gactcgatc 39
<210> 155
   <211> 31
   <212> ADN
   <213> Homo sapiens
<400> 155
   gcttccagca gtgatattgt gatgactcag t 31
<210> 156
   <211> 31
   <212> ADN
   <213> Homo sapiens
<400> 156
   actgagtcat cacaatatca ctgctggaag c 31

## Description

La présente invention est relative à de nouveaux anticorps capables de se lier spécifiquement au récepteur humain du facteur de croissance I apparenté à l'insuline IGF-IR et/ou capables d'inhiber spécifiquement l'activité tyrosine kinase dudit récepteur IGF-IR, notamment monoclonaux d'origine murine, chimériques et humanisés, ainsi que les séquences d'acide aminé et nucléiques codant pour ces anticorps. L'invention comprend également l'utilisation de ces anticorps à titre de médicament pour le traitement prophylactique et/ou thérapeutique de cancers surexprimant l'IGF-IR ou de toute pathologie liée à la surexpression dudit récepteur ainsi que dans des procédés ou nécessaires de diagnostic de maladies liées à la surexpression du récepteur IGF-IR. L'invention comprend enfin des produits et/ou des compositions comprenant de tels anticorps en association avec des anticorps et/ou des composés anti-EGFR et/ou des agents anticancéreux ou conjugués avec des toxines et leur utilisation pour la prévention et/ou le traitement de certains cancers.

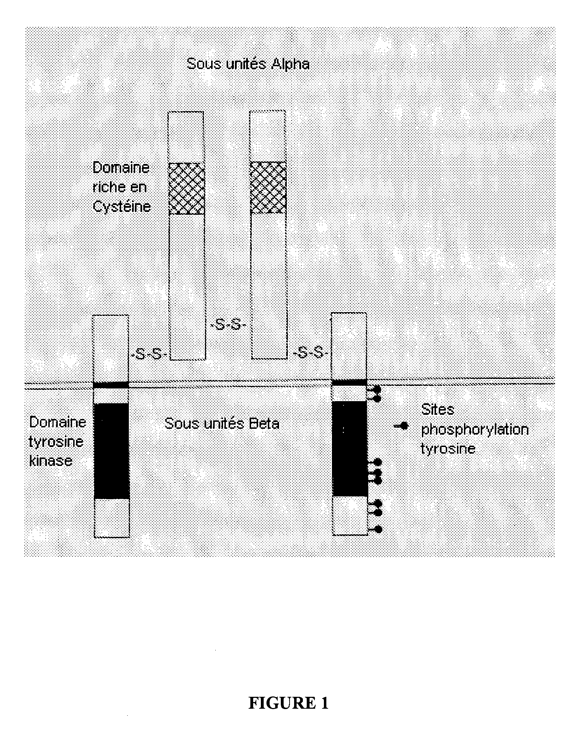

Le récepteur du facteur de croissance I apparenté à l'insuline dénommé IGF-IR ("IGF-IR" pour "Insuline-like Growth Factor-I Receptor") est un récepteur à activité tyrosine kinase comportant 70 % d'homologie avec le récepteur à l'insuline IR ("IR" pour "Insuline Receptor"). L'IGF-IR est une glycoprotéine de poids moléculaire d'environ 350 000. C'est un récepteur hétérotétramérique dont chaque moitié -reliée par des ponts disulfures- est composée d'une sous unité α extracellulaire et d'une sous unité β transmembranaire (voir figure 1). L'IGF-IR fixe l'IGF I et l'IGF II avec une très forte affinité (Kd # 1 nM) mais est également capable de se lier à l'insuline avec une affinité 100 à 1 000 fois moindre. Inversement, l'IR fixe l'insuline avec une très forte affinité alors que les IGFs ne se fixent au récepteur à l'insuline qu'avec une affinité 100 fois inférieure. Le domaine tyrosine kinase de l'IGF-IR et de l'IR présentent une très forte homologie de séquence alors que les zones de plus faible homologie concernent respectivement la région riche en cystéine située sur la sous unité α et la partie C-terminale de la sous unité β. Les différences de séquences observées dans la sous unité α sont situées dans la zone de fixation des ligands et sont donc à l'origine des affinités relatives de l'IGF-IR et de l'IR pour les IGFs et l'insuline respectivement. Les différences dans la partie C-terminale de la sous unité β résultent en une divergence dans les voies de signalisation des deux récepteurs ; l'IGF-IR médiant des effets mitogéniques, de différenciation et d'anti-apoptose, alors que l'activation de l'IR entraîne principalement des effets au niveau des voies métaboliques (Baserga et al., Biochim. Biophys. Acta, 1332:F105-126, 1997 ; Baserga R., Exp. Cell. Res., 253:1-6, 1999).

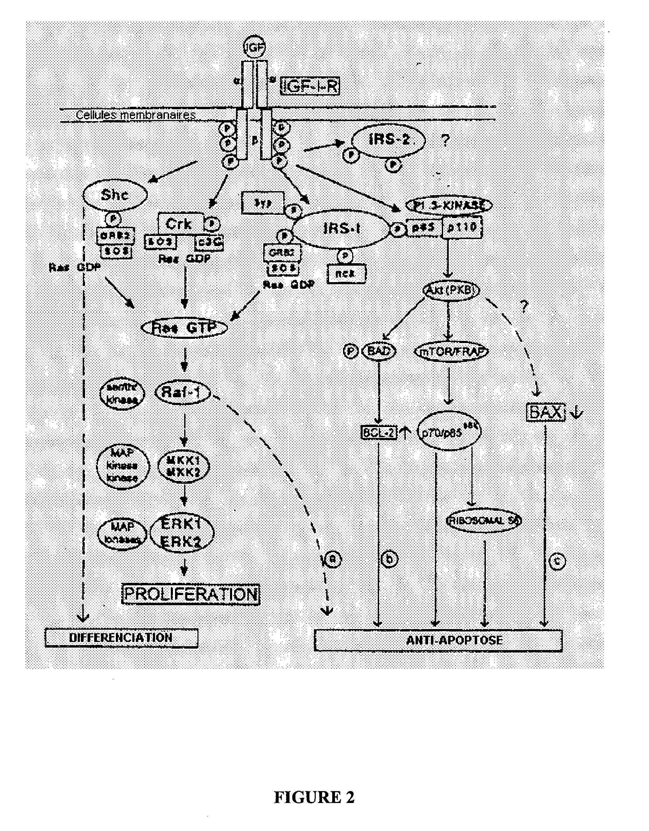

Les protéines tyrosine-kinases cytoplasmiques sont activées par la fixation du ligand au domaine extracellulaire du récepteur. L'activation des kinases entraîne à son tour la stimulation de différents substrats intracellulaires, incluant l'IRS-1, l'IRS-2, Shc et Grb 10 (Peruzzi F. et al., J. Cancer Res. Clin. Oncol., 125:166-173, 1999). Les deux substrats majeurs de l'IGF-IR sont IRS et Shc qui médient, par l'activation de nombreux effecteurs en aval, la plupart des effets de croissance et de différenciation liés à la fixation des IGFs à ce récepteur (figure 2). La disponibilité de substrats peut par conséquent dicter l'effet biologique final lié à l'activation de l'IGF-IR. Lorsque l'IRS-1 prédomine, les cellules tendent à proliférer et à se transformer. Lorsque Shc domine, les cellules tendent à se différencier (Valentinis B. et al., J. Biol. Chem., 274:12423-12430, 1999). Il semble que la voie principalement en cause pour les effets de protection contre l'apoptose soit la voie des phosphatidylinositol 3-kinases (PI 3-kinases) (Prisco M. et al., Horm. Metab. Res., 31:80-89, 1999 ; Peruzzi F. et al., J. Cancer Res. Clin. Oncol., 125:166-173, 1999).

Le rôle du système IGF dans la cancérogenèse est devenu le sujet de recherches intensives dans les dix dernières années. Cet intérêt a suivi la découverte du fait qu'en plus de ses propriétés mitogéniques et anti-apoptotiques, l'IGF-IR semble être requis pour l'établissement et la maintenance d'un phénotype transformé. En fait, il a été bien établi qu'une surexpression ou une activation constitutive de l'IGF-IR conduit, dans une grande variété de cellules, à une croissance des cellules indépendante du support dans des milieux dépourvus de sérum de veau foetal, et à la formation de tumeurs chez la souris nude. Ceci n'est pas en soi une propriété unique puisqu'une grande variété de produits de gènes surexprimés peuvent transformer des cellules, incluant un bon nombre de récepteurs de facteurs de croissance. Mais la découverte cruciale qui a clairement mis en évidence le rôle majeur joué par l'IGF-IR dans la transformation a été la démonstration que les cellules R-, dans lesquelles le gène codant pour l'IGF-IR a été inactivé, sont totalement réfractaires à la transformation par différents agents qui sont habituellement capables de transformer les cellules comme la protéine E5 du papilloma virus bovin, une surexpression de l'EGFR ou du PDGFR, l'antigène T de SV40, ras activé ou la combinaison de ces deux derniers facteurs (Sell C. et al., Proc. Natl. Acad. Sci., USA, 90:11217-11221, 1993 ; Sell C. et al., Mol. Cell. Biol., 14:3604-3612, 1994 ; Morrione A. J., Virol., 69:5300-5303, 1995 ; Coppola D. et al., Mol. Cell. Biol., 14:4588-4595, 1994 ; DeAngelis T et al., J. Cell. Physiol., 164:214-221, 1995).

L'IGF-IR est exprimé dans une grande variété de tumeurs et de lignées tumorales et les IGFs amplifient la croissance tumorale via leur fixation à l'IGF-IR. D'autres arguments en faveur du rôle de IGF-IR dans la cancérogenèse proviennent d'études utilisant des anticorps monoclonaux murins dirigés contre le récepteur ou des dominants négatifs de l'IGF-IR. En effet, des anticorps monoclonaux murins dirigés contre l'IGF-IR inhibent la prolifération de nombreuses lignées cellulaires en culture et la croissance de cellules tumorales *in vivo* (Arteaga C. et al., Cancer Res., 49:6237-6241, 1989 ; Li et al., Biochem. Biophys. Res. Com., 196:92-98, 1993 ; Zia F et al., J. Cell. Biol., 24:269-275, 1996 ; Scotlandi K et al., Cancer Res., 58:4127-4131, 1998). Il a également été montré dans les travaux de Jiang et al. (Oncogene, 18:6071-6077, 1999) qu'un dominant négatif de l'IGF-IR est capable d'inhiber la prolifération tumorale.

La présente invention a pour objet de pouvoir disposer d'un anticorps monoclonal murin, de préférence un anticorps chimérisé ou humanisé, qui reconnaîtra spécifiquement et avec une forte affinité l'IGF-IR. Cet anticorps n'interagira pas ou peu avec le récepteur IR à l'insuline. Sa fixation devra inhiber *in vitro* la croissance des tumeurs exprimant l'IGF-IR en interagissant principalement avec les voies de transduction du signal activées lors des interactions IGFI/IGF-IR et IGF2/IGF-IR. Cet anticorps devra être actif *in vivo* sur tout les types de tumeurs exprimant l'IGF-IR y compris les tumeurs du sein estrogène dépendantes et les tumeurs de la prostate, ce qui n'est pas le cas pour les anticorps monoclonaux (notés AcM ou ACM) anti-IGF-IR actuellement disponibles. En effet l'αIR3, qui fait référence dans le domaine de l'IGF-IR, inhibe totalement la croissance de tumeurs du sein estrogène dépendantes (MCF-7) *in vitro* mais est sans effet sur le modèle correspondant *in vivo* (Artega C. et al., J. Clin. Invest. 84:1418-1423, 1989). De même, le fragment scFv-Fc dérivé du monoclonal murin 1H7, n'est que faiblement actif sur la tumeur du sein MCF-7 et totalement inactif sur une tumeur de la prostate androgène indépendante (Li S.L. et al., Cancer Immunol. Immunother., 49:243-252, 2000). On peut également citer le document Wraight et al. 2000, (Nature Biotechnology, Vol. 18, 5, 521-526) qui décrit l'utilisation d'oligonucléotides antisens dirigé contre l'IGR-IR, en particulier pour traiter le psoriasis.

De manière surprenante, les inventeurs ont mis en évidence un anticorps chimérique (dénommé C7C10) et deux anticorps humanisés dénommés respectivement h7C10 forme humanisée 1 et h7C10 forme humanisée 2, dérivés de l'anticorps monoclonal murin 7C10, reconnaissant l'IGF-IR et répondant à tous les critères énoncés ci-dessus, c'est-à-dire à une non reconnaissance du récepteur à l'insuline, à un blocage *in vitro* de la prolifération IGF1 et/ou IGF2 induite mais également à l'inhibition *in vivo* de la croissance de différentes tumeurs exprimant l'IGF-IR parmi lesquelles un ostéosarcome et une tumeur du poumon non à petites cellules mais également et plus particulièrement la tumeur du sein estrogène dépendante MCF-7 et une tumeur de la prostate androgène indépendante DU-145. De même, et de façon surprenante, l'intensité d'inhibition de la croissance tumorale de la cellule MCF-7 *in vivo* par l'anticorps 7C10 est comparable, voire significativement supérieure, à celle observée avec le tamoxifen l'un des composés de référence dans le traitement des tumeurs du sein estrogènes dépendantes. Par ailleurs, il a été montré que ces anticorps inhibent la phosphorylation de la tyrosine de la chaine béta de l'IGF-IR et de l'IRS1, premier substrat du récepteur. En outre, il a été également établi que ces anticorps provoquent l'internalisation dudit récepteur et sa dégradation contrairement à ce qui est habituellement observé avec les ligands naturels qui permettent le recyclage rapide du récepteur à la surface des cellules. Ces anticorps ont pu être caractérisés par leur séquence peptidique et nucléique, notamment par la séquence de leurs régions déterminant leur complémentarité (CDR) pour l'IGF-IR.

Ainsi, selon une première forme d'exécution, la présente invention a pour objet un anticorps isolé, ou l'un de ses fragments fonctionnels, ledit anticorps ou l'un de sesdits fragments étant capable de se lier spécifiquement au récepteur humain du facteur de croissance I apparenté à l'insuline IGF-IR et, le cas échéant, de préférence capable en outre d'inhiber la fixation naturelle des ligands IGF1 et/ou IGF2 de IGF-IR et/ou capable d'inhiber spécifiquement l'activité tyrosine kinase dudit récepteurIGF-IR, caractérisé, en ce qu'il comprend une chaîne légère comprenant au moins une région CDR déterminant la complémentarité choisie parmi les CDRs de séquence d'acide aminé SEQ ID Nos. 2, 4 ou 6, ou au moins un CDR dont la séquence présente au moins 80 %, de préférence 85 %, 90 %, 95 % et 98 % d'identité après alignement optimal avec la séquence SEQ ID Nos. 2, 4 ou 6, ou en ce qu'il comprend une chaîne lourde comprenant au moins un CDR choisi parmi les CDRs de séquence d'acide aminé SEQ ID Nos. 8, 10 et 12, ou au moins un CDR dont la séquence présente au moins 80 %, de préférence 85 %, 90 %, 95 % et 98 % d'identité après alignement optimal avec la séquence SEQ ID No. 8, 10 et 12.

Dans la présente description, les termes « se lier » et « se fixer » ont la même signification et sont interchangeables.

Dans la présente description, les termes polypeptides, séquences polypeptidiques, peptides et protéines attachés aux composés anticorps ou à leur séquence sont interchangeables.

Il doit être compris ici que l'invention ne concerne pas les anticorps sous forme naturelle, c'est-à-dire qu'ils ne sont pas pris dans leur environnement naturel mais qu'ils ont pu être isolés ou obtenus par purification à partir de sources naturelles, ou bien obtenus par recombinaison génétique, ou par synthèse chimique, et qu'ils peuvent alors comporter des acides aminés non naturels comme cela sera décrit plus loin.

Par région CDR ou CDR, on entend désigner les régions hypervariables des chaînes lourdes et légères des immunoglobulines comme définies par Kabat et al. (Kabat et al., Sequences of proteins of immunological interest, 5th Ed., U.S. Department of Health and Human Services, NIH, 1991, and later editions). Il existe 3 CDRs de chaîne lourde et 3 CDRs de chaîne légère. Le terme CDR ou CDRs est utilisé ici pour désigner suivant les cas, l'une de ces régions ou plusieurs, voire l'ensemble, de ces régions qui contiennent la majorité des résidus d'acide aminés responsables de la liaison par affinité de l'anticorps pour l'antigène ou l'épitope qu'il reconnaît.

Par « pourcentage d'identité » entre deux séquences d'acide nucléique ou d'acide aminé au sens de la présente invention, on entend désigner un pourcentage de nucléotides ou de résidus d'acides aminés identiques entre les deux séquences à comparer, obtenu après le meilleur alignement (alignement optimal), ce pourcentage étant purement statistique et les différences entre les deux séquences étant réparties au hasard et sur toute leur longueur. Les comparaisons de séquences entre deux séquences d'acide nucléique ou d'acide aminé sont traditionnellement réalisées en comparant ces séquences après les avoir alignées de manière optimale, ladite comparaison pouvant être réalisée par segment ou par « fenêtre de comparaison ». L'alignement optimal des séquences pour la comparaison peut être réalisé, outre manuellement, au moyen de l'algorithme d'homologie locale de Smith et Waterman (1981) [Ad. App. Math. 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N ou BLAST P).

Le pourcentage d'identité entre deux séquences d'acide nucléique ou d'acide aminé est déterminé en comparant ces deux séquences alignées de manière optimale dans laquelle la séquence d'acide nucléique ou d'acide aminé à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par exemple, on pourra utiliser le programme BLAST, « BLAST 2 sequences » (Tatusova et al., "Blast 2 sequences - a new tool for comparing protein and nucleotide sequences", FEMS Microbiol Lett. 174:247-250) disponible sur le site http://www.ncbi.nlm.nih.gov/gorf/bl2.html, les paramètres utilisés étant ceux donnés par défaut (en particulier pour les paramètres « open gap penaltie » : 5, et « extension gap penaltie » : 2 ; la matrice choisie étant par exemple la matrice « BLOSUM 62 » proposée par le programme), le pourcentage d'identité entre les deux séquences à comparer étant calculé directement par le programme.

Par séquence d'acide aminé présentant au moins 80 %, de préférence 85 %, 90 %, 95 % et 98 % d'identité avec une séquences d'acide aminé de référence, on préfère celles présentant par rapport à la séquence de référence, certaines modifications, en particulier une délétion, addition ou substitution d'au moins un acide aminé, une troncation ou un allongement. Dans le cas d'une substitution, d'un ou plusieurs acide(s) aminé(s) consécutif(s) ou non consécutif(s), on préfère les substitutions dans lesquelles les acides aminés substitués sont remplacés par des acides aminés « équivalents ». L'expression « acides aminés équivalents » vise ici à désigner tout acide aminé susceptible d'être substitué à l'un des acides aminés de la structure de base sans cependant modifier essentiellement les activités biologiques des anticorps correspondants et telles qu'elles seront définies par la suite, notamment dans les exemples.

Ces acides aminés équivalents peuvent être déterminés soit en s'appuyant sur leur homologie de structure avec les acides aminés auxquels ils se substituent, soit sur des résultats d'essais comparatifs d'activité biologique entre les différents anticorps susceptibles d'être effectués.

A titre d'exemple, on mentionne les possibilités de substitution susceptibles d'être effectuées sans qu'il résulte en une modification approfondie de l'activité biologique de l'anticorps modifié correspondant. On peut remplacer ainsi la leucine par la valine ou l'isoleucine, l'acide aspartique par l'acide glutamine, la glutamine par l'asparagine, l'arginine par la lysine, etc., les substitutions inverses étant naturellement envisageables dans les mêmes conditions.

Les anticorps selon la présente invention sont de préférence des anticorps monoclonaux spécifiques, notamment d'origine murine, chimériques ou humanisés qui pourront être obtenus selon les méthodes standards bien connues de l'homme de l'art.

En général, pour la préparation d'anticorps monoclonaux ou leurs fragments fonctionnels, notamment d'origine murine, on pourra se référer aux techniques qui sont en particulier décrites dans le manuel « Antibodies » (Harlow and Lane, Antibodies: A Laboratory Manual, Cold Spring Harbor Laboratory, Cold Spring Harbor NY, pp. 726, 1988) ou à la technique de préparation à partir d'hybridomes décrite par Kohler et Milstein (Nature, 256:495-497, 1975).

Les anticorps monoclonaux selon l'invention peuvent être obtenus par exemple à partir de cellule d'un animal immunisé contre le récepteur IGF-IR, ou un de ses fragments comportant l'épitope reconnu spécifiquement par lesdits anticorps monoclonaux selon l'invention. Ledit récepteur IGF-IR, ou un de sesdits fragments, pourra notamment être produit selon les modes opératoires usuels, par recombinaison génétique à partir d'une séquence d'acide nucléique contenue dans la séquence de l'ADNc codant pour le récepteur IGF-IR ou par synthèse peptidique à partir d'une séquence d'acides aminés comprise dans la séquence peptidique du récepteur IGF-IR.

Les anticorps monoclonaux selon l'invention pourront par exemple être purifiés sur une colonne d'affinité sur laquelle a préalablement été immobilisé le récepteur IGF-IR ou un de ses fragments comportant l'épitope reconnu spécifiquement par lesdits anticorps monoclonaux selon l'invention. Plus particulièrement, lesdits anticorps monoclonaux pourront être purifiés par chromatographie sur protéine A et/ou G, suivie ou non par une chromatographie par échange d'ions visant à éliminer les contaminants protéiques résiduels ainsi que l'ADN et les LPS, elle-même suivie ou non par une chromatographie d'exclusion sur gel de sépharose afin d'éliminer les agrégats potentiels dus à la présence de dimères ou d'autres multimères. De manière encore plus préférée, l'ensemble de ces techniques peut être utilisé simultanément ou successivement.

Sont également compris par anticorps selon la présente invention, les anticorps chimériques ou humanisés.

Par anticorps chimérique, on entend désigner un anticorps qui contient une région variable (chaîne légère et chaîne lourde) naturelle dérivée d'un anticorps d'une espèce donnée en association avec les régions constantes de chaîne légère et chaîne lourde d'un anticorps d'une espèce hétérologue à ladite espèce donnée.

Les anticorps ou leurs fragments de type chimérique selon l'invention peuvent être préparés en utilisant les techniques de recombinaison génétique. Par exemple, l'anticorps chimérique pourra être réalisé en clonant un ADN recombinant comportant un promoteur et une séquence codant pour la région variable d'un anticorps monoclonal non humain, notamment murin, selon l'invention et une séquence codant pour la région constante d'anticorps humain. Un anticorps chimérique de l'invention codé par un tel gène recombinant sera par exemple une chimère souris-homme, la spécificité de cet anticorps étant déterminé par la région variable dérivée de l'ADN murin et son isotype déterminé par la région constante dérivée de l'ADN humain. Pour les méthodes de préparation d'anticorps chimériques, on pourra par exemple se référer au document Verhoeyn et al. (BioEssays, 8:74, 1988).

Par anticorps humanisés, on entend désigner un anticorps qui contient des régions CDRs dérivées d'un anticorps d'origine non humaine, les autres parties de la molécule d'anticorps étant dérivée d'un (ou de plusieurs) anticorps humains. En outre, certains des résidus des segments du squelette (dénommés FR) peuvent être modifiés pour conserver l'affinité de liaison (Jones et al., Nature, 321:522-525, 1986 ; Verhoeyen et al., Science, 239:1534-1536, 1988 ; Riechmann et al., Nature, 332:323-327, 1988).

Les anticorps humanisés selon l'invention ou leurs fragments peuvent être préparés par des techniques connues de l'homme de l'art (comme par exemple celles décrites dans les documents Singer et al., J. Immun. 150:2844-2857, 1992 ; Mountain et al., Biotechnol. Genet. Eng. Rev., 10:1-142, 1992 ; ou Bebbington et al., Bio/Technology, 10:169-175, 1992). De tels anticorps humanisés selon l'invention sont préférés pour leur utilisation dans des méthodes de diagnostic *in vitro,* ou de traitement prophylactique et/ou thérapeutique *in vivo.*

Par fragment fonctionnel d'un anticorps selon l'invention, on entend désigner en particulier un fragment d'anticorps, tel que des fragments Fv, scFv (sc pour simple chaîne), Fab, F(ab')₂, Fab', scFv-Fc ou diabodies, ou tout fragment dont la durée de demie-vie aurait été augmentée par modification chimique, comme l'ajout de poly(alkylène) glycol tel que le poly(éthylène)glycol ("PEGylation") (fragments pégylés dénommés Fv-PEG, scFv-PEG, Fab-PEG, (Fab')₂-PEG ou Fab'-PEG) (« PEG » pour Poly(Ethylène)Glycol), ou par incorporation dans un liposome, lesdits fragments présentant au moins un des CDRs de séquence SEQ ID No. 2, 4, 6, 8, 10 ou 12 caractéristiques selon l'invention, et, notamment, en ce qu'il est capable d'exercer de manière générale une activité même partielle de l'anticorps dont il est issu, telle qu'en particulier la capacité à reconnaître et à se lier au récepteur IGF-IR, et, le cas échéant, à inhiber l'activité du récepteur IGF-IR.

De préférence, lesdits fragments fonctionnels seront constitués ou comprendront une séquence partielle de la chaîne variable lourde ou légère de l'anticorps dont ils sont dérivés, ladite séquence partielle étant suffisante pour retenir la même spécificité de liaison que l'anticorps dont elle est issue et une affinité suffisante, de préférence au moins égale à 1/100, de manière plus préférée à au moins 1/10 de celle de l'anticorps dont elle est issue, vis-à-vis du récepteur IGF-IR.

Un tel fragment fonctionnel comportera au minimum 5 acides aminés, de préférence 10, 15, 25, 50 et 100 acides aminés consécutifs de la séquence de l'anticorps dont il est issu.

De préférence, ces fragments fonctionnels seront des fragments de type Fv, scFv, Fab, F(ab')₂, F(ab'), scFv-Fc ou diabodies, qui possèdent généralement la même spécificité de fixation que l'anticorps dont ils sont issus. Selon la présente invention, des fragments d'anticorps de l'invention peuvent être obtenus à partir des anticorps tels que décrits précédemment par des méthodes telles que la digestion par des enzymes, comme la pepsine ou la papaïne et/ou par clivage des ponts disulfures par réduction chimique. D'une autre manière les fragments d'anticorps compris dans la présente invention peuvent être obtenus par des techniques de recombinaisons génétiques bien connues également de l'homme de l'art ou encore par synthèse peptidique au moyen par exemple de synthétiseurs automatiques de peptides tels que ceux fournis par la société Applied Biosystems, etc..

De manière plus préférée, l'invention comprend les anticorps, ou leurs fragments fonctionnels, selon la présente invention, notamment chimériques ou humanisés, obtenus par recombinaison génétique ou par synthèse chimique.

Dans un mode de réalisation préféré, l'invention a pour objet un anticorps, ou l'un de ses fragments fonctionnels, selon l'invention, caractérisé en ce qu'il comprend une chaîne lourde comprenant au moins un CDR de séquence SEQ ID No. 12 ou une séquence présentant au moins 80 % d'identité après alignement optimal avec la séquence SEQ ID No. 12.

Parmi les six courtes séquences de CDR, le troisième CDR de la chaîne lourde (CDRH3) a une plus grande variabilité de taille (grande diversité essentiellement due aux mécanismes d'arrangement des gènes qui lui donnent naissance). Il peut être aussi court que 2 acides aminés alors que la taille la plus longue connue est de 26. Fonctionnellement, le CDRH3 joue un rôle à part dans la détermination de la spécificité de l'anticorps (Segal et al., PNAS, 71:4298-4302, 1974 ; Amit et al., Science, 233:747-753, 1986 ; Chothia et al., J. Mol. Biol., 196:901-917, 1987 ; Chothia et al., Nature, 342:877-883, 1989 ; Caton et al., J. Immunol., 144:1965-1968, 1990 ; Sharon et al., PNAS, 87:4814-4817, 1990 ; Sharon et al., J. Immnuol., 144:4863-4869, 1990 ; Kabat et al., J. Immunol, 147:1709-1719, 1991).

Il est connu que seul un faible pourcentage des acides aminés des CDRs contribue à la construction de site de liaison de l'anticorps, mais ces résidus doivent être maintenus dans une conformation tridimensionnelle très spécifique.

De manière plus préférée, la présente invention est relative à un anticorps, ou l'un de ses fragments fonctionnels, selon l'invention, caractérisé en ce qu'il comprend une chaîne lourde comprenant au moins deux des trois CDRs ou les trois CDRs de séquence SEQ ID Nos. 8, 10 et 12, ou au moins deux de trois CDRs ou trois CDRs de séquence présentant respectivement au moins 80 % d'identité après alignement optimal avec la séquence SEQ ID No. 8, 10 et 12.

Dans un mode de réalisation également préféré, l'invention a pour objet un anticorps, ou l'un de ses fragments fonctionnels, selon l'invention, caractérisé en ce qu'il comprend une chaîne légère comprenant au moins un CDR choisi parmi les CDRs de séquence SEQ ID No. 2, 4 ou 6, ou un CDR dont la séquence présente au moins 80 % d'identité après alignement optimal avec la séquence SEQ ID No. 2, 4 ou 6.

Dans un mode de réalisation plus préféré, l'invention a pour objet un anticorps, ou l'un de ses fragments fonctionnels selon l'invention, caractérisé en ce qu'il comprend une chaîne légère comprenant au moins deux des trois CDRs ou les trois CDRs de séquence SEQ ID Nos. 2, 4 et 6, ou au moins deux de trois CDRs ou trois CDRs de séquence présentant respectivement au moins 80 % d'identité après alignement optimal avec la séquence SEQ ID No. 2, 4 et 6.

De manière la plus préférée, l'anticorps, ou l'un de ses fragments fonctionnels selon l'invention, est caractérisé en ce qu'il comprend une chaîne lourde comprenant les trois CDRs de séquence SEQ ID Nos. 8, 10 et 12, ou trois CDRs de séquence présentant respectivement au moins 80 % d'identité après alignement optimal avec la séquence SEQ ID No. 8, 10 et 12 et en ce qu'il comprend en outre une chaîne légère comprenant les trois CDRs de séquence SEQ ID Nos. 2, 4 et 6, ou trois CDRs de séquence présentant respectivement au moins 80 % d'identité après alignement optimal avec la séquence SEQ ID No. 2, 4 et 6.

Sous un autre aspect, la présente invention a pour objet un anticorps, ou l'un de ses fragments fonctionnels, selon l'invention, caractérisé en ce qu'il ne se fixe pas ou qu'il ne se fixe pas de manière significative au récepteur humain IR de l'insuline.

De manière préférée, lesdits fragments fonctionnels selon la présente invention seront choisis parmi les fragments Fv, scFv, Fab, (Fab')₂, Fab', scFv-Fc ou diabodies, ou tout fragment fonctionnel dont la demie vie aurait été augmentée par une modification chimique, notamment par PEGylation, ou par l'incorporation dans un liposome.

Sous un autre aspect, l'invention est relative à un hybridome murin capable de sécréter un anticorps monoclonal selon la présente invention, notamment l'hybridome d'origine murine tel que déposé au Centre National de Culture de Microorganisme (CNCM) (Institut Pasteur, Paris, France) le 19 septembre 2001 sous le numéro I-2717.

L'anticorps monoclonal dénommé ici 7C10, ou l'un de ses fragments fonctionnels, caractérisé en ce que ledit anticorps est sécrété par l'hybridome déposé à la CNCM le 19 septembre 2001 sous le numéro I-2717 fait bien entendu partie de la présente invention.

Dans un mode de réalisation particulier, la présente invention est relative à un anticorps murin, ou l'un de ses fragments fonctionnels, selon l'invention, caractérisé en ce que ledit anticorps comprend une chaîne légère de séquence comprenant la séquence d'acide aminé SEQ ID No. 54, ou une séquence présentant au moins 80 % d'identité après alignement optimale avec la séquence SEQ ID No. 54, ou/et en ce qu'il comprend une chaîne lourde de séquence comprenant la séquence d'acide aminé SEQ ID No. 69, ou une séquence présentant au moins 80 % d'identité après alignement optimal avec la séquence SEQ ID No. 69.

Sous un aspect également particulier, la présente invention est relative à un anticorps chimérique, ou l'un de ses fragments fonctionnels, selon l'invention, caractérisé en ce que ledit anticorps comprend en outre les régions constantes de chaîne légère et de chaîne lourde dérivées d'un anticorps d'une espèce hétérologue à la souris, notamment de l'Homme, et de manière préférée, en ce que les régions constantes de chaîne légère et de chaîne lourde dérivées d'un anticorps humain sont respectivement la région kappa et, gamma-1, gamma-2 ou gamma-4.

Sous un aspect également particulier, la présente invention est relative à un anticorps humanisé, ou l'un de ses fragments fonctionnels, selon l'invention, caractérisé en ce que ledit anticorps comprend une chaîne légère et/ou une chaîne lourde dans lesquelles les segments de squelette FR1 à FR4 (tels que définis ci-après dans les exemples 12 et 13, aux tableaux 5 et 6) de ladite chaîne légère et/ou chaîne lourde sont dérivés respectivement de segments de squelette FR1 à FR4 de chaîne légère et/ou de chaîne lourde d'anticorps humains.

Selon un mode de réalisation préféré, l'anticorps humanisé, ou l'un de ses fragments fonctionnels, selon la présente invention est caractérisé en ce que ledit anticorps humanisé comprend une chaîne légère comprenant la séquence d'acide aminé SEQ ID No. 61 ou 65, ou une séquence présentant au moins 80 % d'identité après alignement optimal avec la séquence SEQ ID No. 61 ou 65, ou/et en ce qu'il comprend une chaîne lourde comprenant la séquence d'acide aminé SEQ ID No. 75, 79 ou 83, ou une séquence présentant au moins 80 % d'identité après alignement optimal avec la séquence SEQ ID No. 75, 79 ou 83.

De préférence, l'anticorps humanisé, ou l'un de ses fragments fonctionnels, selon l'invention est caractérisé en ce que ledit anticorps humanisé comprend une chaîne légère comprenant la séquence d'acide aminé SEQ ID No. 65, et en ce qu'il comprend une chaîne lourde de séquence comprenant la séquence d'acide aminé SEQ ID No. 79 ou 83, de préférence SEQ ID No. 83.

Sous un nouvel aspect, la présente invention est relative à un acide nucléique isolé caractérisé en ce qu'il est choisi parmi les acides nucléiques suivants :
a) un acide nucléique, ADN ou ARN, codant pour un anticorps, ou l'un de ses fragments fonctionnels, selon l'invention ;
b) un acide nucléique complémentaire d'un acide nucléique tel que défini en a) ; et
c) un acide nucléique d'au moins 18 nucléotides capable d'hybrider dans des conditions de forte stringence avec au moins l'un des CDRs de séquence d'acide nucléique SEQ ID No. 1, 3, 5, 7, 9 ou 11, ou avec une séquence présentant au moins 80 %, de préférence 85 %, 90 %, 95 % et 98 %, d'identité après alignement optimal avec la séquence SEQ ID No. 1, 3, 5, 7, 9 ou 11.

Par acide nucléique, séquence nucléique ou d'acide nucléique, polynucléotide, oligonucléotide, séquence de polynucléotide, séquence nucléotidique, termes qui seront employés indifféremment dans la présente description, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADNs.

Il doit être aussi compris ici que la présente invention ne concerne pas les séquences nucléotidiques dans leur environnement chromosomique naturel, c'est-à-dire à l'état naturel. Il s'agit de séquences qui ont été isolées et/ou purifiées, c'est-à-dire qu'elles ont été prélevées directement ou indirectement, par exemple par copie, leur environnement ayant été au moins partiellement modifié. On entend ainsi également désigner ici les acides nucléiques isolés obtenus par recombinaison génétique au moyen par exemple de cellules hôtes ou obtenus par synthèse chimique.

Par séquences nucléiques présentant un pourcentage d'identité d'au moins 80 %, de préférence 85 %, 90 %, 95 % et 98 %, après alignement optimal avec une séquence de préférence, on entend désigner les séquences nucléiques présentant, par rapport à la séquence nucléique de référence, certaines modifications comme en particulier une délétion, une troncation, un allongement, une fusion chimérique et/ou une substitution, notamment ponctuelle. Il s'agit de préférence de séquences dont les séquences codent pour les mêmes séquences d'acide aminés que la séquence de référence, ceci lié à la dégénérescence du code génétique, ou de séquences complémentaires qui sont susceptibles de s'hybrider spécifiquement avec les séquences de référence de préférence dans des conditions de forte stringence notamment telles que définies ci-après.

Une hybridation dans des conditions de forte stringence signifie que les conditions de température et de force ionique sont choisies de telle manière qu'elles permettent le maintien de l'hybridation entre deux fragments d'ADN complémentaires. A titre illustratif, des conditions de forte stringence de l'étape d'hybridation aux fins de définir les fragments polynucléotidiques décrits ci-dessus, sont avantageusement les suivantes.

L'hybridation ADN-ADN ou ADN-ARN est réalisée en deux étapes : (1) préhybridation à 42°C pendant 3 heures en tampon phosphate (20 mM, pH 7,5) contenant 5 x SSC (1 x SSC correspond à une solution 0,15 M NaCl + 0,015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille > 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille > 100 nucléotides. Les conditions d'hybridation de forte stringence décrites ci-dessus pour un polynucléotide de taille définie, peuvent être adaptées par l'homme du métier pour des oligonucléotides de taille plus grande ou plus petite, selon l'enseignement de Sambrook et al., (1989, Molecular cloning : a laboratory manual. 2nd Ed. Cold Spring Harbor).

L'invention est également relative à un vecteur comprenant un acide nucléique selon la présente invention.

L'invention vise notamment les vecteurs de clonage et/ou d'expression qui contiennent une séquence nucléotidique selon l'invention.

Les vecteurs selon l'invention comportent de préférence des éléments qui permettent l'expression et/ou la sécrétion des séquences nucléotidiques dans une cellule hôte déterminée. Le vecteur doit alors comporter un promoteur, des signaux d'initiation et de terminaison de la traduction, ainsi que des régions appropriées de régulation de la transcription. Il doit pouvoir être maintenu de façon stable dans la cellule hôte et peut éventuellement posséder des signaux particuliers qui spécifient la sécrétion de la protéine traduite. Ces différents éléments sont choisis et optimisés par l'homme du métier en fonction de l'hôte cellulaire utilisé. A cet effet, les séquences nucléotidiques selon l'invention peuvent être insérées dans des vecteurs à réplication autonome au sein de l'hôte choisi, ou être des vecteurs intégratifs de l'hôte choisi.

De tels vecteurs sont préparés par des méthodes couramment utilisées par l'homme du métier, et les clones résultant peuvent être introduits dans un hôte approprié par des méthodes standard, telles que la lipofection, l'électroporation, le choc thermique, ou des méthodes chimiques.

Les vecteurs selon l'invention sont par exemple des vecteurs d'origine plasmidique ou virale. Ils sont utiles pour transformer des cellules hôtes afin de cloner ou d'exprimer les séquences nucléotidiques selon l'invention.

L'invention comprend également les cellules hôtes transformées par ou comprenant un vecteur selon l'invention.

L'hôte cellulaire peut être choisi parmi des systèmes procaryotes ou eucaryotes, par exemple les cellules bactériennes mais également les cellules de levure ou les cellules animales, en particulier les cellules de mammifères. On peut également utiliser des cellules d'insectes ou des cellules de plantes.

L'invention concerne également les animaux, excepté l'Homme, qui comprennent au moins une cellule transformée selon l'invention.

Sous un autre aspect, l'invention a pour objet un procédé de production d'un anticorps, ou l'un de ses fragments fonctionnels selon l'invention, caractérisé en ce qu'il comprend les étapes suivantes :
a) la culture dans un milieu et conditions de culture appropriés d'une cellule hôte selon l'invention ; et
b) la récupération desdits anticorps, ou l'un de ses fragments fonctionnels, ainsi produits à partir du milieu de culture ou desdites cellules cultivées.

Les cellules transformées selon l'invention sont utilisables dans des procédés de préparation de polypeptides recombinants selon l'invention. Les procédés de préparation d'un polypeptide selon l'invention sous forme recombinante, caractérisés en ce qu'ils mettent en oeuvre un vecteur et/ou une cellule transformée par un vecteur selon l'invention sont eux-mêmes compris dans la présente invention. De préférence, on cultive une cellule transformée par un vecteur selon l'invention dans des conditions qui permettent l'expression dudit polypeptide et on récupère ledit peptide recombinant.

Ainsi qu'il a été dit, l'hôte cellulaire peut être choisi parmi des systèmes procaryotes ou eucaryotes. En particulier, il est possible d'identifier des séquences nucléotidiques selon l'invention, facilitant la sécrétion dans un tel système procaryote ou eucaryote. Un vecteur selon l'invention portant une telle séquence peut donc être avantageusement utilisé pour la production de protéines recombinantes, destinées à être sécrétées. En effet, la purification de ces protéines recombinantes d'intérêt sera facilitée par le fait qu'elles sont présentes dans le surnageant de la culture cellulaire plutôt qu'à l'intérieur des cellules hôtes.

On peut également préparer les polypeptides selon l'invention par synthèse chimique. Un tel procédé de préparation est également un objet de l'invention. L'homme du métier connaît les procédés de synthèse chimique, par exemple les techniques mettant en oeuvre des phases solides (voir notamment Steward et al., 1984, Solid phase peptides synthesis, Pierce Chem. Company, Rockford, 111, 2ème éd., (1984)) ou des techniques utilisant des phases solides partielles, par condensation de fragments ou par une synthèse en solution classique. Les polypeptides obtenus par synthèse chimique et pouvant comporter des acides aminés non naturels correspondants sont également compris dans l'invention.

Les anticorps, ou l'un de leurs fragments fonctionnels, susceptibles d'être obtenus par un procédé selon l'invention sont également compris dans la présente invention.

Selon un deuxième forme d'exécution, la présente invention concerne un anticorps selon l'invention tel que décrit plus haut, caractérisé en ce qu'il est, en outre, capable de se lier spécifiquement sur le récepteur humain du facteur de croissance de l'épiderme EGFR et/ou capable d'inhiber spécifiquement l'activité tyrosine kinase dudit récepteur EGFR.

De manière générale, les facteurs de croissance sont de petites protéines impliquées dans la régulation de la prolifération et de la différenciation des cellules normales. Certains de ces facteurs de croissance jouent également un rôle important dans l'initiation et la maintenance de la transformation cellulaire, pouvant fonctionner comme facteurs autocrines ou paracrines. C'est notamment le cas, outre de l'IGF1 décrit plus haut, du facteur de croissance de l'épiderme EGF (EGF pour " Epidermal Growth Factor ") qui semble particulièrement impliqué dans l'apparition du phénotype tumoral, la progression des tumeurs et la génération des métastases.

L'EGF et l'lGf1 exercent leur action par l'intermédiaire de leur récepteur respectif dénommé ici EGFR et IGF-IR. Il s'agit dans les deux cas de récepteurs membranaires à activité tyrosine kinase dont la surexpression est décrite dans de nombreux cancers. Il faut cependant noter que l'interaction de ces deux récepteurs n'est pas clairement établie et que les études effectuées par diverses équipes à ce propos donnent des résultats contradictoires quant à la collaboration de ces deux récepteurs.

Des travaux effectués sur des cellules de tumeurs de la prostate montrent que l'interruption de la boucle autocrine EGF/EGFR par un anticorps monoclonal (dénommé ici "ACM" ou "AcM") anti-EGFR se traduit par une perte complète de la réponse des cellules DU145 à l'IGF1 (Connolly J.M. and Rose D.P., Prostate, Apr. 24(4):167-75, 1994 ; Putz T. et al., Cancer Res., Jan. 1, 59(1):227-33, 1999). Ces résultats suggéreraient qu'un blocage du récepteur à l'EGF serait suffisant pour obtenir une inhibition totale des signaux de transformation générés par l'activation des deux récepteurs (EGFR et IGF-IR). En revanche, d'autres études (Pietrzkowski et al., Cell Growth Differ, Apr., 3(4):199-205, 1992 ; Coppola et al., Mol Cell Biol., Jul., 14(7):4588-95, 1994) ont montré qu'une surexpression de l'EGFR nécessite la présence d'un IGF-IR fonctionnel pour exercer son potentiel mitogénique et transformant, alors que l'IGF-IR ne nécessite pas, pour sa part, la présence d'EGFR fonctionnel pour médier son action. Cette seconde série d'études serait plus en accord avec une stratégie tendant à bloquer préférentiellement l'IGF-IR dans le but d'atteindre simultanément les deux récepteurs.

De manière surprenante, les inventeurs ont, dans un premier temps, mis ainsi en évidence qu'une co-inhibition de la fixation de l'IGF1 et/ou IGF2 sur le récepteur IGF-IR et de la fixation de l'EGF sur le récepteur EGFR permettait d'obtenir une synergie d'action significative de ces deux actions contre la croissance tumorale *in vivo* chez des souris nude porteuses d'une tumeur exprimant ces deux récepteurs. Une des hypothèses les plus probables pouvant expliquer cette synergie d'action est que les deux facteurs de croissance EGF et IGF1 (et/ou IGF2) agissent eux-mêmes en synergie dans la transformation de cellules normales en cellules à caractère tumoral et/ou dans la croissance et/ou la prolifération de cellules tumorales pour certaines tumeurs, notamment pour celles surexprimant les deux récepteurs EGFR et IGF-IR et/ou présentant une suractivation du signal de transduction médié par ces deux récepteurs, en particulier au niveau de l'activité tyrosine kinase de ces récepteurs.

Selon un aspect préféré de cette forme d'exécution, l'invention concerne un anticorps tel que décrit plus haut, caractérisé en ce qu'il consiste en un anticorps bispécifique comprenant un second motif inhibant spécifiquement la fixation de l'EGF sur l'EGFR et/ou inhibant spécifiquement l'activité tyrosine kinase dudit récepteur EGFR.

Par le terme « second motif », on entend désigner ci-avant notamment une séquence d'acides aminés comprenant un fragment capable de se lier spécifiquement à l'EGFR, en particulier une région CDR d'une chaîne variable d'un anticorps anti-EGFR, ou l'un des fragments de cette région CDR de longueur suffisante pour exercer cette liaison spécifique, ou encore plusieurs régions CDR d'un anticorps anti-EGFR.

Les anticorps bispécifiques ou bifonctionnels constituent une seconde génération d'anticorps monoclonaux dans lesquelles deux régions variable différentes sont combinées dans la même molécule (Hollinger and Bohlen 1999 Cancer and metastasis rev. 18 : 411-419). Leur utilité a été mise en évidence tant dans le domaine du diagnostic que dans le domaine de la thérapie de part leur capacité à recruter de nouvelles fonctions effectrices ou à cibler plusieurs molécules à la surface de cellules tumorales. Ces anticorps peuvent être obtenus par des méthodes chimiques (Glennie MJ et al. 1987 J. Immunol. 139, 2367-2375 ; Repp R. et al. 1995 J. Hemat. 377-382) ou somatiques (Staerz U.D. and Bevan M.J. 1986 PNAS 83, 1453-1457 ; Suresh M.R. et al. 1986 Method Enzymol. 121 : 210-228) mais également et préférentiellement par des techniques d'ingénierie génétique qui permettent de forcer l'héterodimérisation et facilitent ainsi le procédé de purification de l'anticorps recherché (Merchand et al. 1998 Nature Biotech. 16 : 677-681).

Ces anticorps bispécifiques peuvent être construits comme des IgG entières, comme des Fab'2 bispécifiques, comme des Fab'PEG ou comme des diabodies ou encore comme des scFv bispécifiques mais également comme un anticorps bispécifique tétravalent ou deux sites de fixation sont présents pour chaque antigène ciblé (Park et al. 2000 Mol. Immunol. 37(18) : 1123-30) ou ses fragments comme décrit plus haut.

Outre un avantage économique du fait que la production et l'administration d'un anticorps bispécifique soit moins onéreuse que le production de deux anticorps spécifiques, l'utilisation de tels anticorps bispécifiques a pour avantage de réduire la toxicité du traitement. En effet, l'utilisation d'un anticorps bispécifique permet de diminuer la quantité globale d'anticorps circulants et, par suite, la toxicité éventuelle.

Dans une forme de réalisation préférée de l'invention, l'anticorps bispécifique est un anticorps bivalent ou tetravalent.

Dans la pratique, l'intérêt d'utiliser un anticorps bispécifique tétravalent est qu'il présente une avidité plus importante par rapport à un anticorps bivalent du fait de la présence de deux sites de fixation pour chaque cible, respectivement IGF-IR et EGFR dans la présente invention.

De manière similaire à la sélection des fragments fonctionnels de l'anticorps anti-IGF-IR décrit plus haut, ledit second motif est sélectionné parmi les fragments Fv, Fab, (Fab')₂, Fab', scFv, scFv-Fc et les diabodies, ou toute forme dont la demie-vie aurait été augmentée comme les fragments pégylés tels que Fv-PEG, scFv-PEG, Fab-PEG, (Fab')₂-PEG ou Fab'-PEG. Selon un aspect encore plus préféré de l'invention, ledit second motif anti-EGFR est issu de l'anticorps monoclonal de souris 225, son dérivé chimérique souris-homme C225, ou un anticorps humanisé dérivé de cet anticorps 225.

Sous encore un autre aspect, l'invention a pour objet un anticorps, ou l'un de ses fragments fonctionnels, selon l'invention à titre de médicament, de préférence un anticorps humanisé tel que défini ci-avant. Par anticorps, il faut comprendre, pour la suite de la présente description, aussi bien un anticorps anti-IGF-IR qu'un anticorps bispécifique anti IGF-IR / EGFR.

L'invention concerne également une composition pharmaceutique comprenant à titre de principe actif un composé consistant en un anticorps, ou l'un de ses fragments fonctionnels, selon l'invention, de préférence additionné d'un excipient et/ou d'un véhicule pharmaceutiquement acceptable.

Selon encore une autre forme d'exécution, la présente invention concerne également une composition pharmaceutique telle que décrite plus haut qui comprend un deuxième composé choisi parmi les composés capables d'inhiber spécifiquement la fixation de l'EGF sur le récepteur humain du facteur de croissance de l'épiderme EGFR et/ou capable d'inhiber spécifiquement l'activité tyrosine kinase dudit récepteur EGFR.

Dans un aspect préféré de l'invention, ledit deuxième composé est choisi parmi les anticorps anti-EGFR isolés, ou leurs fragments fonctionnels, capables d'inhiber par compétition la fixation de l'EGF sur l'EGFR. Plus particulièrement, ledit anticorps anti-EGFR est choisi parmi les anticorps anti-EGFR monoclonaux, chimériques ou humanisés, ou leurs fragments fonctionnels. Encore plus particulièrement, lesdits fragments fonctionnels de l'anticorps anti-EGFR sont choisis parmi les fragments Fv, Fab, (Fab')2, Fab', scFv-Fc et les diabodies, ou tout fragment dont la demie-vie aurait été augmentée, comme des fragments pégylés. Ledit anticorps peut consister, de manière encore plus préférée, en l'anticorps monoclonal de souris 225, son dérivé chimérique souris-homme C225 (dénommé encore IMC-C225) ou un anticorps humanisé dérivé de cet anticorps 225.

Une autre forme d'exécution complémentaire de l'invention consiste en une composition telle que décrite plus haut qui comprend, en outre, comme produit de combinaison pour une utilisation simultanée, séparée ou étalée dans le temps, un agent cytotoxique/cytostatique et/ou un inhibiteur de l'activité tyrosine kinase respectivement des récepteurs à l'IGF-I et/ou à l'EGF.

On entend par "utilisation simultanée", l'administration des deux composés de la composition selon l'invention compris dans une seule et même forme pharmaceutique.

On entend par "utilisation séparée", l'administration, en même temps, des deux composés de la composition selon l'invention, compris dans des formes pharmaceutiques distinctes.

On entend par "utilisation étalée dans le temps", l'administration successive des deux composés de la composition selon l'invention, compris chacun dans une forme pharmaceutique distincte.

D'une façon générale, la composition selon l'invention augmente considérablement l'efficacité du traitement du cancer. En d'autres termes, l'effet thérapeutique de l'anticorps anti-IGF-IR selon l'invention est potentialisé de manière inattendue par l'administration d'un agent cytotoxique. Un autre avantage subséquent majeur produit par une composition selon l'invention, concerne la possibilité d'utiliser des doses efficaces en principe actif plus faibles, ce qui permet d'éviter ou de réduire les risques d'apparition des effets secondaires, en particulier l'effet de l'agent cytotoxique. De plus, cette composition selon l'invention permettrait d'atteindre l'effet thérapeutique escompté plus rapidement.

Dans un mode de réalisation particulièrement préféré, ladite composition comme produit de combinaison selon l'invention est caractérisée en ce que ledit agent cytotoxique/cytostatique est choisi parmi les agents interagissant avec l'ADN, les antimétabolites, les inhibiteurs de topoisomérases I ou II, ou encore les agents inhibiteurs ou stabilisateurs du fuseau ou encore tout agent susceptible d'être utilisé en chimiothérapie. De tels agents cytotoxiques/cytostatiques, pour chacune des classes d'agents cytotoxiques précitées, sont par exemple cités dans l'édition 2001 du VIDAL, à la page consacrée aux composés attachés à la cancérologie et l'hématologie colonne « Cytotoxiques », ces composés cytotoxiques cités par référence à ce document sont cités ici comme agents cytotoxiques préférés.

Dans un mode de réalisation particulièrement préféré, ladite composition comme produit de combinaison selon l'invention est caractérisée en ce que ledit agent cytotoxique est couplé chimiquement audit anticorps pour une utilisation simultanée.

Dans un mode de réalisation particulièrement préféré, ladite composition selon l'invention est caractérisée en ce que ledit agent cytotoxique/cytostatique est choisi parmi les agents inhibiteurs ou stabilisateurs du fuseau, de préférence la vinorelbine et/ou la vinflunine et/ou la vincristine.

Afin de faciliter le couplage entre ledit agent cytotoxique et ledit anticorps selon l'invention, on pourra notamment introduire des molécules espaceurs entre les deux composés à coupler, telles que des poly(alkylènes)glycols comme le polyéthylèneglycol, ou encore des acides aminés, ou, dans un autre mode de réalisation, utiliser des dérivés actifs desdits agents cytotoxiques dans lesquels auront été introduites des fonctions capables de réagir avec ledit anticorps selon l'invention. Ces techniques de couplage sont bien connues de l'homme de l'art et ne seront pas développées dans la présente description.

Dans un autre mode de réalisation préféré, ledit inhibiteur de l'activité tyrosine kinase des récepteurs à l'IGF-I et/ou à l'EGF est sélectionné dans le groupe consistant en des agents naturels dérivés, des dianilinophtalimides, des pyrazolo- ou pyrrolo-pyridopyrimidines ou encore des quinazilines. De tels agents inhibiteurs sont bien connus de l'homme de l'art et décrit dans la littérature (Ciardiello F., Drugs 2000, Suppl. 1, 25-32).

D'autres inhibiteurs de l'EGFR peuvent consister en, sans aucune limitation, les anticorps monoclonaux C225 et 22Mab anti-EGFR (ImClone Systems Incorporated), ABX-EGF (Abgenix/Cell Genesys), EMD-7200 (Merck KgaA) ou les composés ZD-1834, ZD-1838 et ZD-1839 (AstraZeneca), PKI-166 (Novartis), PKI-166/CGP-75166 (Novartis), PTK 787 (Novartis), CP 701 (Cephalon), leflunomide (Pharmacia/Sugen), CI-1033 (Warner Lambert Parke Davis), CI-1033/PD 183, 805 (Warner Lambert Parke Davis), CL-387, 785 (Wyeth-Ayerst), BBR-1611 (Boehringer Mannheim GmbH/Roche), Naamidine A (Bristol Myers Squibb), RC-3940-11 (Pharmacia), BIBX-1382 (Boehringer Ingelheim), OLX-103 (Merck & Co), VRCTC-310 (Ventech Research), EGF fusion toxin (Seragen Inc.), DAB-389 (Seragen/Lilgand), ZM-252808 (Impérial Cancer Research Fund), RG-50864 (INSERM), LFM-A12 (Parker Hughes Cancer Center), WHI-P97 (Parker Hughes Cancer Center), GW-282974 (Glaxo), KT-8391 (Kyowa Hakko) ou le » EGFR Vaccine » (York Medical/Centro de Immunologia Molecular.

Selon encore une autre forme d'exécution de l'invention, la composition telle que décrite plus haut, peut également comprendre un autre composé anticorps dirigé contre le domaine extracellulaire du récepteur HER2/neu, comme produit de combinaison pour une utilisation simultanée, séparée ou étalée dans le temps, destinée à la prévention et au traitement de cancer, notamment les cancers surexprimant ledit récepteur HER2/neu et le récepteur IGF-IR et/ou EGFR, comme notamment le cancer du sein.

On pourra notamment se référer aux publications de Albanell et al. (J. of the National Cancer Institute, 93(24):1830-1831, 2001) et de Lu et al. (J. of the National Cancer Institute, 93(24):1852-1857, 2001) justifiant l'intérêt inattendu d'associer un anticorps anti-HER2/neu avec un anticorps anti-IGF-IR selon la présente invention.

De manière particulière, ledit anticorps anti-HER2/neu de la composition selon l'invention est l'anticorps dénommé Trastuzumab (dénommé encore Herceptin).

L'invention est, sous un autre aspect, relative à une composition caractérisée en ce que l'un, au moins, desdits anticorps, ou l'un de leur fragment fonctionnel, est conjugué avec une toxine cellulaire et/ou un radioélément.

De préférence, ladite toxine ou ledit radioélément est capable d'inhiber au moins une activité cellulaire de cellules exprimant le récepteur IGF-IR et/ou EGFR, de manière plus préférée capable d'empêcher la croissance ou la prolifération de ladite cellule, notamment d'inactiver totalement ladite cellule.

De préférence encore, ladite toxine est une toxine d'entérobactéries, notamment l'exotoxine A de Pseudomonas.

Les radioéléments (ou radioisotopes) préférés conjugués aux anticorps employés pour la thérapie sont des radioisotopes émetteurs de rayons gamma et de préférence l'Iode¹³¹, l'Yttrium⁹⁰, l'Or¹⁹⁹, le Palladium¹⁰⁰, le Cuivre⁶⁷, le Bismuth²¹⁷ et l'Antimoine²¹¹. Les radioisotopes émetteurs de rayons béta et alpha peuvent également être utilisés pour la thérapie.

Par toxine ou radioélément conjugué à au moins un anticorps, ou l'un de leur fragment fonctionnel, selon l'invention, on entend désigner tout moyen permettant de lier ladite toxine ou ledit radioélément audit au moins un anticorps, notamment par couplage covalent entre les deux composés, avec ou sans introduction de molécule de liaison.

Parmi les agents permettant de lier de manière chimique (covalente), électrostatique, non covalente tout ou partie des composants du conjugué, on peut citer notamment la benzoquinone, la carbodiimide et plus particulièrement l'EDC (1-Ethyl-3[3-Diméthylaminopropyl]carbodiimide hydrochloride), la dimaléimide, l'acide dithio-bis-nitrobenzoïque (DTNB), le N-succinimidyl-S-acétyl-thioacétate (SATA), les agents pontants possédant un ou plusieurs groupements phénylazide réagissant avec les ultraviolets (U.V.) et de préférence le N-[-4-(azidosalicylamino)butyl]-3'-(2'-pyridyldithio)propionamide (APDP), le N-succinimidyl-3-(2-pyridyldithio)propionate (SPDP), le 6-hydrazinonicotimide (HYNIC).

Une autre forme de couplage, notamment pour les radioéléments, peut consister en l'utilisation d'un chélateur d'ions bi-fonctionnel.

Parmi ces chélates, on peut citer les chélates dérivés de l'EDTA (acide éthylènediamine-tetraacétique) ou du DTPA (acide diéthylènetriamine-pentaacétique) qui ont été développés pour lier les métaux, notamment les métaux radioactifs, et les immunoglobulines. Ainsi, le DTPA et ses dérivés peuvent substitués par différents groupes sur la chaîne carbonée afin d'augmenter la stabilité et la rigidité du complexe ligand-métal (Krejcarek et al. (1977) ; Brechbiel et al. (1991) ; Gansow (1991) ; brevet US 4 831 175).

Par exemple, l'acide diéthylènetriamine-pentaacétique (DTPA) et ses dérivés, qui sont depuis longtemps largement utilisés en médecine et en biologie soit sous leur forme libre, soit sous la forme de complexe avec un ion métallique, présentent la caractéristique remarquable de former des chélates stables avec les ions métalliques et être couplés avec des protéines d'intérêt thérapeutique ou diagnostique telles que des anticorps pour le développement de radioimmunoconjugués en thérapie du cancer (Meases et al., (1984) ; Gansow et al. (1990)).

De préférence également, ledit au moins un anticorps formant ledit conjugué selon l'invention est choisi parmi ses fragments fonctionnels, notamment les fragments amputés de leur composante Fc tels que les fragments scFv.

La présente invention comprend en outre l'utilisation de la composition selon l'invention pour la préparation d'un médicament.

Plus particulièrement, selon une autre forme d'exécution, l'invention concerne l'utilisation d'un anticorps, ou l'un de ses fragments fonctionnels, et/ou d'une composition pour la préparation d'un médicament destiné à la prévention ou au traitement d'une maladie induite par une surexpression et/ou une activation anormale du récepteur IGF-IR et/ou EGFR, et/ou liée à une hyperactivation de la voie de transduction du signal médié par l'interaction de 1-IGF1 ou IGF2 avec IGF-IR et/ou de l'EGF avec l'EGFR et/ou HER2/neu.

De préférence, ladite utilisation selon l'invention est caractérisée en ce que l'administration dudit médicament n'induit pas ou peu d'effets secondaires liés à une inhibition du récepteur IR de l'insuline, c'est-à-dire à une inhibition de l'interaction du récepteur IR avec ses ligands naturels due à la présence dudit médicament, notamment par une inhibition compétitive liée à la fixation dudit médicament sur l'IR.

La présente invention comprend en outre l'utilisation d'un anticorps, ou l'un de ses fragments fonctionnels, de préférence humanisé, et/ou d'une composition selon l'invention pour la préparation d'un médicament destiné à inhiber la transformation de cellules normales en cellules à caractère tumoral, de préférence IGF, notamment IGF1 et/ou IGF2, dépendante et/ou EGF dépendante et/ou HER2/neu dépendante.

La présente invention est relative également à l'utilisation d'un anticorps, ou l'un de ses fragments fonctionnels, de préférence humanisé, et/ou d'une composition selon l'invention pour la préparation d'un médicament destiné à inhiber la croissance et/ou la prolifération de cellules tumorales, de préférence IGF, notamment IGF1 et/ou IGF2 dépendante et/ou EGF dépendante et/ou estrogènes" dépendante et/ou HER2/neu dépendante.

De manière générale, la présente invention a pour objet l'utilisation d'un anticorps, ou l'un de ses fragments fonctionnels, de préférence humanisé, et/ou d'une composition selon l'invention, pour la préparation d'un médicament destiné à la prévention ou au traitement de cancer de préférence exprimant l'IGF-IR et/ou l'EGFR, et/ou de cancer présentant de préférence une hyperactivation de la voie de transduction du signal médié par l'interaction de l'IGF1 ou IGF2 avec IGF-IR, comme par exemple la surexpression de IRS1 et/ou de l'EGF avec l'EGFR.

La présente invention a également pour objet l'utilisation d'un anticorps, ou l'un de ses fragments fonctionnels, de préférence humanisé, et/ou d'une composition selon l'invention, pour la préparation d'un médicament destiné à la prévention ou au traitement du psoriasis, psoriasis dont l'hyperprolifération épidermique peut être liée à l'expression ou la surexpression de l'IGF-IR et/ou de l'EGFR, et/ou à l'hyperactivation de la voie de transduction du signal médié par l'interaction d'IGF-IR avec ses ligands naturels (Wraight C.J. et al. Nat. Biotechnol., 2000, 18(5):521-526. Reversal of epidermal hyperproliferation in psoriasis by insulin-like growth factor I receptor antisense oligonucleotides) et/ou de l'EGFR avec ses ligands naturels.

Parmi les cancers qui peuvent être prévenus et/ou traiter, on préfère le cancer de la prostate, les ostéosarcomes, le cancer du poumon, le cancer du sein, le cancer de l'endomètre ou le cancer du côlon ou tout autre cancer surexprimant IGF-IR.

Sous encore un autre aspect, la présente invention a pour objet une méthode de diagnostic, de préférence *in vitro,* de maladies liées à une surexpression ou une sousexpression, de préférence une surexpression du récepteur IGF-IR et/ou EGFR à partir d'un échantillon biologique dont on suspecte la présence anormale en récepteur IGF-IR et/ou EGFR, caractérisée en ce qu'on met en contact ledit échantillon biologique avec un anticorps, ou l'un de ses fragments fonctionnels, selon l'invention, ledit anticorps pouvant être, le cas échéant, marqué.

De préférence, lesdites maladies liées par la surexpression du récepteur IGF-IR et/ou EGFR dans ladite méthode de diagnostic seront des cancers.

Ledit anticorps, ou l'un de ses fragments fonctionnels, peut se présenter sous forme d'immunoconjugué ou d'anticorps marqué afin d'obtenir un signal détectable et/ou quantifiable.

Les anticorps marqués selon l'invention ou leurs fragments fonctionnels incluent par exemple des anticorps dits immunoconjugués qui peuvent être conjugués par exemple avec des enzymes telles que la péroxydase, la phosphatase alkaline, l'α-D-galactosidase, la glucose oxydase, la glucose amylase, l'anhydrase carbonique, l'acétyl-cholinestérase, le lysozyme, la malate déhydrogénase ou la glucose-6 phosphate déhydrogénase ou par une molécule comme la biotine, la digoxigénine ou la 5-bromo-désoxyuridine. Des marqueurs fluorescents peuvent être également conjugués aux anticorps ou leurs fragments fonctionnels selon l'invention et incluent notamment la fluorescéine et ses dérivés, le fluorochrome, la rhodamine et ses dérivés, la GFP (GFP pour « Green Fluorescent Protein »), le dansyl, l'umbelliférone etc... Dans de tels conjugués, les anticorps de l'invention ou leurs fragments fonctionnels peuvent être préparés par des méthodes connues de l'homme de l'art. Ils peuvent être couplés aux enzymes ou aux marqueurs fluorescents directement ou par l'intermédiaire d'un groupe espaceur ou d'un groupe de liaisons tel qu'un polyaldéhyde, comme le glutaraldéhyde, l'acide éthylènediaminetétraacétique (EDTA), l'acide diéthylènetriaminepentaacétique (DPTA), ou en présence d'agents de couplage tels que ceux cités ci-avant pour les conjugués thérapeutiques. Les conjugués comportant des marqueurs de type fluorescéine peuvent être préparés par réaction avec un isothiocyanate.

D'autres conjugués peuvent inclure également des marqueurs chimioluminescents tels que le luminol et les dioxétanes, des marqueurs bioluminescents tels que la luciférase et la luciférine, ou encore des marqueurs radioactifs tels que Iode¹²³, Iode¹²⁵, Iode¹²⁶, Iode¹³³, Brome⁷⁷, Technetium^{99m}, Indium¹¹¹, Indium^{113m}, Gallium⁶⁷, Gallium⁶⁸, Ruthénium⁹⁵, Ruthénium⁹⁷, Ruthénium¹⁰³, Ruthénium¹⁰⁵, Mercure¹⁰⁷, Mercure²⁰³, Rhénium^{99m}, Rhénium¹⁰¹, Rhénium¹⁰⁵, Scandium⁴⁷, Tellurium^{121m}, Tellurium^{122m}, Tellurium^{125m}, Thulium¹⁶⁵, Thulium¹⁶⁷, Thulium¹⁶⁸, Fluor¹⁸, Yttrium¹⁹⁹, l'Iode¹³¹. Les méthodes connues de l'homme de l'art existant pour coupler les radioisotopes thérapeutiques aux anticorps soit directement soit via un agent chélatant tel l'EDTA, le DTPA citées ci-avant peuvent être utilisées pour les radioéléments utilisables en diagnostic. On peut également citer ici le marquage avec du Na[I¹²⁵] par la méthode à la chloramine T. [Hunter W.M. et Greenwood F.C. (1962) Nature 194:495] ou encore avec le Technetium^{99m} par la technique de Crockford *et coll.* (brevet US 4 424 200) ou attaché via du DTPA comme décrit par Hnatowich (brevet US 4 479 930).

Ainsi, les anticorps, ou leurs fragments fonctionnels, selon l'invention peuvent être employés dans un procédé pour la détection et/ou la quantification d'une surexpression ou d'une sousexpression, de préférence une surexpression du récepteur IGF-IR et/ou EGFR dans un échantillon biologique, caractérisé en ce qu'il comprend les étapes suivantes :
a) la mise en contact de l'échantillon biologique avec un anticorps, ou l'un de ses fragments fonctionnels, selon l'invention ; et
b) la mise en évidence du complexe IGF-IR et/ou EGFR/anticorps éventuellement formé.

Dans un mode de réalisation particulier, les anticorps, ou leurs fragments fonctionnels, selon l'invention, pourront être employés dans un procédé pour la détection et/ou la quantification du récepteur IGF-IR et/ou EGFR dans un échantillon biologique, pour le suivi de l'efficacité d'un traitement prophylactique et/ou thérapeutique d'un cancer IGF et/ou EGF dépendant ou encore d'un psoriasis.

Plus généralement, les anticorps, ou leurs fragments fonctionnels, selon l'invention peuvent être avantageusement mis en oeuvre dans toute situation où l'expression du récepteur IGF-IR et/ou EGFR doit être observée de manière qualitative et/ou quantitative.

De préférence, l'échantillon biologique est constitué par un fluide biologique, tel que le sérum, le sang total, des cellules, un échantillon de tissu ou des biopsies d'origine humaine.

Toute procédure ou test classique peut être mis en oeuvre pour réaliser une telle détection et/ou dosage. Ledit test peut être un test par compétition ou par sandwich, ou tout test connu de l'homme de l'art dépendant de la formation d'un complexe immun de type anticorps-antigène. Suivant les applications selon l'invention, l'anticorps ou l'un de ses fragments fonctionnels peut être immobilisé ou marqué. Cette immobilisation peut être réalisée sur de nombreux supports connus de l'homme de l'art. Ces supports peuvent notamment inclure le verre, le polystyrène, le polypropylène, le polyéthylène, le dextran, le nylon, ou des celluloses naturelles ou modifiées. Ces supports peuvent être soit solubles ou insolubles.

A titre d'exemple, une méthode préférée met en jeu des processus immunoenzymatiques selon la technique ELISA, par immunofluorescence, ou radio-immunologique (RIA) ou équivalent.

Ainsi, la présente invention comprend également les kits ou nécessaires pour la mise en oeuvre d'une méthode de diagnostic de maladies induites par une surexpression ou une sousexpression du récepteur IGF-IR et/ou EGFR ou pour la mise en oeuvre d'un procédé pour la détection et/ou la quantification d'une surexpression ou d'une sousexpression du récepteur IGF-IR et/ou EGFR dans un échantillon biologique, de préférence une surexpression dudit récepteur, caractérisé en ce que ledit kit ou nécessaire comprend les éléments suivants :
a) un anticorps, ou l'un de ses fragments fonctionnels, selon l'invention ;
b) éventuellement, les réactifs pour la constitution du milieu propice à la réaction immunologique ;
c) éventuellement, les réactifs permettant la mise en évidence des complexes IGF-IR et/ou EGFR/anticorps produits par la réaction immunologique.

L'invention est en outre relative à l'utilisation d'une composition comme produit de combinaison selon l'invention, pour la préparation d'un médicament destiné à la prévention ou au traitement de cancer, notamment des cancers pour lesquels ledit agent cytotoxique ou ledit anticorps anti-HER2/neu est généralement prescrit et, notamment, pour lesquels cancers, les cellules tumorales expriment ou surexpriment le récepteur IGF-IR et/ou EGFR.

L'invention a également pour objet l'utilisation d'un anticorps selon l'invention, pour la préparation d'un médicament destiné au ciblage spécifique d'un composé biologiquement actif vers des cellules exprimant ou surexprimant le récepteur IGF-IR et/ou EGFR.

On entend désigner ici par composé biologiquement actif tout composé capable de moduler, notamment d'inhiber, l'activité cellulaire, en particulier leur croissance, leur prolifération, la transcription ou la traduction de gène.

L'invention a aussi pour objet un réactif de diagnostic *in vivo* comprenant un anticorps selon l'invention, ou l'un de ses fragments fonctionnels, de préférence marqué, notamment radiomarqué, et son utilisation en imagerie médicale, en particulier pour la détection de cancer lié à l'expression ou à la surexpression par une cellule du récepteur IGF-IR et/ou EGFR.

L'invention est également relative à une composition comme produit de combinaison ou à un conjugué anti-IGF-IR et/ou EGFR/toxine ou radioélément, selon l'invention, à titre de médicament.

De préférence, ladite composition comme produit de combinaison ou ledit conjugué selon l'invention sera additionné d'un excipient et/ou d'un véhicule pharmaceutiquement acceptable.

Dans la présente description, on entend désigner par véhicule pharmaceutiquement acceptable, un composé ou une combinaison de composés entrant dans une composition pharmaceutique ne provoquant pas de réactions secondaires et qui permet par exemple la facilitation de l'administration du ou des composés actifs, l'augmentation de sa durée de vie et/ou de son efficacité dans l'organisme, l'augmentation de sa solubilité en solution ou encore l'amélioration de sa conservation. Ces véhicules pharmaceutiquement acceptables sont bien connus et seront adaptés par l'homme de l'art en fonction de la nature et du mode d'administration du ou des composés actifs choisis.

De préférence, ces composés seront administrés par voie systémique, en particulier par voie intraveineuse, par voie intramusculaire, intradermique, intrapéritonéale ou sous-cutanée, ou par voie orale. De manière plus préférée, la composition comprenant les anticorps selon l'invention, sera administrée à plusieurs reprises, de manière étalée dans le temps.

Leurs modes d'administration, posologies et formes galéniques optimaux peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés.

D'autres caractéristiques et avantages de l'invention apparaissent dans la suite de la description avec les exemples et les figures dont les légendes sont représentées ci-après.

### LEGENDES DES FIGURES

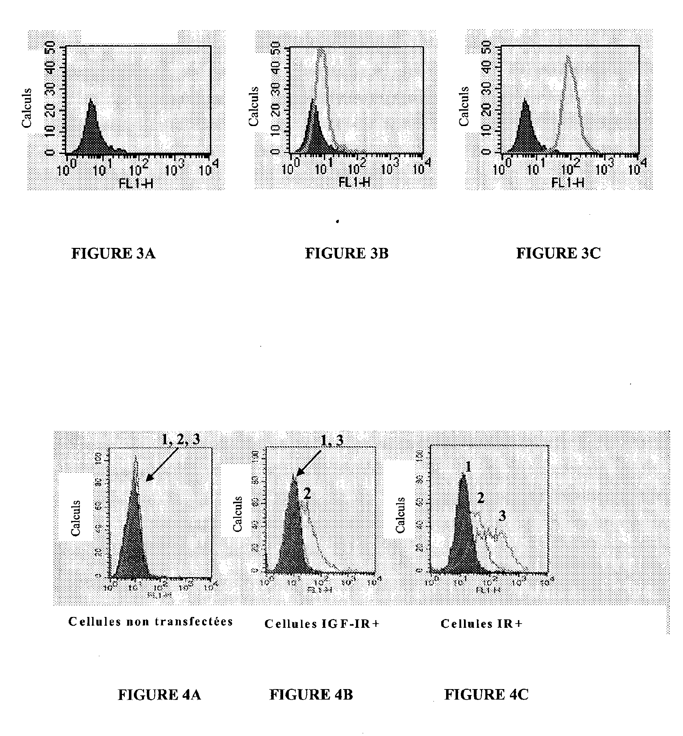

Figure 1 : Représentation schématique de l'IGF-IR.
Figure 2 : Schéma de la transduction des signaux médiée par l'IGF-IR lors de la fixation des IGFs.
Figures 3A, 3B et 3C : Reconnaissance de l'IGF-IR natif exprimé à la surface des cellules MCF-7 par l'anticorps monoclonal 7C10.
   Pour cette expérience, les cellules MCF-7 sont incubées avec l'anticorps 7C10 ou avec un anticorps contrôle négatif, puis révélées à l'aide d'un anticorps secondaire anti-espèce fluorescent. Le marquage est lu au FACS. Le premier histogramme (figure 3A) correspond aux cellules MCF-7 seules. Dans le deuxième histogramme (figure 3B) la courbe non grisée correspond au marquage non spécifique par un anticorps murin isotype contrôle. Dans le troisième histogramme (figure 3C), la courbe non grisée montre la reconnaissance de l'IGF-IR par l'ACM 7C10.
Figures 4A, 4B et 4C : Marquage de cellules d'insectes Sf9 exprimant respectivement l'IGF-IR ou l'IR.
   La figure 4A montre le marquage de cellules non transfectées seules (1) ou marquées avec des anticorps monoclonaux commerciaux témoins reconnaissant respectivement l'IGF-IR (2) ou l'IR (3). En figure 4B, des cellules Sf9 exprimant uniquement l'IGF-IR sont marquées avec l'αIR3 (2) ou l'anti-IR (3), le pic (1) représentant les cellules seules. En figure 4C, des cellules Sf9 exprimant uniquement l'IR sont marquées avec un anti-IR (3) ou l'αIR3 (2), le pic (1) représentant les cellules seules.

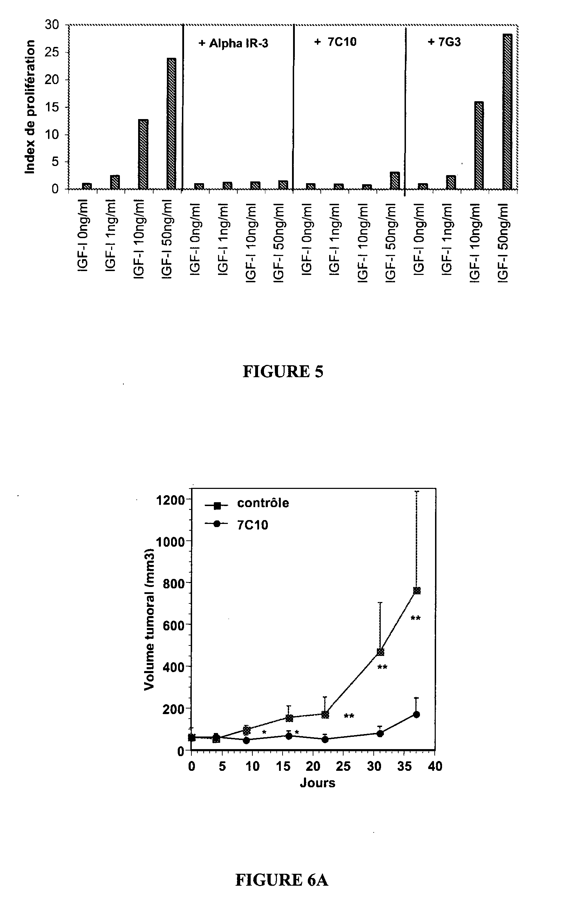

Figure 5 : Effet inhibiteur de l'anticorps 7C10 sur la prolifération des cellules MCF-7 induite par l'IGF-I.
   Les cellules MCF-7 sont incubées en présence de concentrations croissantes d'IGFI en présence ou en absence des ACM à tester. La prolifération cellulaire est évaluée par suivi de l'incorporation de ³H Thymidine. L'anticorps commercial αIR3 est utilisé comme contrôle positif de l'expérience. Le 7G3 est une IgG1 murine anti-IGF-IR sans activité sur la prolifération et utilisée comme isotype contrôle.
Figures 6A, 6B et 6C :
   - figure 6A : effet i*n vivo* de l'anticorps monoclonal 7C10 sur la croissance de tumeurs MCF-7 établies chez la souris nude ;

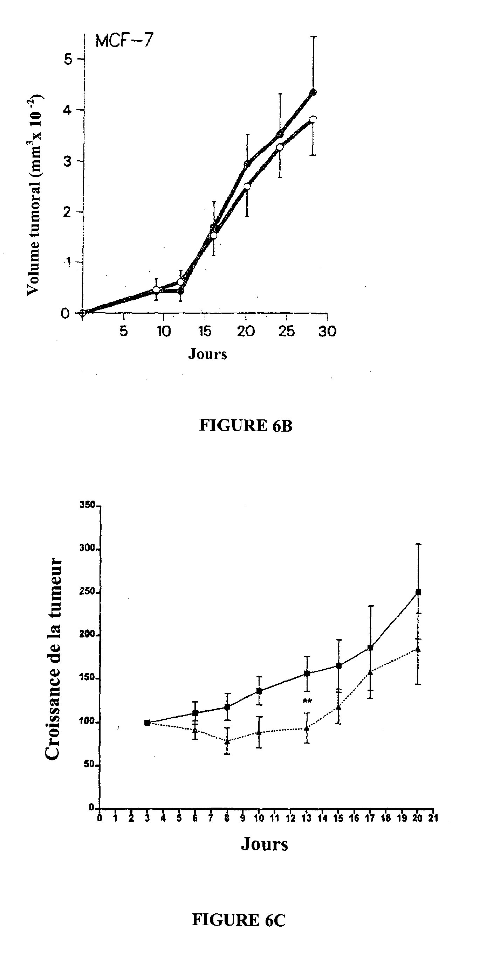

   - figures 6B et 6C : figures provenant respectivement des publications d'Arteaga et al. (J. Clin. Invest., 84, 1418-1423, 1989) et de Li et al. (Cancer Immunol. Immonother., 49, 243-252), et montrant pour la figure 6B l'effet de αIR3 murin (également noté aIR3) et pour la figure 6C l'effet d'un scFv-Fc recombinant dérivé de l'anticorps 1H7 sur la croissance tumorale.

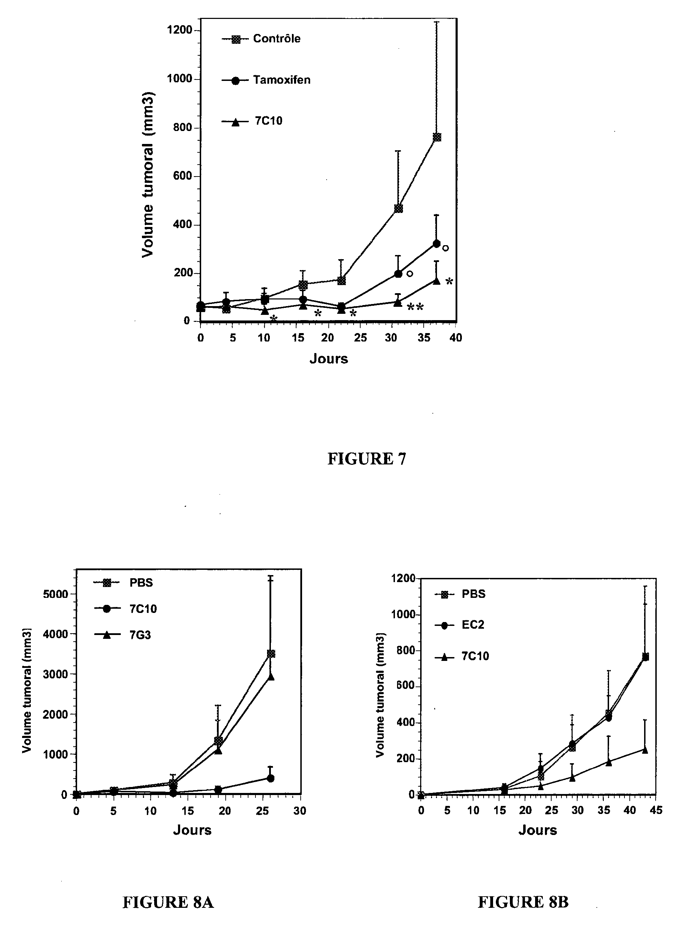

Figure 7 : Etude comparée de l'effet de l'AcM 7C10 et du tamoxifen sur la croissance *in vivo* de la tumeur MCF-7.

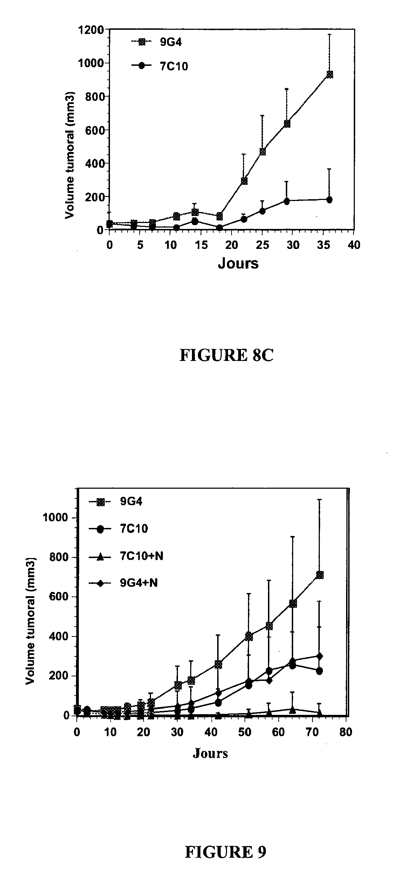

Figures 8A, 8B et 8C : Etude de l'activité antitumorale de l'anticorps murin 7C10 dans différents modèles de xénogreffe de cellules tumorales *in vivo.*
   La figure 8A montre les résultats obtenus sur un modèle d'ostéosarcome SK-ES-1, la figure 8B concerne une tumeur de la prostate androgène indépendante DU-145 et la figure 8C un modèle de tumeur du poumon non à petites cellules A549. Dans ces trois modèles, le traitement a été effectué 2 fois par semaine en i.p. à raison de 250 µg/dose/souris. Les courbes 7G3, EC2 et 9G4 correspondent respectivement à trois IgG1 murines utilisées comme isotype contrôle d'expérience dans chacun des modèles.
Figure 9 : Etude de l'effet antitumoral de l'AcM 7C10 comparé à la navelbine (vinorelbine) ainsi que de la synergie des deux composés sur la croissance *in vivo* de la lignée A549.

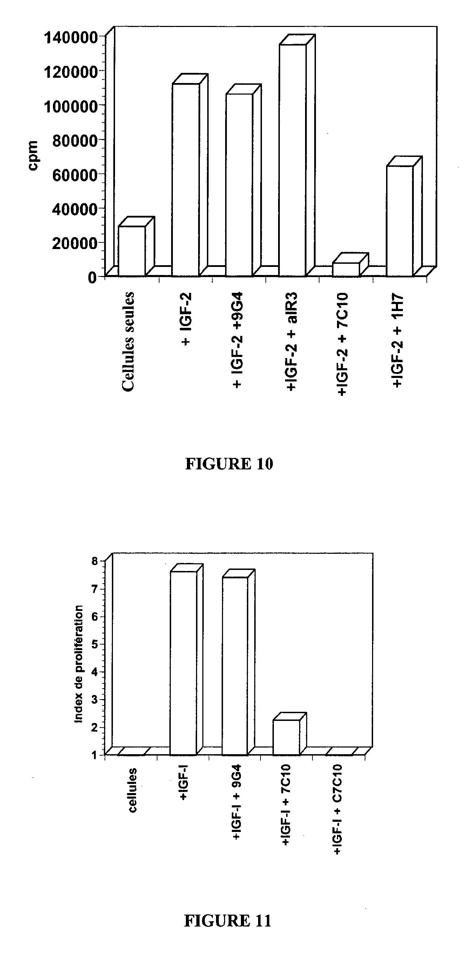

Figure 10 : Activité comparée des AcM αIR3, 7C10 et 1H7 sur la prolifération IGF-2 induite des cellules MCF-7.
Figure 11 : Comparaison des AcM 7C10 murin et C7C10 chimérique pour l'inhibition de la prolifération IGF1 des cellules MCF-7 *in vitro.* L'anticorps 9G4 est une IgG1 murine utilisée comme isotype contrôle d'expérience.

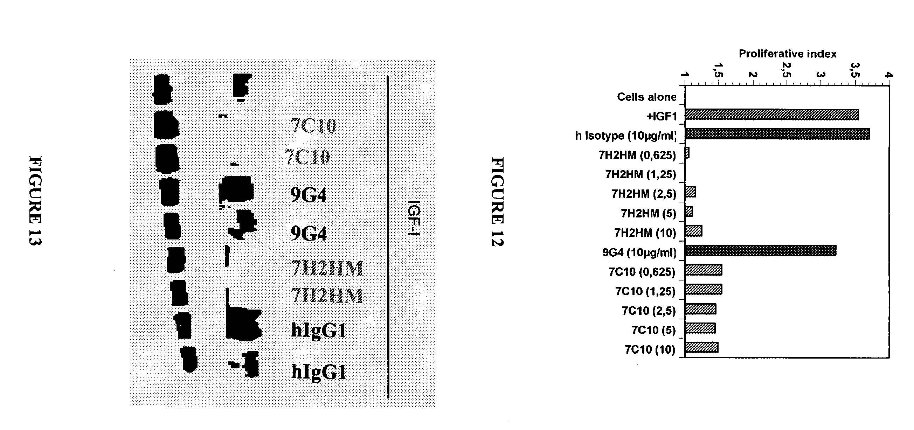

Figure 12 : Effet comparé des AcM 7C10 et h7C10 (humanisé 1, noté ici 7H2HM) sur le modèle *in vitro* de prolifération IGF1 induite des cellules MCF-7.
Figure 13 : Effet des AcM 7C10 et h7C10 (humanisé 1, noté ici 7H2HM) sur la transduction du signal induite par l'IGF1. La première ligne de spots correspond à la révélation, par un anticorps anti-phosphotyrosine, de la phosphorylation de la chaîne β immunoprécipitée à partir de cellules incubées en présence d'IGF1 seul ou d'IGF1 additionné des différents anticorps à tester. Le 9G4 et l'hIgG1 sont respectivement les isotypes contrôle des formes 7C10 et h7C10 (également noté 7H2HM). La seconde ligne de spots correspond à la révélation de la chaîne β et montre que la quantité déposée dans l'ensemble des puits est parfaitement équivalente.

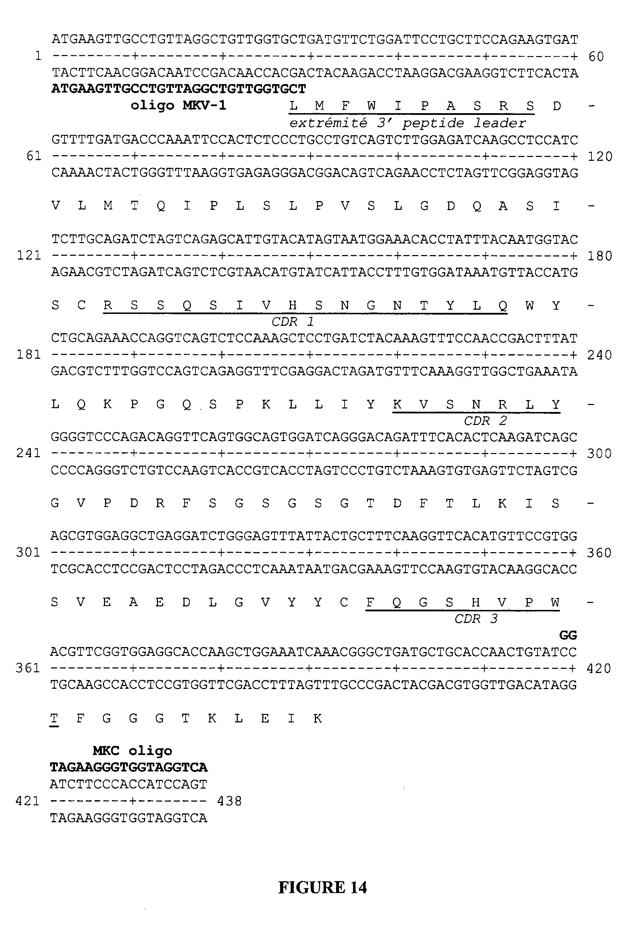

Figure 14 : Séquence de l'ADNc (SEQ ID No. 48), de son brin complémentaire (SEQ ID No. 50) et sa traduction en acides aminés (SEQ ID No. 49), du fragment de PCR amplifié à partir de l'hybridome de souris 7C10 avec les amorces MKV-1 et MKC et qui code pour l'extrémité 3' du peptide leader et 7C10 VL.

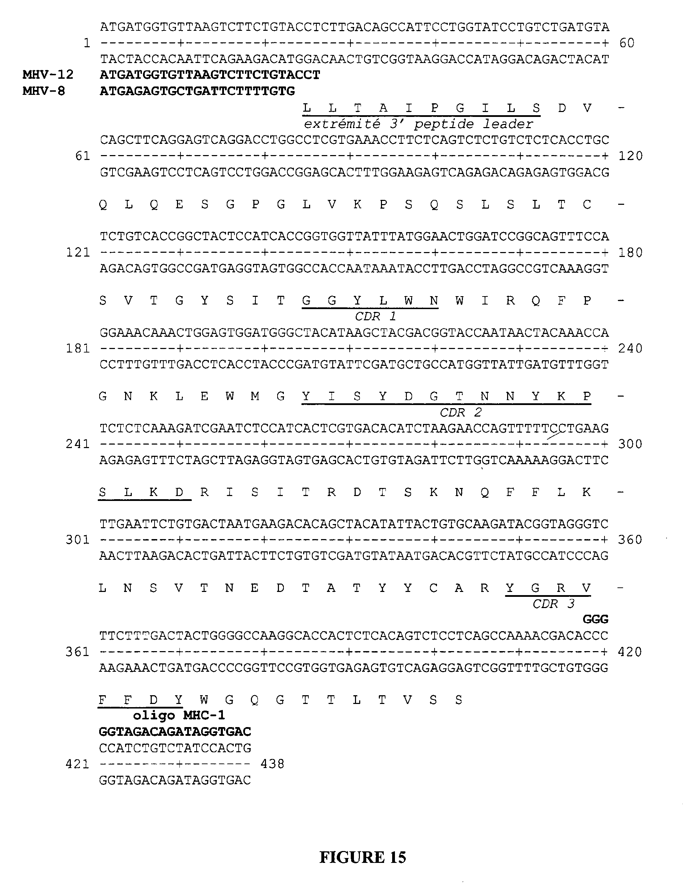

Figure 15 : Séquence de l'ADNc (SEQ ID No. 51), de son brin complémentaire (SEQ ID No. 53) et sa traduction en acides aminés (SEQ ID No. 52), du fragment de PCR amplifié à partir de l'hybridome de souris 7C10 avec les couples amorces MHV-12 et MHC-1, ou MHV-8 et MHC-1 et qui code pour l'extrémité 3' du peptide leader et 7C10 VH.

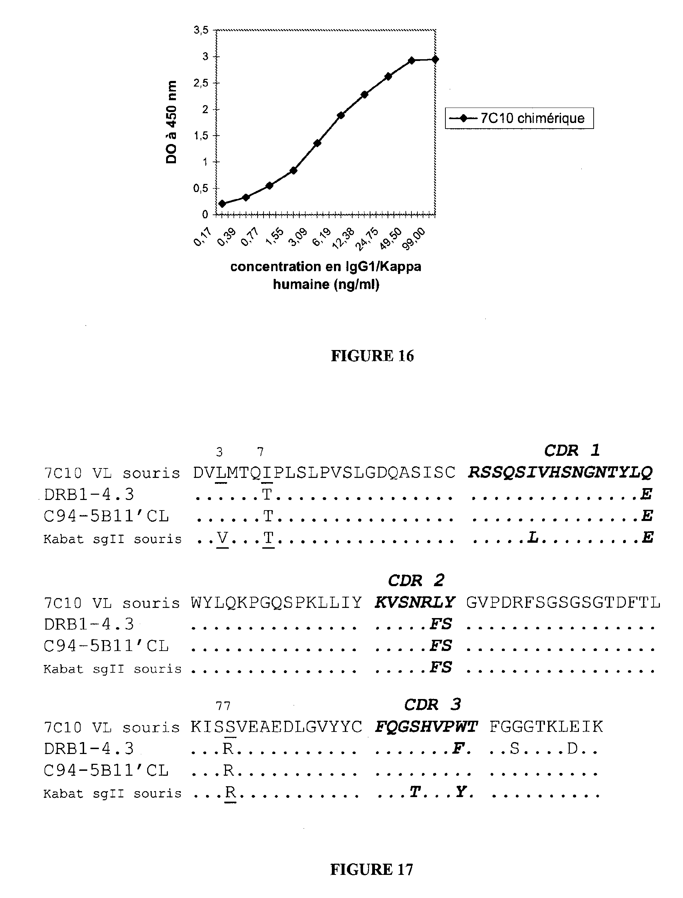

Figure 16 : Reconnaissance de l'IGF-1 récepteur par l'anticorps chimérique 7C10, également dénommé C7C10 (surnageant de culture de cellules cos7 transfectées).
Figure 17 : Comparaison de la séquence en acides aminés de 7C10 VL de souris (SEQ ID No. 54) avec celles d'autres anticorps de souris ayant la plus forte homologie de séquence.
   La numérotation des acides aminés est celle de Kabat et al. (1991). Les résidus dans les régions charpentes (hors CDRs) qui diffèrent entre 7C10 VL et Kabat sous-groupe II de souris (SEQ ID No. 57) sont soulignés. Un point indique que le résidu est identique à cette position par rapport à la séquence de 7C10 VL. DRB1-4.3 (SEQ ID No. 55) représente la séquence de la chaîne légère d'un anticorps de souris anti-human MHC CLASS II B-Chain (numéro d'accès dans la banque de données de Kabat est N011794). C94-SB11'CL (SEQ ID No. 56) représente la séquence de la chaîne légère d'un anticorps de souris (numéro d'accès dans la banque de données de Kabat est P019314).

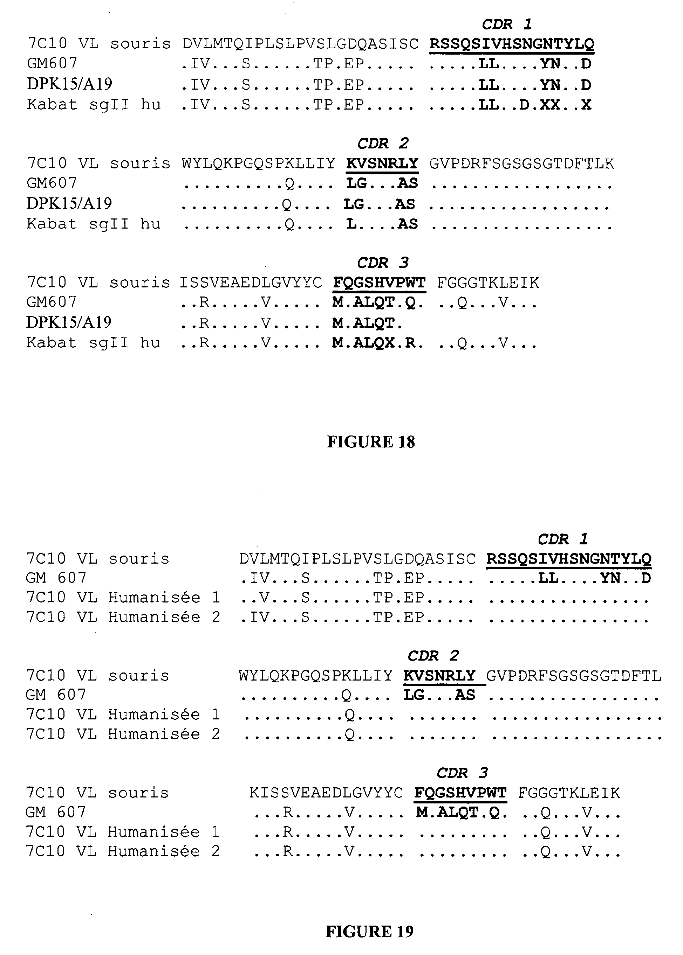

Figure 18 : Comparaison des séquences en acides aminés de 7C10 VL de souris (SEQ ID No. 54) avec celles de chaînes légères humaines appartenant au sous-groupe II humain de Kabat (SEQ ID No. 60) et ayant la plus forte homologie de séquence.
   Les séquences en acides aminés sont alignées et comparées avec celle de 7C10 VL de souris. Un point indique que le résidu est identique à cette position par rapport à la séquence de 7C10 VL. GM607 (SEQ ID No. 58) représente la séquence de la chaîne légère kappa sécrétée par la lignée lymphoblastoide humaine GM607 (Klobeck et al., Nucleic Acids Res., 12:6995-7006, 1984a et Klobeck et al., Nature, 309:73-76, 1984b, le numéro d'accès dans la banque de données Kabat est N011606). DPK15/A19 (SEQ ID No. 59) représente la séquence de la lignée germinale humaine V kappa II.
Figure 19 : Comparaison des séquences d'acides aminés des régions variables des chaînes légères (VL) de 7C10 de souris (SEQ ID No. 54), de l'anticorps humain GM 607 (SEQ ID No. 58) et des deux versions de 7C10 humanisées 1 et 2 (SEQ ID Nos. 61 et 65).
   Les séquences en acides aminés sont alignées et comparées avec celle de 7C10 VL de souris. Un point indique que le résidu est identique à cette position par rapport à la séquence de 7C10 VL. GM607 représente la séquence de la chaîne légère kappa sécrétée par la lignée lymphoblastoide humaine GM607 (Klobeck et al., 1984a et 1984b, numéro d'accès dans la banque de données Kabat : N011606).

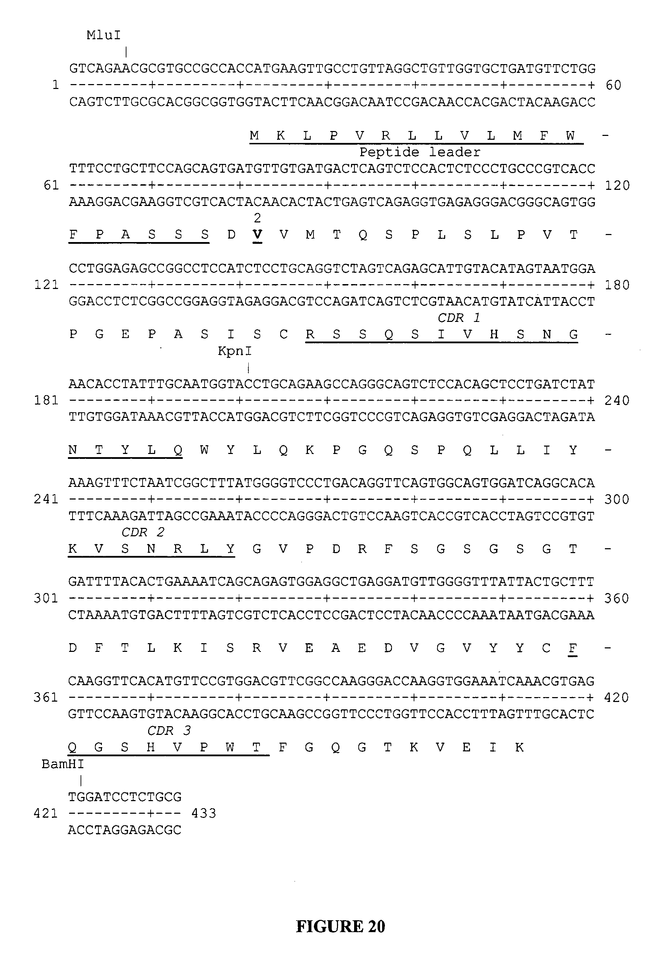

Figure 20 : Séquence de l'ADNc (SEQ ID No. 62), de son brin complémentaire (SEQ ID No. 64) et sa traduction en acides aminés (SEQ ID No. 63), du gène construit par assemblage *de novo* codant pour le peptide leader et la version humanisée 1 de 7C10 VL.

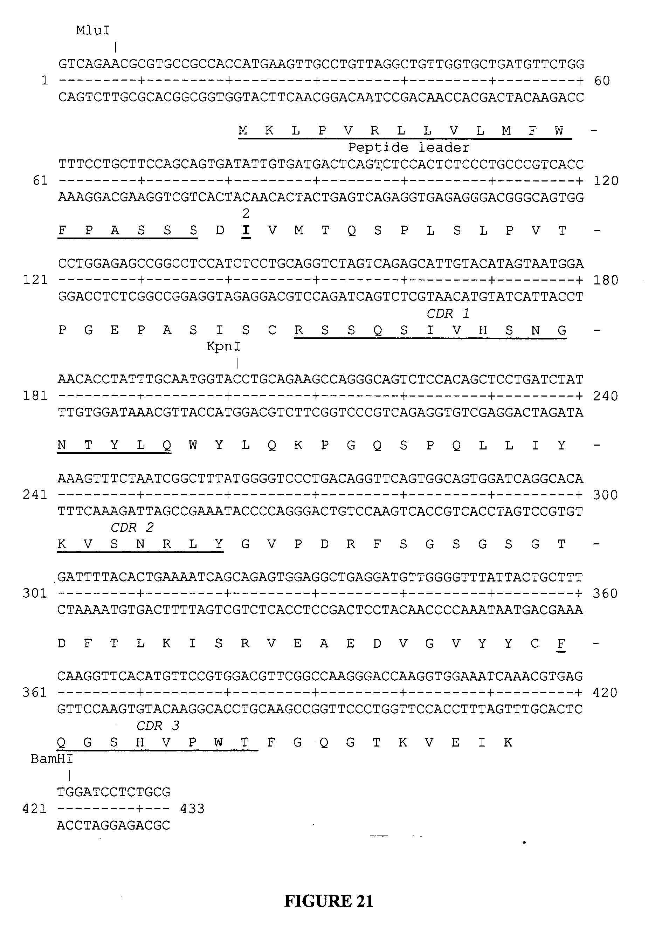

Figure 21 : Séquence de l'ADNc (SEQ ID No. 66), de son brin complémentaire (SEQ ID No. 68) et sa traduction en acides aminés (SEQ ID No. 67), du gène construit par assemblage *de novo* codant pour le peptide leader et la version humanisée 2 de 7C10 VL.

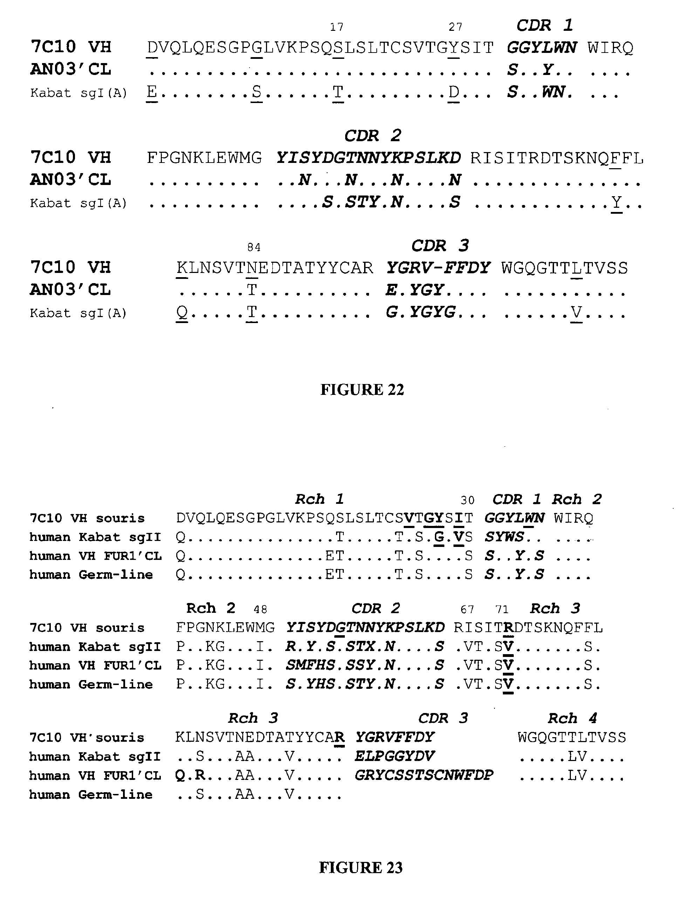

Figure 22 : Comparaison des séquences en acides aminés de 7C10 VH de souris (SEQ ID No. 69) avec celles de chaînes lourdes de souris humaines appartenant au sous-groupe I(A) souris de Kabat et ayant la plus forte homologie de séquence.
   La numérotation des acides aminés est celle de Kabat et al. (1991). Les résidus dans les régions charpentes (hors CDRs) qui diffèrent entre 7C10 VH et Kabat sous-groupe I(A) (SEQ ID No. 71) de souris sont soulignés. Un point indique que le résidu est identique à cette position par rapport à la séquence de 7C10 VH souris. AN03'CL (SEQ ID No. 70) représente la séquence de la chaîne lourde d'un anticorps de souris (numéro d'accès dans la banque de données de Kabat : P001289).
Figure 23 : Comparaison des séquences en acides aminés de 7C10 VH de souris (SEQ ID No. 69) avec celles de chaînes lourdes humaines appartenant au sous-groupe II humain de Kabat (SEQ ID No. 72) et ayant la plus forte homologie de séquence.
   Les résidus soulignés font partie des structures canoniques définies par Chothia et al. (1989). Un point indique que le résidu est identique à cette position par rapport à la séquence de 7C10 VH souris. Human VH FUR1'CL (SEQ ID No. 73) représente la séquence de la chaîne lourde d'un anticorps humain anti-lamin B IgM/K d'origine auto-immune (Mariette et al., Arthritis and Rheumatism, 36:1315-1324, 1993 ; numéro d'accès dans Kabat : N020619). Human germline (SEQ ID No. 74) représente la séquence de la lignée germinale 4.22 VH IV humaine (Sanz et al., EMBO. J. 8:3741-3748, 1989).

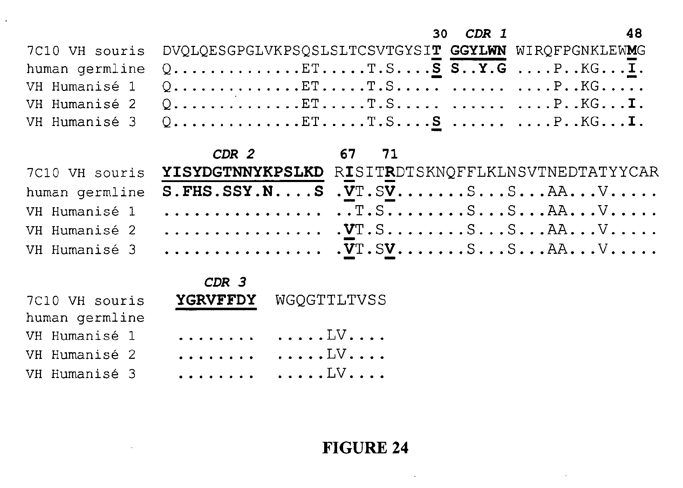

Figure 24 : Comparaison des séquences d'acides aminés des régions variables des chaînes lourdes (VH) de 7C10 de souris (SEQ ID No. 69) et des trois versions humanisées par CDR-grafting VH humanisé 1, 2 et 3 (respectivement SEQ ID Nos. 75, 79 et 83).
   La numérotation des résidus correspond à celle de Kabat. Les séquences sont alignées et comparées à celle de 7C10 VH de souris. Un point indique que le résidu est identique à cette position par rapport à la séquence de 7C10 VH souris.

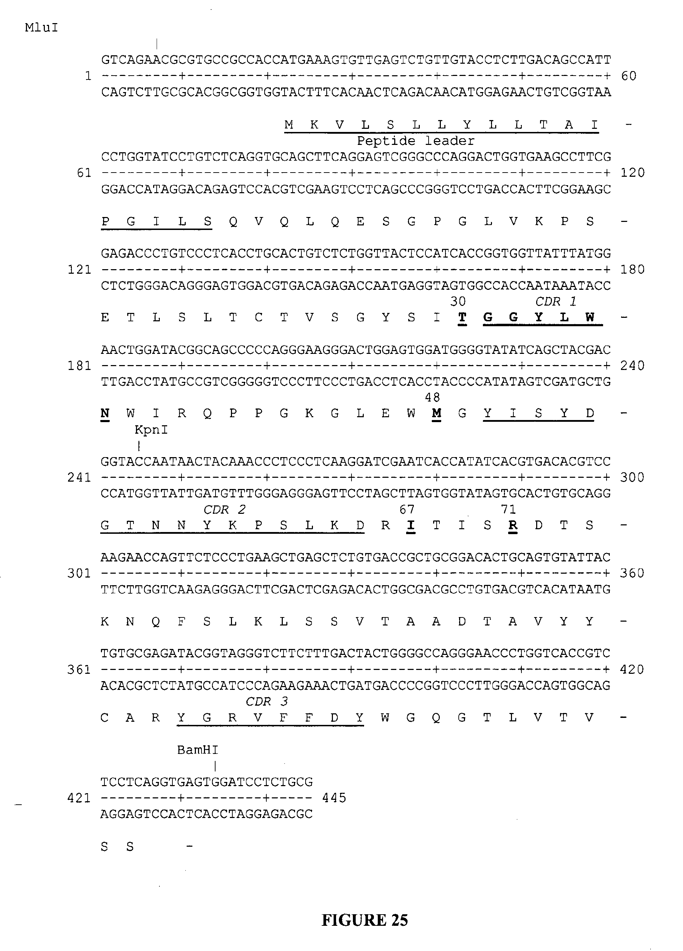

Figure 25 : Séquence de l'ADNc (SEQ ID No. 76), de son brin complémentaire (SEQ ID No. 78) et sa traduction en acides aminés (SEQ ID No. 77), du gène construit par assemblage *de novo* codant pour le peptide leader et la version humanisée 1 de 7C10 VH.

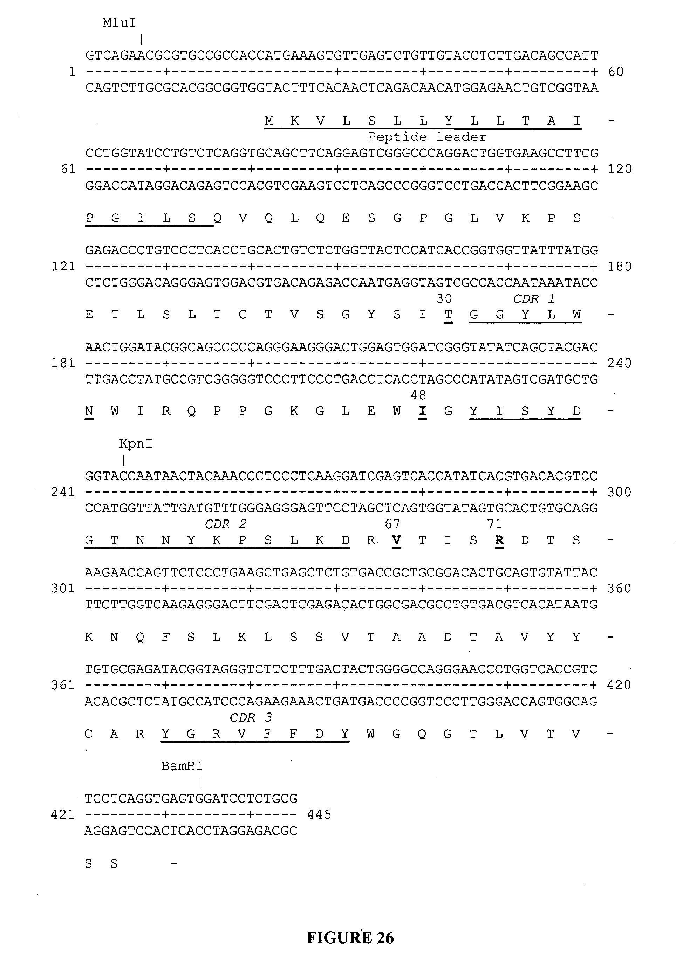

Figure 26 : Séquence de l'ADNc (SEQ ID No. 80), de son brin complémentaire (SEQ ID No. 82) et sa traduction en acides aminés (SEQ ID No. 81), du gène construit par assemblage *de novo* codant pour le peptide leader et la version humanisée 2 de 7C 10 VH.

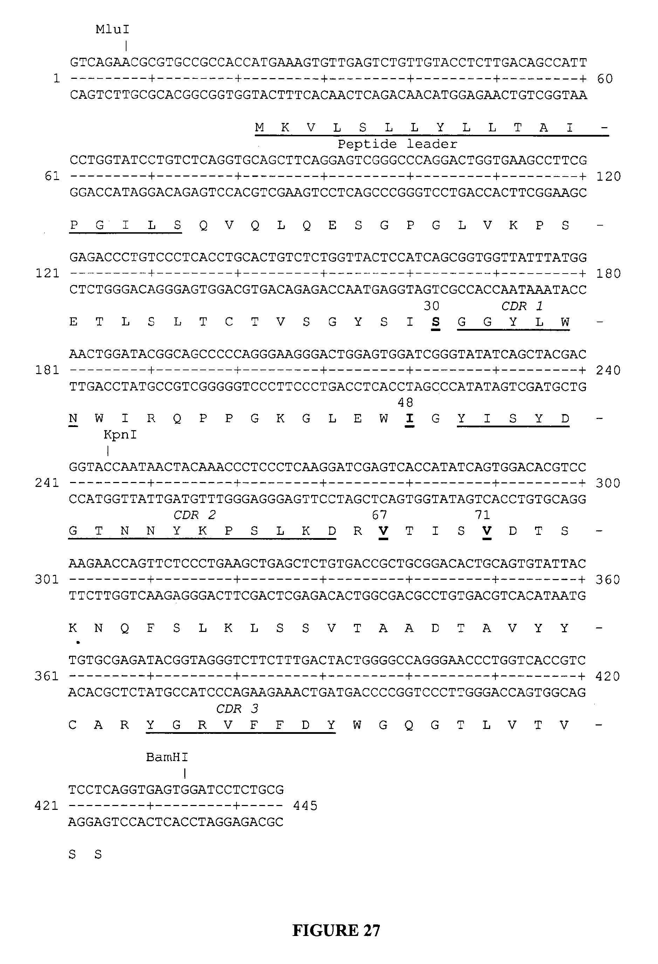

Figure 27 : Séquence l'ADNc (SEQ ID No. 84), de son brin complémentaire (SEQ ID No. 86) et sa traduction en acides aminés (SEQ ID No. 85), du gène construit par assemblage *de novo* codant pour le peptide leader et la version humanisée 3 de 7C10 VH.

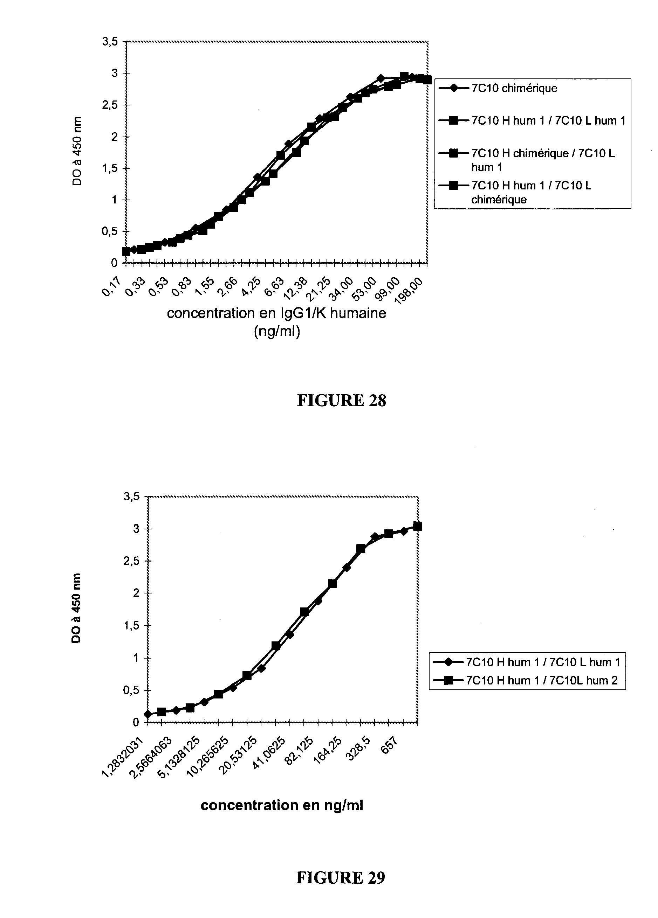

Figure 28 : Comparaison de l'activité de reconnaissance de l'IGF-1 récepteur par l'anticorps chimérique 7C10 (dénommé "C7C10") et sa version humanisée 1 (7C10 hum 1) en ELISA.
Figure 29 : Influence sur l'activité de reconnaissance de l'IGF-1 récepteur des versions humanisées 1 et 2 de la chaîne légère de l'anticorps 7C10 en ELISA.

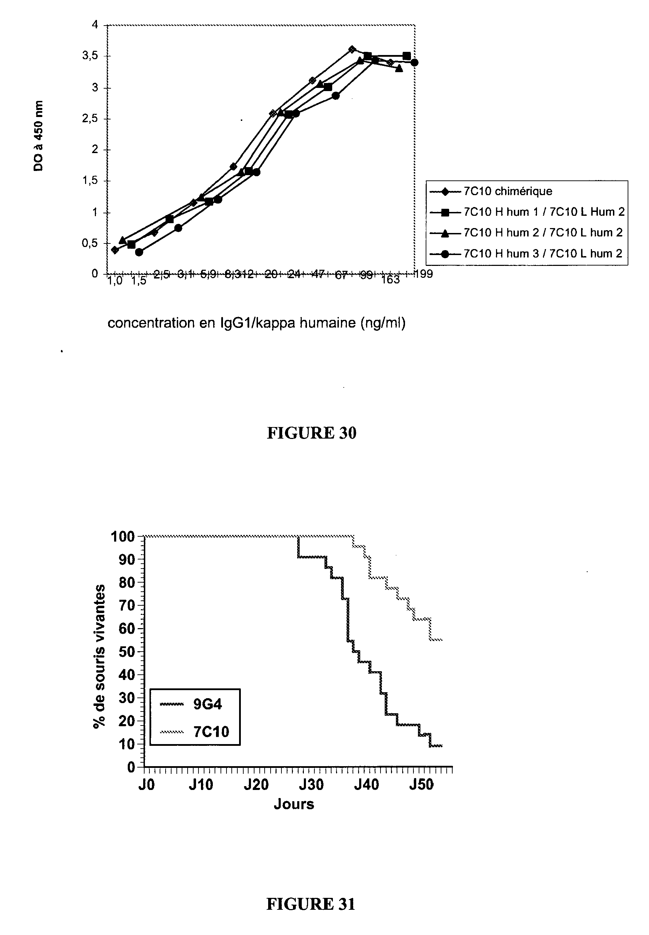

Figure 30 : Comparaison de l'activité de reconnaissance de l'IGF-1 récepteur par l'anticorps chimérique 7C10 et trois versions humanisées de la chaîne lourde (7C10 hum 1, 2 et 3) en association avec 7C10 VL humanisée 2 en ELISA.
Figure 31 : Activité antitumorale de l'anticorps 7C10 dans un modèle orthotopique A549.

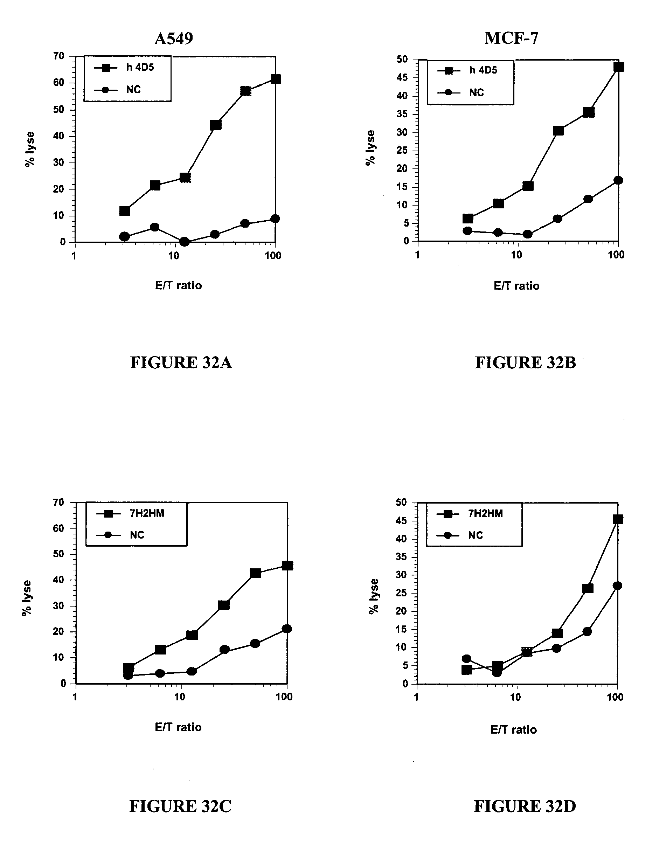

Figures 32A, 32B, 32C et 32D : Etude de l'ADCC observée au niveau de cellules A549 et MCF-7 cultivées durant 4 heures en présence de l'anticorps 7H2HM (respectivement, figures 32C et 32D). L'anticorps h4D5 est utilisé en parallèle comme témoin positif d'expérience pour les cellules A549 et MCF-7 (respectivement figures 32A et 32B).

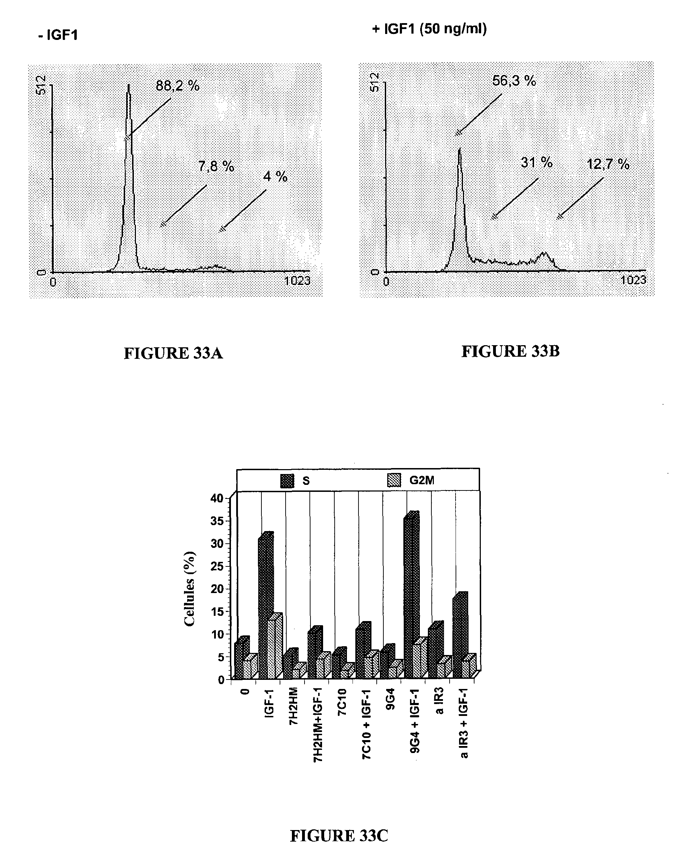

Figures 33A, 33B et 33C : Effet des anticorps 7C10 et 7H2HM sur le cycle cellulaire des cellules MCF-7.
   La figure 33A représente la proportion de cellules MCF-7 en phase G0/G1, S et G2/M en absence d'IGF1, exprimée en pourcentage de cellules MCF-7 totales observées significative.
   La figure 33B représente la proportion de cellules MCF-7 en phase G0/G1, S et G2/M en présence d'IGF1, exprimée en pourcentage de cellules MCF-7 totales observées.
   La figure 33C représente la proportion de cellules MCF-7 en phase S (■) et G2/M (□) exprimée en pourcentage de cellules MCF-7 totales observées, en présence des composés indiqués sur la figure comparée à un témoin contrôle en absence d'IGF1 (« 0 »).

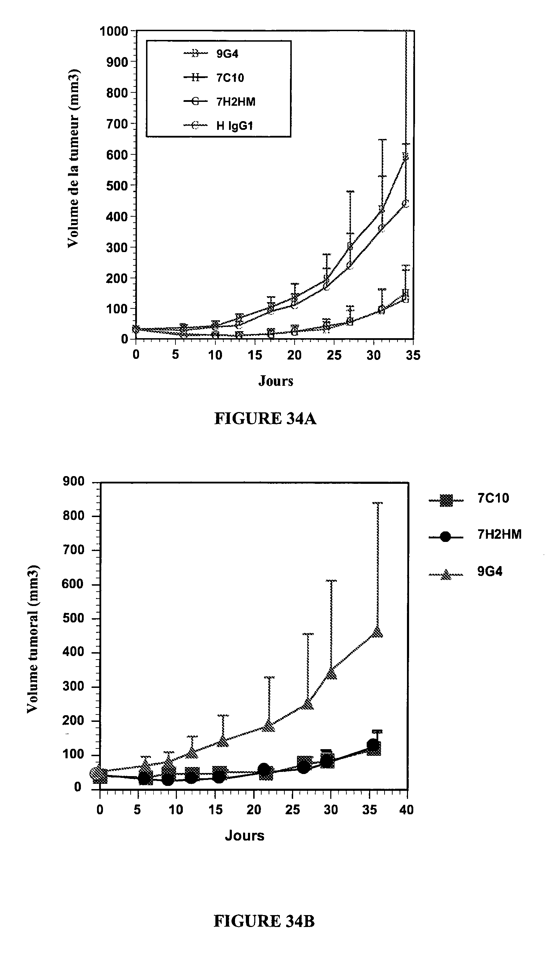

Figures 34A et 34B : Effet comparé des anticorps 7C10 et 7H2HM sur la croissance des cellules A549 in vitro (figure 34A) et sur la croissance des cellules MCF-7 in vivo (figure 34B).

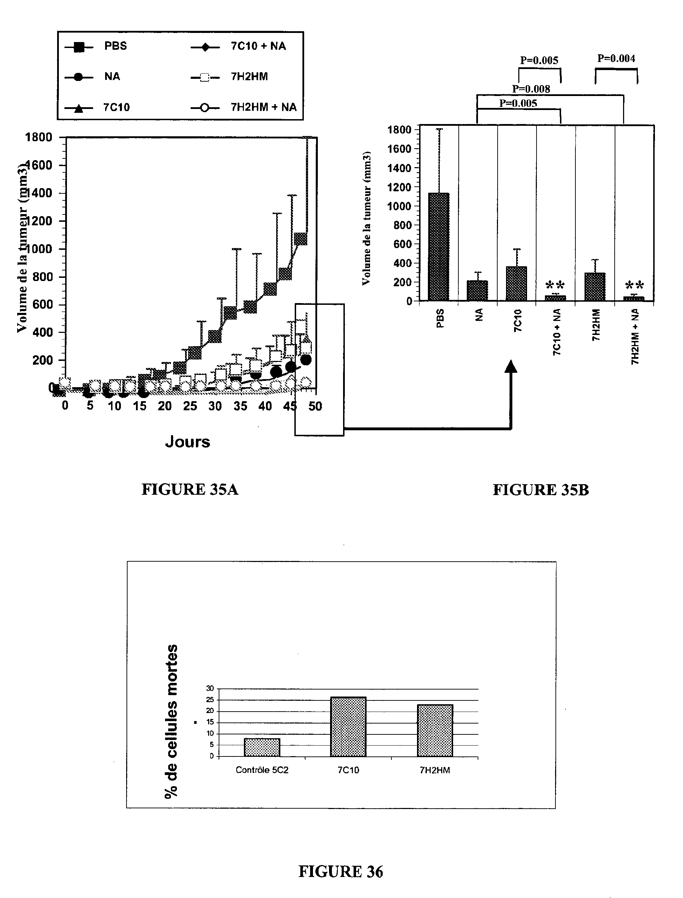

Figures 35A et 35B : Etude de la synergie de l'anticorps 7H2HM associé à la navelbine (NA) sur le modèle A549 *in vivo,* comparée aux témoins de contrôle. La figure 35A représente l'évolution du volume de la tumeur implantée en fonction du traitement effectué à partir du commencement du traitement et sur environ 50 jours (figure 35A).
La Figure 35B représente de manière particulière les résultats obtenus pour cette évolution comparée à environ 48 jours. Dans cette figure, les résultats obtenus avec l'anticorps 7C10 ont été introduits à titre de comparaison (les astérisques (*) correspondent à la comparaison groupe contrôle/groupe (7C10 + Na) ou groupe contrôle/ groupe (7H2HM + Na) dans un test t).
Figure 36 : Etude de l'effet des anticorps 7C10 et 7H2HM sur l'apoptose.
   Cette figure représente la potentialisation de l'effet de la doxorubicine par les anticorps 7C10 et 7H2HM (doxorubicine 2 µg/ml).

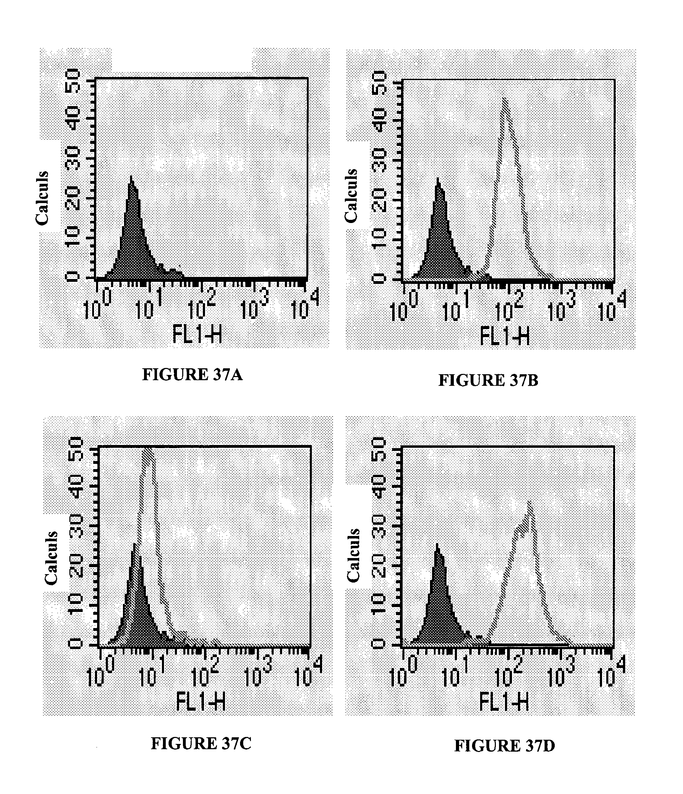

Figures 37A à 37D : Mise en évidence par un marquage au FACS de la présence de l'EGFR et de l'IGF-IR à la surface de cellules A549.

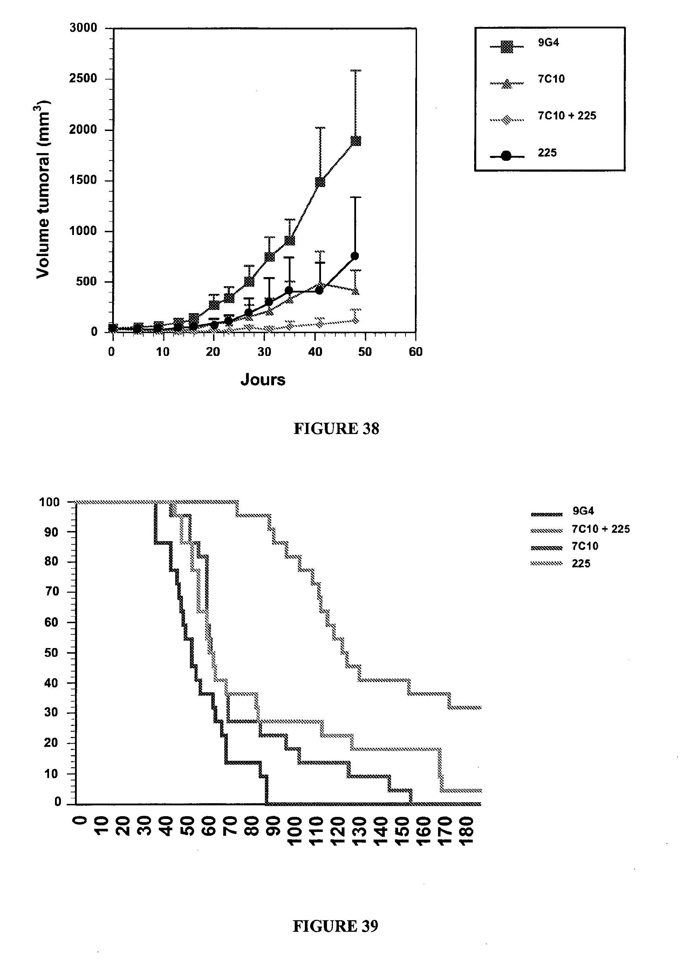

Figure 38 : Effet d'une co-administration des ACM 7C10 et 225 sur la croissance in vivo de la tumeur A549.
Figure 39 : Effet d'une co-administration des ACM 7C10 et 225 sur la survie de souris implantées en orthotopique avec des cellules A549.

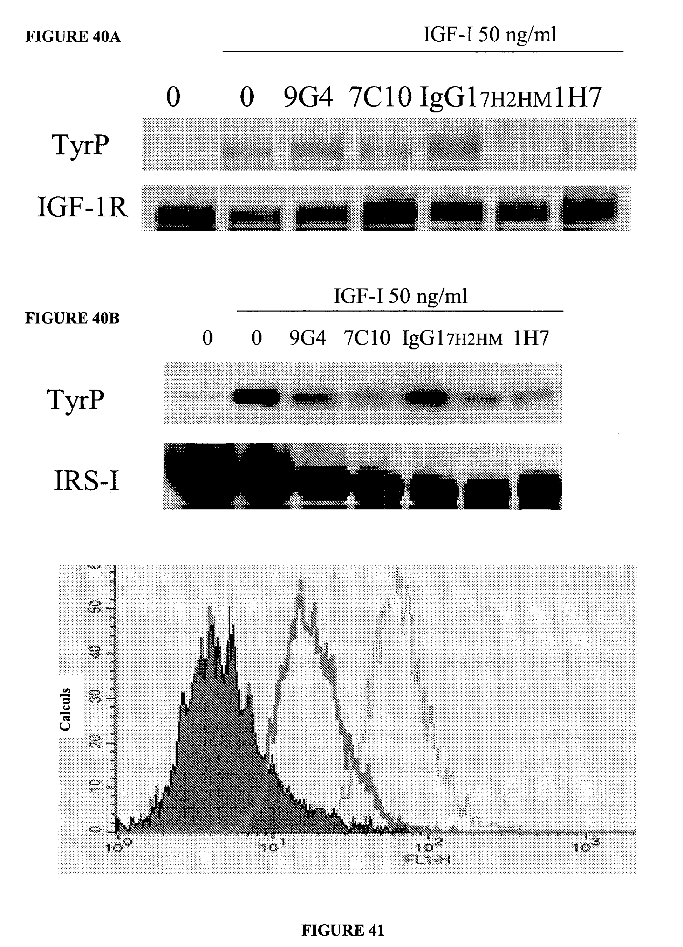

Figures 40A et 40B : Mise en évidence de l'inhibition de la tyrosine-phosphorylation de la chaîne béta de l'IGF-IR et de l'IRS-1 par les ACM 7C10 et 7H2HM.
Figure 41 : Mise en évidence de l'induction de l'internalisation de l'IGF-IR par les ACM 7C10 et 7H2HM.

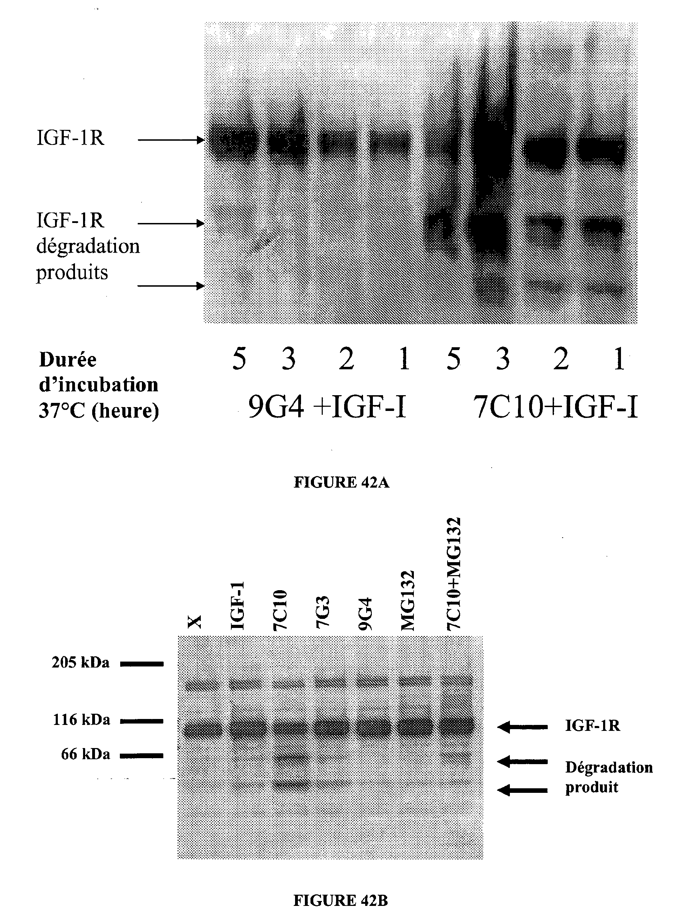

Figures 42A à 42C : Mise en évidence de la dégradation de l'IGF-IR par les ACM 7C10 et 7H2HM.

### Exemple 1. Génération et sélection de l'anticorps monoclonal (AcM) murin.

Dans le but de générer des AcM dirigés spécifiquement contre l'IGF-IR et ne reconnaissant pas l'IR, un protocole comprenant 6 étapes de criblage a été envisagé.

Il consistait à :
- immuniser des souris avec l'IGF-IR recombinant, pour générer des hybridomes,
- cribler les surnageants de culture par ELISA sur la protéine recombinante ayant servi à l'immunisation,
- tester tous les surnageants d'hybridomes positifs en ELISA sur le récepteur natif surexprimé à la surface de cellules tumorales MCF-7,
- évaluer les surnageants d'hybridomes positifs dans les deux premiers criblages en terme de reconnaissance différentielle de l'IGF-IR et de l'IR sur des cellules d'insectes infectées avec des bacullovirus exprimant respectivement l'IGF-IR ou l'IR,
- vérifier que les anticorps sélectionnés à cette étape étaient capables d'inhiber *in vitro* la prolifération IGF1 induite des cellules MCF-7,
- s'assurer de l'activité *in vivo,* chez la souris nude du candidat retenu en terme d'impact sur la croissance de la tumeur MCF-7.

L'ensemble de ces différentes étapes et des résultats obtenus sera brièvement décrit ci-après dans l'exemple 1.

Pour l'étape d'immunisation, des souris ont été injectées deux fois, par voie sous-cutanée avec 8 µg d'IGF-IR recombinant. Trois jours avant la fusion des cellules de la rate avec les cellules du myélome murin Sp2OAg14, les souris ont été stimulées par une injection intra-veineuse de 3 µg du récepteur recombinant. Quatorze jours après la fusion, les surnageants d'hybridomes ont été criblés par ELISA, sur des plaques sensibilisées par l'IGF-IR recombinant. Les hybridomes dont les surnageants ont été trouvés positifs ont été conservés et amplifiés avant d'être testés au FACScan afin de vérifier que les anticorps produits étaient également capables de reconnaître l'IGF-IR natif. Pour ce faire, des cellules MCF-7 issues d'une tumeur du sein estrogène dépendante et surexprimant l'IGF-IR ont été incubées avec chacun des surnageants de culture produits par les hybridomes sélectionnés en ELISA. Les complexes récepteur natif/AcM à la surface de la cellule ont été révélés par un anticorps secondaire anti-espèce couplé à un fluorochrome. Les figures 3A à 3C montrent un histogramme type obtenu avec le surnageant de l'hybridome 7C10 (figure 3C) comparé à un marquage cellules seules + anticorps secondaire (figure 3A) ou à un marquage utilisant un isotype contrôle (figure 3B).

A ce stade de la sélection, seuls les hybridomes sécrétant des AcM reconnaissant à la fois le récepteur recombinant et le récepteur natif ont été sélectionnés et clonés. Les AcM sécrétés par ces hybridomes ont été produits puis purifiés avant d'être testés au FACScan, selon la méthode décrite ci-dessus, sur des cellules d'insectes Sf9 exprimant l'IGF-IR ou l'IR afin d'éliminer les hybridomes reconnaissant à la fois les deux récepteurs. La figure 4A montre un recouvrement total des histogrammes 1, 2, 3 correspondant respectivement aux cellules non infectées + anticorps secondaires (1), aux cellules non infectées marquées par l'αIR3 + anticorps secondaires (2) et aux cellules non infectées marquées par un anticorps anti-IR + anticorps secondaires (3). Ce premier résultat montre bien l'absence d'IGF-IR et d'IR détectables à la surface de ces cellules d'insecte non infectées. La figure 4B montre un marquage de cellules infectées par un bacullovirus exprimant l'IGF-IR. Dans cette seconde figure l'αIR3, utilisé comme témoin positif, marque bien comme attendu les cellules (pic 2), alors que l'anti-IR (pic 3) se superpose au pic de cellules seules. Enfin, en figure 4C, il est montré que l'anti-IR marque bien comme attendu les cellules Sf9 exprimant l'IR (pic 3), mais de manière inattendue, l'αIR3 décrit dans la littérature comme spécifique de l'IGF-IR, semble reconnaître également l'IR (pic 2).

Les résultats obtenus dans ce troisième système de criblage sont résumés dans le tableau 1 et montrent la génération d'un AcM : le 7C10, satisfaisant aux critères de reconnaissance de l'IGF-IR et de non reconnaissance de l'IR. L'isotypage de l'AcM 7C10 a montré qu'il s'agissait d'une IgG1.

**TABLEAU 1 : Réactivité comparée d'AcM 7C10 sur des cellules d'insectes Sf9 exprimant l'IGF-IR ou l'IR**

| | **MFI (Moyenne de l'intensité de fluorescence)** | | |
|---|---|---|---|
| | **Cellules non infectées** | **Cellules IGF1R +** | **Cellules IR +** |
| **Cellules** | 8 | 8 | 7 |
| **Anti-IR** | 4,6 | 9 | **91** |
| **Anti-IGF-IR(αIR3)** | 9 | **35** | **32** |
| **EC2** | 8 | 13 | 11 |
| **Anti-souris FITC** | 4,3 | 9 | 13 |
| **Milieu UltraCulture** | 9 | 10 | 11 |
| **15B9** | 7,5 | **25** | **77,8** |
| **9F5D** | 8 | **41** | **40** |
| **13G5** | 7,8 | **37** | **24** |
| **7C10** | 8,6 | **49** | 13 |

Les deux derniers criblages prévus pour la sélection de l'AcM consistaient à vérifier que ce dernier était bien capable d'inhiber la prolifération cellulaire induite par l'IGF-1 *in vitro* et *in vivo* sur la lignée cellulaire MCF-7.

Pour la sélection *in vitro,* les cellules MCF-7 ont été ensemencées, déprivées en sérum de veau foetal, puis incubées en présence de concentrations croissantes d'IGF-1 (de 1 à 50 ng/ml) en présence ou en absence de l'anticorps 7C10 à tester additionné à une concentration finale de 10 µg/ml. Dans cette expérience, l'AcM commercial αIR3 a été introduit comme témoin positif et l'AcM 7G3 (isolé parallèlement au 7C10 et reconnaissant faiblement le récepteur natif (MFI au FACS de 50 comparé à 200 pour l'ACM 7C10)) comme isotype contrôle. La prolifération cellulaire est estimée par suivi au compteur P de l'incorporation de thymidine tritiée par les cellules. Les résultats sont exprimés en index de prolifération. Les données présentées dans la figure 5 montrent que l'IGF1 est capable de stimuler de façon dose dépendante la prolifération des cellules MCF-7. L'AcM αIR3, utilisé comme contrôle positif inhibe complètement la prolifération des cellules MCF-7 induite par l'IGF-1. De la même manière, l'AcM 7C10 est capable d'inhiber significativement la croissance des cellules MCF-7 induite par l'IGF-1. Enfin, l'AcM 7G3 utilisé comme contrôle isotypique s'avère bien, comme attendu, sans effet sur la croissance cellulaire tumorale *in vitro* de la cellule MCF-7.

La sélection *in vivo* a été effectuée dans un modèle de tumeur établie. Pour ce faire, des souris nudes ont reçu un implant sous-cutané d'estrogène à libération lente, indispensable à la prise de la tumeur dans un modèle murin. Vingt quatre heures après implantation des estrogènes, 5.10⁶ cellules MCF-7 sont greffées sur le flanc droit de la souris en sous-cutané. Cinq jours après cette greffe cellulaire les tumeurs sont mesurables et des lots de 6 souris sont constitués au hasard. Le traitement des souris est effectué deux fois par semaine, durant 5 à 6 semaines, à la dose de 250 µg/dose/souris. Dans le groupe contrôle, les souris sont traitées de la même façon avec un isotype contrôle murin. Les résultats présentés dans la figure 6A montrent une inhibition très significative de la croissance tumorale induite par l'anticorps 7C10. Cette activité est particulièrement inattendue si l'on se réfère aux données disponibles concernant l'αIR3, toujours utilisé comme référence dans le domaine du récepteur à l'IGF1, et connu pour n'avoir aucune activité *in vivo* sur la croissance des tumeurs estrogènes dépendantes (voir figure 6B). De même, comparé aux résultats obtenus avec l'anticorps recombinant scFv-Fc dérivé de l'AcM murin 1H7 (voir figure 6C), l'AcM 7C10 est beaucoup plus efficace dans l'inhibition *in vivo* de la croissance des cellules MCF-7.

### Exemple 2. Comparaison de l'effet du 7C10 et du tamoxifen sur la croissance in vivo de la tumeur MCF-7

Dans le but de déterminer la puissance du traitement par l'anticorps 7C10 dans le cadre du cancer du sein estrogène dépendant, le 7C10 a été comparé au tamoxifen composé couramment utilisé pour le traitement du carcinome mammaire dans le cadre des formes évoluées avec progression locale et/ou métastatiques et dans le cadre de la prévention des récidives (voir VIDAL 2000, pages 1975-1976).

Dans les cancers du sein hormono-dépendants, il existe une corrélation significative entre l'expression des récepteurs aux estrogènes (ER) et celle de l'IGF-IR (Surmacz E. et al., Breast Cancer Res. Treat., Feb., 47(3):255-267, 1998). Par ailleurs, il semble que les estrogènes (E2) agissent en synergie avec l'IGF1 (parfois noté IGF-1 ou IGFI) pour stimuler la prolifération cellulaire. Il a en effet été montré qu'un traitement par E2 augmentait d'environ 10 fois le taux de mRNA de l'IGF-IR ainsi que le niveau d'expression de la protéine (Lee A.V. et al., Mol. Endocrinol., May, 13(5):787-796, 1999). Cette augmentation se traduit par une augmentation significative de la phosphorylation de l'IGF-IR. De plus, les E2 stimulent significativement l'expression de l'IRS-1 ("IRS-1" pour "Insulin Receptor Substrat-1") qui est l'un des substrats de l'IGF-IR phosphorylé.

Le tamoxifen est largement utilisé depuis plusieurs années en hormonothérapie pour le traitement des patientes atteintes de cancers de sein E2-dépendants (Forbes J.F., Semin. Oncol., Feb., 24 (1.Suppl.1):S1-5-S1-19, 1997). Cette molécule entre en compétition avec l'estradiol et inhibe la fixation de celui-ci à son récepteur (Jordan V.C., Breast Cancer Res. Treat., 31(1):41-52, 1994). Il a par ailleurs été démontré que le tamoxifen est capable d'inhiber la prolifération IGF-IR dépendante en inhibant l'expression du récepteur et sa phosphorylation (Guvakova M.A. et al., Cancer Res., July 1, 57(13):2606-2610,1997). L'ensemble de ces données semble indiquer que l'IGF-IR est un important médiateur de la prolifération induite par l'interaction E2/ER.

L'utilisation à long terme du tamoxifen étant associée avec une augmentation significative du risque de cancer de l'endomètre (Fisher et al., J. of National Cancer Institute, 86,7:527-537, 1994 ; VIDAL 2000, 1975-1976) et de récidive collatérale de cancer du sein E2 indépendants (Li C.I. et al., J Natl. Cancer Inst., July 4, 93(13):1008-1013, 2001). Dans ce contexte, une comparaison de l'effet antitumoral *in vivo* de l'anticorps 7C10 et du tamoxifen a été effectuée sur le modèle MCF-7 afin de déterminer la part de l'activité liée à l'IGF-IR dans la prolifération ER médiée. Pour ce faire, 7.10⁶ cellules MCF-7 ont été implantées en sc (sous-cutanée) chez des souris nude, 24 heures après implantation chez ces mêmes souris d'un granule d'estradiol à libération prolongée (0,72 mg/comprimé libéré sur 60 jours), indispensable à l'établissement de toute tumeur humaine E2 dépendante chez cette espèce animale. Cinq jours après cette implantation, les tumeurs sont mesurées et des groupes de 6 souris constitués. Ces groupes sont respectivement traités avec 1) l'anticorps 7C10 injecté en ip (intra péritonéal) à raison de 250 µg/souris, 2 fois par semaine, 2) 10 µg de tamoxifen repris dans en PBS contenant 3 % hydroxypropyl-cellulose (HPC) ip ou 3) le solvant dans lequel est repris le tamoxifen (hydroxypropyl cellulose). Le tamoxifen est administré quotidiennement pendant 4 semaines excepté le week-end. Les souris traitées avec l'ACM 7C10 reçoivent également quotidiennement une injection de PBS 3 % HPC. Une étude a préalablement été effectuée pour vérifier que le solvant seul est sans influence sur la croissance tumorale.

Les résultats présentés dans la figure 7 montrent que l'ACM 7C10 est capable d'inhiber significativement la croissance de la tumeur MCF-7 *in vivo* (les astérisques (*) correspondent à la comparaison groupe contrôle/groupe 7C10 dans un test t). De façon surprenante, l'anticorps 7C10 semble être significativement plus efficace que le tamoxifen pour l'inhibition de la croissance tumorale (les ronds (°) correspondent à la comparaison groupe Tamoxifen/groupe 7C10 dans un test t) suggérant que ce type de traitement par ACM puisse se substituer au traitement par le tamoxifen.

### Exemple 3. Mise en évidence de l'activité antitumorale de l'AcM 7C10 in vivo sur des tumeurs humaines de différentes origines

### a) Activité in vivo de l'anticorps 7C10 dans trois modèles tumoraux

Afin de généraliser l'activité de l'anticorps 7C10 à d'autres tumeurs exprimant le récepteur à l'IGF1, le 7C10 a été testé *in vivo* dans un modèle de tumeur de la prostate androgène indépendant DU145 (également noté DU-145), dans un modèle d'ostéosarcome SKES-1 et dans un modèle de tumeur du poumon non à petites cellules A549. Le protocole est comparable à celui décrit ci-dessus pour MCF-7 et les résultats présentés figures 8A à 8C montrent une activité significative de cet ACM dans les 3 modèles tumoraux. L'activité observée dans le modèle de tumeur de la prostate est à noter tout particulièrement dans la mesure où la simple chaîne scFv de l'ACM 1 H7 est sans activité dans un modèle de tumeur de la prostate androgène indépendante (Li et al., 2000).

### b) Activité in vivo de l'anticorps 7C10 dans un modèle orthotopique A549.

Les modèles de xénogreffe classique comme décrit ci-dessus ne permettent pas l'étude des drogues sur la dissémination métastatique. En effet, les tumeurs implantées en s.c. (sous-cutané) restent localisées au site d'injection et ne sont donc pas réellement le reflet de la situation chez l'homme. Afin d'évaluer notre anticorps dans un modèle plus proche de la réalité, les cellules A549 ont été implantées en localisation intra pleurale. Ce modèle, bien décrit (Clin. Cancer Res. 2000 Jan; 6(1): 297-304) permet d'observer une dissémination métastatique proche de celle observée chez l'homme avec des métastases médiastinales, pulmonaires cardiaques et vertébrales. Dans l'étude qui a été effectuée, 10⁶ cellules A549 ont été injectées en intra pleurale chez des souris nudes femelles. 7 jours après l'implantation, les souris ont été réparties en 2 lots de 22. L'un de ces lots a reçu une dose d'appel de 500 µg/souris et a ensuite été traité deux fois par semaine à raison de 250 µg de 7C10/dose. Le second lot a été traité selon le même schéma avec l'isotype contrôle 9G4. La figure 31 montre un délai significatif de la survie chez les souris traitées avec l'ACM 7C10 indiquant que cet anticorps est capable d'avoir une action sur la dissémination métastatique.

### Exemple 4. Comparaison de l'AcM 7C10 avec la navelbine in vivo ; effet d'une co-administration des deux traitements

La navelbine est un composé de chimiothérapie indiqué dans le cancer du poumon non à petites cellules et dans le cancer du sein métastatique. L'étude comparative du 7C10 et de la navelbine et la synergie éventuelle entre les deux produits a été étudiée sur le modèle tumoral A549. Pour cette étude 5.10⁶ cellules A549 ont été greffées en sous-cutané sur le flanc droit de la souris. Cinq jours après la greffe cellulaire, les tumeurs sont mesurables et les traitements avec l'AcM et/ou la navelbine sont commencés. La dose d'AcM est toujours de 250 µg/dose/souris, deux fois par semaine, en intra-péritonéale. Concernant la navelbine, elle sera administrée à la dose maximale tolérée par la souris soit 10 mg/kg, en intra-péritonéale. Pour ce traitement trois injections seront effectuées à 7 jours d'intervalle. Lors des co-administrations, les deux produits sont mélangés avant injection.

Les résultats présentés dans la figure 9 montrent de façon surprenante que, dans ce modèle, l'anticorps 7C10 est aussi actif que le traitement classique par la navelbine. Une synergie très significative des deux produits est également observée avec cinq souris sur sept ne présentant pas de tumeurs mesurables à J72.

### Exemple 5. Etude de l'inhibition in vitro de la croissance IGF2 induite des tumeurs MCF-7

Comme signalé précédemment, l'IGF-IR est surexprimé par de nombreuses tumeurs mais il a été décrit par ailleurs que dans une bonne partie des cancers du sein et du côlon notamment, le signal de prolifération est donné à ce récepteur via l'IGF2 (parfois noté IGF-II ou IGFII). Il est donc primordial de s'assurer que l'AcM 7C10 est également capable d'inhiber la croissance IGF2 induite sur la tumeur MCF-7 *in vitro.* Pour ce faire, des cellules ont été ensemencées en plaque 96 puits, déprivées de sérum de veau foetal et stimulées par l'addition de 200 ng d'IGF2 par ml final de milieu, en présence et en absence des AcM à tester introduits à une concentration de 10 µg/ml. Les résultats présentés dans la figure 10 montrent que l'IGF2, comme l'IGF1 stimule significativement la croissance des cellules MCF-7. L'addition d'un isotype contrôle, le 9G4 reste sans effet sur cette stimulation. Comme déjà décrit par De Léon et al. (Growth Factors, 6:327-334, 1992), aucun effet n'est observé lors de l'addition de l'AcM αIR3. En revanche, le 7C10 inhibe totalement la croissance induite par l'IGF2. Son activité est significativement meilleure que celle du 1H7.

### Exemple 6. Activité biologique des anticorps 7C10 chimériques (C7C10) et humanisés (h7C10)

### a) Comparaison 7C10/C7C10 et 7C10/h7C10 sur le modèle MCF-7 in vitro

La forme chimérique de l'AcM 7C10 et la forme humanisée 1 (notée ici 7H2HM) purifiée ont été testées *in vitro* dans le modèle MCF-7 comme décrit ci-dessus. Les résultats présentés respectivement dans les figures 11 et 12 montrent que ces deux formes ont parfaitement conservé leurs propriétés d'inhiber la croissance IGF1 induite de la tumeur MCF-7.

### b) Effet comparé des AcM 7C10 et h7C10 sur la transduction du signal induit par la fixation de l'IGF1 à son récepteur.

L'activité d'inhibition de la croissance IGF1 induite *in vitro* sur la lignée MCF-7 devrait être la traduction d'une inhibition de la transduction du signal médié par l'IGF1 lors de la fixation de l'AcM 7C10 au récepteur. Afin de vérifier cette hypothèse, des cellules MCF-7 ont été incubées avec ou sans IGF1, en présence ou en absence des anticorps à tester. Après un temps court d'incubation, les cellules ont été lysées, la chaîne P immunoprécipitée et la phosphorylation de cette sous unité estimée à l'aide d'un anticorps antiphosphotyrosine kinase. Les résultats présentés dans la figure 13 montrent que la fixation du 7C10 ou du h7C10 inhibent significativement la phosphorylation de la sous unité β de l'IGF-IR contrairement à un anticorps irrelevant murin (9G4) ou humain (noté IgG1 sur le schéma).

### c) Implication de l'anticorps 7H2HM dans les mécanismes d'ADCC

L'inhibition de la transduction du signal décrite ci-dessus dans le paragraphe b) est le principal mécanisme d'action impliqué dans l'activité biologique des anticorps 7C10 et 7H2HM. Il est cependant probable que lors de son administration chez l'homme, l'anticorps 7H2HM, d'isotype IgG1, soit capable d'induire une lyse cellulaire par un mécanisme de type ADCC (Antibody Dependent Cellular Cytotoxicity). Afin de vérifier ce point, des cellules NK (Natural Killer) provenant du sang périphérique de donneurs humains sont mises en présence de cellules A549 ou MCF-7 préalablement incubées 4 heures avec de 10 µg d'anticorps 7H2HM pour 5.10⁵ cellules et, marquées au ⁵¹Cr (50 µg). Dans cette expérience l'herceptin (notée sur les figures 32A et 32B h 4D5) est utilisée comme témoin positif d'expérience. Les figures 32A à 32D montrent que, comme attendu, l'herceptin induit une ADCC significative sur les deux cellules A549 et MCF-7 (voir respectivement les figures 32A et 32B). Le 7H2HM est également capable d'induite une ADCC sur les cellules A549 (voir figure 32C), mais ce phénomène est de moindre amplitude sur les cellules MCF-7 (voir figure 32D).

### d) Effet des anticorps 7C10 et 7H2HM sur le cycle cellulaire

L'inhibition de la croissance cellulaire observée in vitro sur la alignée MCF-7 devrait se traduire par un effet sur le cycle cellulaire. Afin de répondre à cette question 4.10⁵ cellules sont ensemencées en plaque 6 puits. 24 heures après ensemencement, le sérum de veau est enlevé et l'IGF1 ajouté en présence ou en absence des anticorps à tester. Après 24 heures d'incubation, les cellules sont récupérées pour l'étude du cycle cellulaire. La figure 33B met en évidence l'effet de l'IGF1 sur l'entrée en cycle et la croissance des cellules MCF-7 ceci comparé à l'entrée en cycle et la croissance des cellules MCF-7 en absence d'IGF1 (voir figure 33A). Après addition du facteur de croissance une diminution significative de la phase GO/G1 (de 88,2 % à 56,3 %) au profit des phases S (de 7,8 % à 31 %) et G2/M (de 4 % à 12,7 %) est observée. Lors de l'addition des anticorps 7C10 et 7H2HM (voir figure 33C), une inhibition significative de l'entrée en cycle est observée. Il est à noter que l'anticorps murin et son homologue humanisé ont une activité comparable sur le cycle cellulaire. L'αIR3, introduit comme témoin positif semble légèrement moins actif que le 7C10 et le 7H2HM dans ce test. L'anticorps 9G4 utilisé comme isotype contrôle est sans effet sur le cycle cellulaire.

### e) Activité comparée in vivo des anticorps 7C10 et 7H2HM sur le modèle A549

Afin de confirmer l'activité de l'anticorps humanisé 7H2HM *in vivo,* ce dernier a été comparé au 7C10 dans le modèle de tumeur du poumon non à petites cellules A549. Cette expérience a été effectuée exactement comme décrit ci-avant excepté la dose d'anticorps qui est de 125 µg/dose deux fois par semaine au lieu de 250 µg/dose deux fois par semaine et ce du fait de la non disponibilité de fortes quantités de 7H2HM. L'anticorps 9G4 a été utilisé comme contrôle isotypique pour le 7C10 et une immunoglobuline humaine irrelevante d'isotype IgG1 (ci après dénommée HIgG1) a été utilisée comme contrôle pour l'anticorps humanisé 7H2HM.

La figure 34A montre qu'il n'y a pas de différences significatives entre les courbes contrôles 9G4 et HIgG1. Comme attendu, une inhibition significative de la croissance tumorale est observée avec l'anticorps murin 7C10. Concernant l'anticorps humanisé 7H2HM, l'activité observée est exactement de la même intensité que celle observée avec sa contrepartie murine. Cette donnée, additionnée aux observations décrites ci-avant *in vitro,* indique que l'humanisation n'a pas modifié les propriétés de l'anticorps généré. D'autre part, dans les modèles de xénogreffe chez la souris, l'activité de l'anticorps humanisé semble être intégralement liée à un mécanisme d'inhibition de la transduction du signal. En effet si une part ADCC était en jeu dans l'inhibition de la croissance tumorale chez la souris Nude, une différence devrait être observée entre l'activité des anticorps murins et humanisés.

Une expérience *in vivo* a également été effectuée sur le modèle de tumeur du sein MCF-7 et montre que, comme attendu, l'anticorps 7H2HM est parfaitement comparable à l'anticorps murin 7C10 pour l'inhibition de la croissance de cette tumeur *in vivo* (figure 34B).

### f) Mise en évidence d'une synergie entre le 7H2HM et la navelbine

Le protocole décrit dans l'exemple 4 a été repris dans le but de reproduire les résultats obtenus avec le 7C10 avec son homologue humanisé : l'anticorps 7H2HM.

Les résultats présentés aux figures 35A et 35B montrent que, comme dans le cas du 7C10, une synergie significative est mise en évidence entre l'anticorps humanisé 7H2HM et la navelbine.

### g) Effet des anticorps 7C10 et 7H2HM sur l'apoptose des cellules MCF-7 in vitro

Comme indiqué ci-avant, l'IGF-IR est capable de conférer une protection contre l'apoptose lorsqu'il est surexprimé à la surface des cellules. Par ailleurs il a été montré dans ces exemples que les anticorps 7C10 et 7H2HM étaient capable de potentialiser un composé actif en chimiothérapie. Afin de tester le pouvoir des anticorps 7C10 et 7H2HM à induire l'apoptose, et d'expliquer en partie leur potentiel de synergie avec la chimiothérapie, des expériences ont été menées sur les cellules MCF-7 en présence ou en absence de doxorubicine, un médicament connu pour induire l'apoptose de cette lignée cellulaire *in vitro.* Dans ces expériences, les cellules MCF-7 sont ensemencées à 2.10⁴/cm² en boîte de Pétri et cultivées 24 h dans du RPMI sans rouge de phénol supplémenté avec 10% de sérum de veau foetal (SVF). Les cellules sont ensuite lavées 2 fois avec du PBS et remises en culture dans du milieu à 0 % SVF. Un temps d'adaptation de 10 minutes à 37°C leur est laissé avant l'ajout des anticorps à 10µg/ml. Après 10 minutes supplémentaires à 37°C, on ajoute au milieu de culture l'IGF-I recombinant (Sigma) à une concentration finale de 50 ng/ml. Les cellules sont à nouveau laissées à 37°C pendant une heure pour permettre la fixation des anticorps et de l'IGF-I. Enfin, la doxorubicine (Sigma) est ajoutée au milieu de culture à 2µg/ml et les cellules sont incubées 24 heures à 37°C.

Les expériences ont également été menées avec la navelbine à une concentration de 10 µg/ml.

L'analyse de la viabilité cellulaire est effectuée par analyse au cytomètre de flux après marquage à l'annexine V-FITC (20 min, 4°C) et DAPI (2µg/ml). Le pourcentage de cellules mortes considéré est la population marquée Annexine + / DAPI +. L'anticorps 5C2 est utilisé comme isotype contrôle.

Les résultats représentés à la figure 36 montrent que la doxorubicine induit une apoptose chez 8 % des cellules MCF-7. Lorsque les cellules sont traitées conjointement avec l'anticorps 7C10 et la doxorubicine une augmentation significative de la mort cellulaire est observée. Un même effet est montré avec l'anticorps 7H2HM. Le même type de résultats a été observé lorsque l'anticorps est associé à la navelbine.

### Exemple 7. Stratégie de clonage des gènes codant pour les régions variables des chaînes lourde et légère de l'anticorps monoclonal (AcM) 7C10

L'ARN total a été extrait à partir de 10⁷ cellules d'hybridomes secrétant l'anticorps 7C10 en utilisant le TRI REAGENT™ (selon les instructions données par le fournisseur, SIGMA, T9424). Le premier brin de cADN a été synthétisé à l'aide du kit 'First strand cDNA synthesis' d'Amersham-Pharmacia (#27-9261-01, selon les instructions données par le fournisseur). Pour les deux chaînes, la réaction a été amorcée avec l'oligonucléotide Not I-d(T)18, compris dans le Kit.

L'hybride cADN : mARN ainsi obtenu a été utilisé pour l'amplification par PCR des gènes codant pour les chaînes lourde et légère de l'AcM 7C10. Les PCR ont été réalisées en utilisant une combinaison d'oligonucléotides spécifiques pour les chaînes lourdes et légères (Kappa) des immunoglobulines de souris. Les amorces correspondant aux extrémités 5' s'hybrident dans la région correspondant aux peptides de signal (Tableau 2 pour chaînes lourdes, Tableau 3 pour chaînes légères). Ces amorces ont été compilées à partir d'un grand nombre de séquences d'anticorps de souris trouvées dans les banques de données (Jones S.T. et al., Bio/Technology 9:88-89, 1991). Les amorces correspondant aux extrémités 3' s'hybrident dans les régions constantes des chaînes lourdes (domaine CH1 de la sous classe IgG1, non loin de la jonction V-C, amorce MHC-1 Tableau 4) et légères (domaine Kappa non loin de la jonction V-C, amorce MKC Tableau 4).

**TABLEAU 2 : Amorces oligonucléotidiques pour la région 5' des domaines variables des chaînes lourdes d'immunoglobuline de souris (MHV) ("MHV" pour "Mouse Heavy Variable")**

| | | |
|---|---|---|
| MHV-1 : | 5' ATGAAATGCAGCTGGGTCATSTTCTT 3' | (SEQ ID No. 13) |
| MHV-2 : | 5' ATGGGATGGAGCTRTATCATSYTCTT 3' | (SEQ ID No. 14) |
| MHV-3 : | 5' ATGAAGWTGTGGTTAAACTGGGTTTT 3' | (SEQ ID No. 15) |
| MHV-4 : | 5' ATGRACTTTGGGYTCAGCTTGRT 3' | (SEQ ID No. 16) |
| MHV-5 : | 5' ATGGACTCCAGGCTCAATTTAGTTTT 3' | (SEQ ID No. 17) |
| MHV-6 : | 5' ATGGCTGTCYTRGSGCTRCTCTTCTG 3' | (SEQ ID No. 18) |
| MHV-7 : | 5' ATGGRATGGAGCKGGRTCTTTMTCTT 3' | (SEQ ID No. 19) |
| MHV-8 : | 5' ATGAGAGTGCTGATTCTTTTGTG 3' | (SEQ ID No. 20) |
| MHV-9 : | 5' ATGGMTTGGGTGTGGAMCTTGCTATT 3' | (SEQ ID No. 21) |
| MHV-10 : | 5' ATGGGCAGACTTACATTCTCATTCCT 3' | (SEQ ID No. 22) |
| MHV-11 : | 5' ATGGATTTTGGGCTGATTTTTTTTATTG 3' | (SEQ ID No. 23) |
| MHV-12 : | 5'ATGATGGTGTTAAGTCTTCTGTACCT 3' | (SEQ ID No. 24) |
| NB KEY : | R=A/G, Y=T/C, W=A/T, K=T/G, M=A/C, S=C/G. | |

**TABLEAU 3 : Amorces oligonucléotidiques pour la région 5' des domaines variables des chaînes kappa (légères) d'immunoglobuline de souris (MKV) ("MKV" pour "Mouse Kappa Variable")**

| | | |
|---|---|---|
| MKV-1 : | 5' ATGAAGTTGCCTGTTAGGCTGTTGGTGCT 3' | (SEQ ID No. 25) |
| MKV-2 : | 5' ATGGAGWCAGACACACTCCTGYTATGGGT 3' | (SEQ ID No. 26) |
| MKV-3 : | 5' ATGAGTGTGCTCACTCAGGTCCT 3' | (SEQ ID No. 27) |
| MKV-4 : | 5' ATGAGGRCCCCTGCTCAGWTTYTTGG 3' | (SEQ ID No. 28) |
| MKV-5 : | 5' ATGGATTTWCAGGTGCAGATTWTCAGCTT 3' | (SEQ ID No. 29) |
| MKV-5A : | 5' ATGGATTTWCARGTGCAGATTWTCAGCTT 3' | (SEQ ID No. 30) |
| MKV-6 : | 5' ATGAGGTKCYYTGYTSAGYTYCTGRG 3' | (SEQ ID No. 31) |
| MKV-7 : | 5' ATGGGCWTCAAGATGGAGTCACA 3' | (SEQ ID No. 32) |
| MKV-8 : | 5' ATGTGGGGAYCTKTTTYCMMTTTTTCAAT 3' | (SEQ ID No. 33) |
| MKV-9 : | 5' ATGGTRTCCWCASCTCAGTTCCTT 3' | (SEQ ID No. 34) |
| MKV-10 : | 5' ATGTATATATGTTTGTTGTCTATTTC 3' | (SEQ ID No. 35) |
| MKV-11 : | 5' ATGGAAGCCCCAGCTCAGCTTCTCTT 3' | (SEQ ID No. 36) |
| MKV-12A : | 5' ATGRAGTYWCAGACCCAGGTCTTYRT 3' | (SEQ ID No. 37) |
| MKV-12B : | 5' ATGGAGACACATTCTCAGGTCTTTGT 3' | (SEQ ID No. 38) |
| MKV-13 : | 5' ATGGATTCACAGGCCCAGGTTCTTAT 3' | (SEQ ID No. 39) |
| NB KEY : | R=A/G, Y=T/C, W=A/T, K=T/G, M=A/C, S=C/G. | |

**TABLEAU 4 : Amorces oligonucléotidiques pour les extrémités 3' des gènes V_{H} et V_{L} de souris**

| Chaîne légère (MKC) : | |
|---|---|
| 5' ACTGGATGGTGGGAAGATGG 3' | (SEQ ID No. 40) |

| Région constante du domaine Kappa de souris : | |
|---|---|
| | (SEQ ID No. 41) |
| | (SEQ ID No. 42) |
| | |
| Chaîne lourde (MHC-1) | |
| 5' CCAGTGGATAGACAGATG 3' | (SEQ ID No. 44) |

| Domaine CH1 de gamma-1 de souris (sous-classe IgG1) : | |
|---|---|
| | (SEQ ID No. 46) |
| | (SEQ ID No. 45) |
| | (SEQ ID No. 47) |

### Exemple 8. Séquences des immunoglobulines clonées à partir de l'hybridome de souris 7C10

En suivant la stratégie d'amplification décrite ci-dessus, des produits de PCR correspondant aux régions variables des chaînes lourde (VH) et légère (VL) ont été clonés en utilsant le « pGEM®-T Easy Vector Systems » (Promega). Pour 7C10 VL, des produits de PCR ont été obtenus avec l'amorce MKC en combinaison avec les amorces MKV1 et MKV2. Pour 7C10 VH, des produits de PCR ont été obtenus avec l'amorce MHC-1 en association avec les amorces MHV8 et MHV12. Un séquençage approfondi des produits de PCR clonés dans les vecteurs pGEM-T easy a révélé deux séquences différentes pour la chaîne légère et une séquence unique pour la chaîne lourde.

### a) Région variable isolée à partir de l'oligo MKV1

La séquence d'ADN obtenue est caractéristique d'une région variable d'Ig fonctionnelle. Cette nouvelle séquence est donc présumée être celle codant pour 7C10 VL. Les séquences ADN (SEQ ID Nos. 48 et 50) et acides aminés (SEQ ID No. 49) du cDNA codant pour 7C10 VL sont représentées à la figure 14.

### b) Région variable isolée à partir de l'oligo MKV2

Le gène codant pour cette chaîne légère provient d'un transcrit de mRNA aberrant qui est présent dans tous les partenaires de fusion standard dérivés de la tumeur originelle MOPC-21 dont fait parti le myélome de souris Sp2/Oag14 qui a été utilisé pour produire l'hybridome 7C10. Cette séquence comporte une recombinaison aberrante entre les gènes V et J (délétion de quatre bases nucléotidiques entraînant un changement du cadre de lecture) et une mutation de la cystéine invariable en position 23 en tyrosine. Ces changements suggèrent que cette chaîne légère serait non fonctionnelle bien que néanmoins transcrite en ARN messager. La séquence ADN de cette pseudo chaîne légère n'est pas montrée.

### c) Région variable isolée à partir des oligos MHV8 et MHV12

Les séquences ADN obtenues avec ces deux oligos sont identiques, en dehors de la séquence codée par l'oligo lui-même. Cette séquence est une nouvelle séquence codant pour une chaîne lourde fonctionnelle présumée être celle de l'anticorps monoclonal 7C10. Les séquences ADN (SEQ ID Nos. 51 et 53) et acides aminés (SEQ ID No. 52) du cDNA codant pour 7C10 VH sont représentées à la figure 15.

### Exemple 9. Construction des gènes chimériques souris-homme

L'anticorps chimérique 7C10 a été construit de manière à avoir les régions 7C10 VL et VH de souris reliées aux régions constantes humaines kappa et gamma-1, respectivement. Des oligos ont été utilisés pour modifier les extrémités 5' et 3' des séquences flanquant l'ADN codant pour 7C10 VL et VH afin de permettre leur clonage dans des vecteurs d'expression en cellules mammaliennes. Ces vecteurs utilisent le promoteur fort HCMV pour transcrire efficacement les chaînes lourde et légère de l'anticorps chimérique 7C10. D'autre part, ces vecteurs contiennent également l'origine de réplication de SV40 permettant une réplication efficace de l'ADN et par voie de conséquence une expression transitoire des protéines en cellules cos.

### Exemple 10. Expression et évaluation de l'activité de reconnaissance de l'IGF-1 récepteur de l'anticorps chimérique 7C10

Les deux plasmides contenant l'ADN codant pour l'anticorps 7C10 chimérique ont été co-transfectés dans des cellules cos-7 (ATCC number CRL-1651) pour étudier l'expression transitoire de l'anticorps recombinant. Après 72 heures d'incubation, le milieu de culture a été prélevé, centrifugé pour éliminer les débris cellulaires et analysé par technique ELISA pour la production en IgG1 humaine (voir Exemple 16) et la reconnaissance du récepteur à l'IGF-1 (voir Exemple 17).

Les tests ELISA de mesure de concentrations en IgG1/Kappa humaines ont montré que l'expression de l'anticorps chimérique 7C10 dans les cellules cos7 était comprise entre 300 et 500 ng/ml ce qui est comparable aux valeurs obtenues avec la majorité des anticorps.

Les tests ELISA de reconnaissance du récepteur à l'IGF-1 montrent que l'anticorps chimérique le reconnaît spécifiquement et avec une bonne avidité relative (voir figures 3A, 3B et 3C). Ceci apporte la preuve fonctionnelle que les bonnes VH et VL de l'anticorps 7C10 ont été identifiées. De plus, cette forme chimérique de 7C10 apparaît comme étant un outil indispensable à l'évaluation de l'affinité des formes humanisées.

### Exemple 11. Modélisation moléculaire des régions variables de l'anticorps de souris 7C10

Afin d'aider et d'affiner le processus d'humanisation par « CDR-grafting », un modèle moléculaire des régions VL et VH de l'anticorps de souris 7C10 a été construit. Le modèle est basé sur la structure cristallographique de la chaîne lourde 1AY1 et de la chaîne légère 2PCP.

### Exemple 12. Processus d'humanisation par CDR-grafting de la région variable de la chaîne légère de l'anticorps 7C10 (7C10 VL)

### a) Comparaison de la séquence en acides aminés de 7C10 VL avec toutes les séquences de souris VL connues

Comme étape préliminaire à l'humanisation par CDR-grafting, la séquence en acides aminés de 7C10 VL a été comparée dans un premier temps à toutes les séquences de VL de souris présentes dans la banque de données de Kabat (adresse Internet : ftp://ftp.ebi.ac.uk/pub/database/kabat/fasta_format/, dernière mise à jour des données date de 1999). 7C10 VL a ainsi été identifié comme appartenant au sous-groupe II des chaînes légères Kappa comme défini par Kabat et al. (In Sequences of proteins of immunological interest (5th edn.), NIH publication No 91-3242, US Department of Health and Human Services, Public Health Service, National Institutes of Health, Bethesda, 1991). Des régions VL d'anticorps monoclonaux de souris ayant une identité de séquences allant jusqu'à 95 % ont été identifiées (DRB1-4.3 (SEQ ID No. 55) : 95 % et C94-5B11'CL (SEQ ID No. 56) : 95 %, voir figure 17). Afin de tenter d'identifier les résidus hors du commun dans la séquence de 7C10 VL, la séquence en acides aminés de 7C10 VL (SEQ ID No. 54) a été alignée avec la séquence consensus du sous-groupe II des chaînes kappa de souris (SEQ ID No. 57) comme défini par Kabat (voir figure 17).

A la position Kabat numéro 3, la valine (V) normalement présente dans le sous-groupe II des chaînes légères Kappa selon Kabat (71 %) est remplacée par une leucine (L). Une leucine à cette position n'est pas rare puisqu'on la trouve par exemple dans DRB1-4.3 et C94-5B11'CL. D'après le modèle moléculaire, ce résidu ne semble pas jouer un rôle particulier. En conséquence la conservation de ce résidu dans la forme humanisée ne sera pas envisagée.

A la position Kabat numéro 7, la thréonine (T) normalement présente dans le sous-groupe II des chaînes légères Kappa selon Kabat (66 %) est remplacée par une isoleucine (I). Une isoleucine à cette position est relativement rare puisqu'on ne la trouve que 15 fois parmi toutes les séquences VL de souris connues et jamais parmi des séquences VL humaines. Le modèle moléculaire montre que ce résidu (17) pointe vers la surface de la molécule mais ne contacte pas les CDRs (le résidu d'un CDR le plus proche serait l'arginine à la position Kabat numéro 42). De plus, il semble peu probable que ce résidu 17 contacte directement l'antigène. En conséquence la conservation de ce résidu dans la forme humanisée ne sera pas envisagée du moins dans un premier temps.

A la position Kabat numéro 77, l'arginine (R) normalement présente dans le sous-groupe II des chaînes légères Kappa selon Kabat (95,5 %) est remplacée par une sérine (S). Une Sérine à cette position n'est pas rare.

### b) Comparaison de la séquence en acides aminés de 7C10 VL avec toutes les séquences humaines VL connues

Afin d'identifier le meilleur candidat humain pour le « CDR-grafting » on a recherché la région VL kappa d'origine humaine ayant la plus grande homologie possible avec 7C10 VL. A cette fin, on a comparé la séquence en acides aminés de 7C10 VL kappa de souris avec toutes les séquences VL kappa humaines présentes dans la base de données de Kabat. 7C10 VL de souris avait la plus grande homologie de séquence avec les régions VL kappa humaines du sous-groupe II comme défini par Kabat et al. (1991). Des régions VH d'anticorps monoclonaux d'origine humaine ont été identifiées ayant une identité de séquences allant jusqu'à 75,9 % (GM607 (SEQ ID No. 58), voir figure 18) sur la totalité des 112 acides aminés composant la région variable. Une lignée germinale d'origine humaine, DPK15/A19 (SEQ ID No. 59), ayant une identité de séquence de 76 % (voir figure 18) fut aussi identifiée. GM607 (Klobeck et al., 1984). GM607 fut donc choisi comme séquence humaine receveuse des CDRs (selon la définition de Kabat) de 7C10 VL de souris. En comparant les séquences de GM607 avec celle de la séquence consensus du sous-groupe II humain (SEQ ID No. 60) (figure 18), aucun résidu particulier dans les régions charpentes (Rch) ne put être identifié, indiquant par là même que GM607 était un bon candidat pour le CDR-grafting.

### c) Versions humanisées de 7C10 VL

L'étape suivante dans le procédé d'humanisation consista à joindre les CDRs de 7C10 VL de souris aux régions charpentes (Rch) de la chaîne légère humaine sélectionnée, GM607 (Klobeck et al., 1984). A ce stade du procédé le modèle moléculaire des régions Fv de souris de 7C10 est particulièrement utile dans le choix des résidus de souris à conserver car pouvant jouer un rôle soit dans le maintien de la structure tridimensionnelle de la molécule (structure canonique des CDRs, interface VH/VL, etc.) ou dans la liaison à l'antigène. Dans les Rch, chaque différence entre les acides aminés de souris (7C10 VL) et humain (GM607) a été examinée scrupuleusement (voir Tableau 5). De plus, les résidus particuliers à la séquence 7C10 VL de souris que l'on avait identifiés (voir Exemple 12.a)) ont été pris en compte si besoin était.

Dans la première version humanisée par « CDR-grafting » de 7C10 VL, humain 1, un seul changement dans les régions charpentes (Rch) de GM607 a été effectué. Ce changement concerne le résidu 2 (nomenclature de Kabat) situé dans Rch 1. Ce résidu entre en effet dans la composition de la structure canonique du CDR 1 de 7C10 VL et pourrait donc être critique pour le maintien de cette loupe dans sa bonne conformation. La valine présente à cette position dans la séquence 7C10 VL de souris est donc conservée à cette même position dans la forme humanisée (voir le Tableau 5 et la figure 19 pour la séquence en acides aminés (SEQ ID No. 61) et la figure 20 pour la séquence ADN (SEQ ID Nos. 62 et 64) et la séquence en acides aminés comprenant le peptide signal (SEQ ID No. 63).

Dans la deuxième version humanisée par « CDR-grafting » de 7C10 VL, humain 2, aucun changement dans les Rchs de la chaîne légère humaine GM 607 n'a été fait. Tous les résidus des Rchs sont donc d'origine humaine y compris le résidu 2 qui a donc été muté pour remplacer la valine présente dans 7C10 VL de souris par une isoleucine trouvée à cette même position dans la chaîne légère humaine GM 607 (voir le Tableau 5 et la figure 19 pour la séquence en acides aminés (SEQ ID No. 65) et la figure 21 pour la séquence ADN (SEQ ID Nos. 66 et 68) et la séquence en acides aminés comprenant le peptide signal (SEQ ID No. 67)). Cette forme humaine 2 est donc totalement humanisée (en dehors bien entendu des CDRs eux-mêmes) puisque tous les résidus des Rchs sont ceux de la chaîne légère d'origine humaine, GM 607.

**TABLEAU 5 : Alignement des séquences d'amino acides ayant conduit au dessin des régions 7C10 V_{L} humaines refaçonnées**

| **Kabat** | **#** | **FR ou CDR** | **Chaîne légère 7C10 de souris** | **Lignée germinale humaine DPK15/A19** | **GM 607** | **7C10 L humain 1 refaçonné** | **7C10 L humain 2 refaçonné** | **Commentaires** |
|---|---|---|---|---|---|---|---|---|
| 1 | 1 | FR1 | D | D | D | D | D | |
| 2 | 2 | \| | V* | I* | I* | V* | I* | Cano L1 4(16) Zone Vernier |
| 3 | 3 | \| | L | V | V | V | V | |
| 4 | 4 | \| | M | M | M | M | M | Zone Vernier |
| 5 | 5 | \| | T | T | T | T | T | |
| 6 | 6 | \| | Q | Q | Q | Q | Q | |
| 7 | 7 | \| | I | S | S | S | S | |
| 8 | 8 | \| | P | P | P | P | P | |
| 9 | 9 | \| | L | L | L | L | L | |
| 10 | 10 | \| | S | S | s | s | s | |
| 11 | 11 | \| | L | L | L | L | L | |
| 12 | 12 | \| | P | P | P | P | P | |
| 13 | 13 | \| | V | V | V | V | V | |
| 14 | 14 | \| | S | T | T | T | T | |
| 15 | 15 | \| | L | P | P | P | P | |
| 16 | 16 | \| | G | G | G | G | G | |
| 17 | 17 | \| | D | E | E | E | E | |
| 18 | 18 | \| | Q | P | P | P | P | |
| 19 | 19 | \| | A | A | A | A | A | |
| 20 | 20 | \| | S | S | S | S | S | |
| 21 | 21 | \| | I | I | I | I | I | |
| 22 | 22 | \| | S | S | S | S | S | |
| 23 | 23 | FR1 | C | C | C | C | C | |
| 24 | 24 | **CDR1** | **R** | **R** | **R** | **R** | **R** | |
| 25 | 25 | \| | **S*** | **S*** | **S*** | **S*** | **S*** | Cano L1 4(16) |
| 26 | 26 | \| | **S** | **S** | **s** | **S** | **S** | |
| 27 | 27 | \| | **Q** | **Q** | **Q** | **Q** | **Q** | |
| 27A | 28 | \| | **S** | **S** | **S** | **S** | **S** | |
| 27B | 29 | \| | **I*** | **L*** | **L*** | **i*** | **i*** | Cano L1 4(16) |
| 27C | 30 | \| | **V** | **L** | **L** | **i** | **I** | |
| 27D | 31 | \| | **H** | H | **H** | **H** | **H** | |
| 27E | 32 | \| | **S** | **S** | **s** | **s** | **S** | |
| 28 | 33 | \| | **N** | **N** | **N** | **N** | **N** | |
| 29 | 34 | \| | **G** | **G** | **G** | **G** | **G** | |
| 30 | 35 | \| | **N** | **Y** | **Y** | **n** | **N** | |
| 31 | 36 | \| | **T** | **N** | **N** | **t** | **T** | |
| 32 | 37 | \| | **Y** | **Y** | **Y** | **Y** | **Y** | |
| 33 | 38 | \| | **L*** | **L*** | **L*** | **L*** | **L*** | Cano L1 4(16) |
| 34 | 39 | **CDR1** | **Q** | **D** | **D** | **q** | **Q** | |
| 35 | 40 | FR2 | W | W | W | W | W | Zone Vernier |
| 36 | 41 | \| | Y | Y | Y | Y | Y | VH/VL inter Zone Vernier |
| 37 | 42 | \| | L | L | L | L | L | |
| 38 | 43 | \| | Q | Q | Q | Q | Q | VL/VH inter |
| 39 | 44 | \| | K | K | K | K | K | |
| 40 | 45 | \| | P | P | P | P | P | |
| 41 | 46 | \| | G | G | G | G | G | |
| 42 | 47 | \| | Q | Q | Q | Q | Q | |
| 43 | 48 | \| | S | S | S | S | S | |
| 44 | 49 | \| | P | P | P | P | P | VL/VH inter (+) |
| 45 | 50 | \| | K | Q | Q | Q | Q | |
| 46 | 51 | \| | L | L | L | L | L | VL/VH inter Zone Vernier |
| 47 | 52 | \| | L | L | L | L | L | Zone Vernier |
| 48 | 53 | \| | I | I | I | I* | I* | Cano L2 1 (7) Zone Vernier |
| 49 | 54 | FR2 | Y | Y | Y | Y | Y | Zone Vernier |
| 50 | 55 | **CDR2** | **K** | **L** | **L** | **k** | **K** | |
| 51 | 56 | \| | **V*** | **G*** | **G*** | **V*** | **V*** | Cano L2 1(7) |
| 52 | 57 | \| | **S*** | **S*** | **S*** | **S*** | **S*** | Cano L2 1(7) |
| 53 | 58 | \| | **N** | **N** | **N** | **N** | **N** | |
| 54 | 59 | \| | **R** | **R** | **R** | **R** | **R** | |
| 55 | 60 | \| | **L** | **A** | **A** | **I** | **L** | |
| 56 | 61 | **CDR2** | **Y** | **S** | **S** | **y** | **Y** | |
| 57 | 62 | FR3 | G | G | G | G | G | |
| 58 | 63 | \| | V | V | V | V | V | |
| 59 | 64 | \| | P | P | P | P | P | |
| 60 | 65 | \| | D | D | D | D | D | |
| 61 | 66 | \| | R | R | R | R | R | |
| 62 | 67 | \| | F | F | F | F | F | |
| 63 | 68 | \| | S | S | S | S | S | |
| 64 | 69 | \| | G* | G* | G* | G* | G* | Cano L2 1(7) Zone Vernier |
| 65 | 70 | \| | S | S | S | S | S | |
| 66 | 71 | \| | G | G | G | G | G | Zone Vernier |
| 67 | 72 | \| | S | S | S | S | S | |
| 68 | 73 | \| | G | G | G | G | G | Zone Vernier |
| 69 | 74 | \| | T | T | T | T | T | Zone Vernier |
| 70 | 75 | \| | D | D | D | D | D | |
| 71 | 76 | \| | F* | F* | F* | F* | F* | Cano L1 4(16) Zone Vernier |
| 72 | 77 | \| | T | T | T | T | T | |
| 73 | 78 | \| | L | L | L | L | L | |
| 74 | 79 | \| | K | K | K | K | K | |
| 75 | 80 | \| | I | I | I | I | I | |
| 76 | 81 | \| | S | S | S | S | S | |
| 77 | 82 | \| | S | R | R | R | R | |
| 78 | 83 | \| | V | V | V | V | V | |
| 79 | 84 | \| | E | E | E | E | E | |
| 80 | 85 | \| | A | A | A | A | A | |
| 81 | 86 | \| | E | E | E | E | E | |
| 82 | 87 | \| | D | D | D | D | D | |
| 83 | 88 | \| | L | V | V | V | V | |
| 84 | 89 | \| | G | G | G | G | G | |
| 85 | 90 | \| | V | V | V | V | V | |
| 86 | 91 | \| | Y | Y | Y | Y | Y | |
| 87 | 92 | \| | Y | Y | Y | Y | Y | VL/VH inter |
| 88 | 93 | FR3 | C | C | C | C | c | |
| 89 | 94 | **CDR3** | **F** | **M** | **M** | **f** | **F** | VL/VH inter |
| 90 | 95 | \| | **Q*** | **Q*** | **Q*** | **Q*** | **Q*** | Cano L3 1 (9) |
| 91 | 96 | \| | **G** | **A** | **A** | **g** | **G** | VL/VH inter |
| 92 | 97 | \| | **S** | **L** | **L** | **S** | **S** | |
| 93 | 98 | \| | **H** | **Q** | **Q** | **h** | **H** | |
| 94 | 99 | \| | **V** | **T** | **T** | **V** | **V** | |
| 95 | 100 | \| | **P*** | **P*** | **P*** | **P*** | **P*** | Cano L3 1(9) |
| 96 | 101 | \| | **W** | | **Q** | **W** | **W** | VL/VH inter (+) |
| 97 | 102 | **CDR3** | **T** | | **T** | **T** | **T** | |
| 98 | 103 | FR4 | F | | F | F | F | VL/VH inter (+) Zone Vernier |
| 99 | 104 | \| | G | | G | G | G | |
| 100 | 105 | \| | G | | Q | Q | Q | |
| 101 | 106 | \| | G | | G | G | G | |
| 102 | 107 | \| | T | | T | T | T | |
| 103 | 108 | \| | K | | K | K | K | |
| 104 | 109 | \| | L | | V | V | V | |
| 105 | 110 | \| | E | | E | E | E | |
| 106 | 111 | \| | I | | I | I | I | |
| 107 | 112 | FR4 | K | | K | K | K | |

**Légende :** La première colonne (Kabat) indique la position du résidu d'acide aminé selon Kabat et al. (1991) ; la deuxième colonne (#) indique la position du résidu d'acide aminé dans la séquence régulière ; la troisième colonne (FR ou CDR) a été réalisée pour identifier facilement les segments du squelette (FR1, FR2, FR3 et FR4) et les segments CDR (CDR1, CDR2 et CDR3) ("CDR" pour "Complementary-Determining Region") avec les trois CDRs séparant les quatre FRs ; la quatrième colonne (Chaîne légère de souris 7C10) représente la séquence d'amino acides (SEQ ID No. 54) de la région V_{L} de l'anticorps de souris 7C10 ; la cinquième colonne (Lignée germinale humaine DPK15/A19) représente la séquence d'amino acides (SEQ ID No. 59) de la chaîne légère humaine V kappa II de la lignée germinale ; la sixième colonne (GM 607) représente la séquence d'amino acides (SEQ ID No. 58) de la région V_{L} de l'anticorps GM 607 humain ; les septième et huitième colonnes (7C10 L humains 1 et 2 refaçonnés) représentent les séquences d'amino acides des anticorps 7C10 VL humanisée 1 et 2 (respectivement SEQ ID Nos. 61 et 65). "*" indique les parties de la structure canonique de la boucle CDR telle que définie par Chothia et al. (Nature, 342, 877-883, 1989).

### Exemple 13. Processus d'humanisation par CDR-grafting de la région variable de la chaîne lourde de l'anticorps 7C10 (7C10 VH)

### a) Comparaison de la séquence en acides aminés de 7C10 VH avec toutes les séquences de souris VH connues

Comme étape préliminaire à l'humanisation par CDR-grafting, la séquence en acides aminés de 7C10 VH a été comparée dans un premier temps à toutes les séquences de VH de souris présentes dans la banque de données de Kabat (adresse Internet : ftp://ftp.ebi.ac.uk/pub/database/kabat/fasta_format/, dernière mise à jour des données date de 1999). 7C10 VH a ainsi été identifié comme appartenant au sous-groupe I(A) des chaînes lourdes comme défini par Kabat et al. (1991). Des régions VH d'anticorps monoclonaux de souris ayant une identité de séquences allant jusqu'à 90,5 % ont été identifiées (AN03'CL (SEQ ID No. 70), voir figure 22). Afin de tenter d'identifier les résidus hors du commun dans la séquence de 7C10 VH, nous avons aligné la séquence en acides aminés de 7C10 VH (SEQ ID No. 69) avec la séquence consensus (SEQ ID No. 71) du sous-groupe I(A) des chaînes lourdes de souris comme défini par Kabat (voir figure 22).

Le résidu 17 (numérotation de Kabat), Thr pour la séquence consensus du sous-groupe I(A) et Ser dans 7C10 VH, est localisé à la surface de la molécule du côté de l'interface avec la région constante. Ce résidu ne semble pas important.

Le résidu 27 (numérotation de Kabat), Asp pour la séquence consensus du sous-groupe I(A) et Tyr dans 7C10 VH, est un résidu canonique pour le CDR 1. Tyr à cette position n'est pas rare et est probablement critique pour le maintien du CDR 1 dans sa bonne conformation.

Le résidu 84 (numérotation de Kabat), Thr pour la séquence consensus du sous-groupe I(A) et Asn dans 7C10 VH. Asn, a été trouvé 93 fois dans VH de souris et 3 fois dans VH humaine. D'après le modèle moléculaire, c'est un résidu de surface éloigné du paratope.

La numérotation des acides aminés est celle de Kabat et al. (1991). Les résidus dans les régions charpentes (hors CDRs) qui diffèrent entre 7C10 VH et Kabat sous-groupe I(A) de souris sont soulignés. AN03'CL représente la séquence de la chaîne lourde d'un anticorps de souris (numéro d'accès dans la banque de données de Kabat est P001289).

### b) Comparaison de la séquence en acides aminés de 7C10 VH avec toutes les séquences humaines VH connues

Afin d'identifier le meilleur candidat humain pour le « CDR-grafting », on a recherché la région VH d'origine humaine ayant la plus grande homologie possible avec 7C10 VH. A cette fin on a comparé la séquence en acides aminés de 7C10 VH de souris avec toutes les séquences VH humaines présentes dans la base de données de Kabat. 7C10 VH de souris avait la plus grande homologie de séquence avec les régions VH humaines du sous-groupe II comme défini par Kabat et al. (1991). Des régions VH d'anticorps monoclonaux d'origine humaine ont été identifiées ayant une identité de séquences allant jusqu'à 67,3 % (human VH FUR1'CL (SEQ ID No. 73, voir figure 23) sur la totalité des 98 acides aminés codés par le gène variable (c'est-à-dire hors CDR3 et région J). Une lignée germinale d'origine humaine, 4.22 VH IV (Sanz et al., 1989), ayant une identité de séquence de 68,4 %, selon les mêmes critères que pour VH FUR1'CL, fut aussi identifiée (human Germ-line (SEQ ID No. 74), voir figure 23). La séquence codée par la lignée germinale 4.22 VH IV fut choisie comme séquence humaine receveuse des CDRs (selon la définition de Kabat) de 7C10 VH de souris plutôt que VH FUR1'CL car en comparant les séquences de 4.22 VH IV et VH FUR1'CL avec celle de la séquence consensus du sous-groupe II humain (human Kabat sg II (SEQ ID No. 72), voir figure 23 et tableau 6), aucun résidu atypique dans les régions charpentes (Rch) ne put être identifié pour 4.22 VH IV alors que la présence de deux résidus atypiques (Gln et Arg aux positions 81 et 82A selon la nomenclature de Kabat, respectivement) furent identifiés dans la séquence codée par VH FUR1'CL.

### c) Versions humanisées de 7C10 VH

L'étape suivante dans le procédé d'humanisation consista à joindre les CDRs de 7C10 VH de souris aux régions charpentes (Rch) de la lignée germinale humaine 4.22 VH IV (Sanz et al., 1989). A ce stade du procédé, le modèle moléculaire des régions Fv de souris de 7C10 est particulièrement utile dans le choix des résidus de souris à conserver car pouvant jouer un rôle soit dans le maintien de la structure tridimensionnelle de la molécule (structure canonique des CDRs, interface VH/VL, etc.) ou dans la liaison à l'antigène (appartenance au paratope). Dans les Rch, chaque différence entre les acides aminés de souris (7C10 VH) et humain (4.22 VH IV) a été examinée scrupuleusement (voir Tableau 6). De plus, les résidus particuliers à la séquence 7C10 VH de souris que l'on avait identifiés (voir Exemple 8.a)) ont été pris en compte si besoin était.

Dans la première version humanisée par «CDR-grafting» de 7C10 VH, humanisé 1, quatre changements dans les régions charpentes (Rch) de 4.22 VH IV ont été effectués (voir le Tableau 6, figure 24 pour la séquence en acides aminés (SEQ ID No. 75) et la figure 25 pour la séquence d'ADN (SEQ ID Nos. 76 et 78) et la séquence d'acides aminés comprenant le peptide signal (SEQ ID No. 77)). Ces quatre changements concernent :
- Le résidu 30 (nomenclature de Kabat) situé dans Rch 1. Ce résidu entre en effet dans la composition structurale du CDR1 de 7C10 VH (comme défini par Chothia et al., 1989) et pourrait donc être critique pour le maintien de cette loupe dans sa bonne conformation. La Thr présente à cette position dans la séquence 7C10 VH de souris est donc conservée à cette même position dans la forme humanisée.
- Le résidu 48 (nomenclature de Kabat) situé dans Rch 2. Ce résidu est proche des CDRs, bien que d'après le modèle moléculaire pas en contact direct avec ces derniers, et pourrait influer sur leur conformation fine. La méthionine présente à cette position dans la séquence 7C10 VH de souris est donc conservée à cette même position dans la forme humanisée 1.
- Le résidu 67 (nomenclature de Kabat) situé dans Rch 3. Ce résidu est proche des CDRs et d'après le modèle moléculaire pourrait contacter la Lysine 60 (nomenclature de Kabat) dans le CDR 2. L'isoleucine présente à cette position dans la séquence 7C10 VH de souris est donc conservée à cette position dans la forme humanisée 1.
- Le résidu 71 (nomenclature de Kabat) situé dans Rch 3. Ce résidu fait partie de la structure canonique du CDR 2 et devrait donc être critique pour maintenir cette loupe dans sa bonne conformation. L'arginine présente à cette position dans la séquence 7C10 VH de souris est donc conservée à cette position dans la forme humanisée 1.

Dans la deuxième version humanisée par «CDR-grafting» de 7C10 VH, humanisé 2, deux changements dans les régions charpentes (Rch) de 4.22 VH IV ont été effectués. Ces deux changements concernent les résidus 30 et 71 (nomenclature de Kabat), déjà décrits dans la forme humanisée 1 (voir le Tableau 6, figure 24 pour la séquence en acides aminés (SEQ ID No. 79) et la figure 26 pour la séquence d'ADN (SEQ ID Nos. 80 et 82) et la séquence d'acides aminés comprenant le peptide signal (SEQ ID No. 81)).

Dans la troisième forme humanisée par «CDR-grafting» de 7C10 VH, humanisé 3, aucun changement dans les régions charpentes (Rch) de 4.22 VH IV n'a été effectué. Tous les résidus des Rchs sont donc d'origine humaine y compris les résidus 30, 48, 67 et 71 (nomenclature de Kabat) qui ont été conservés (voir le Tableau 6, figure 24 pour la séquence en acides aminés (SEQ ID No. 83) et la figure 27 pour la séquence d'ADN (SEQ ID Nos. 84 et 86) et la séquence d'acides aminés comprenant le peptide signal (SEQ ID No. 85)). Cette forme humanisée 3 est donc totalement humanisée (en dehors bien entendu des CDRs eux-mêmes comme défini par Kabat) puisque tous les résidus des Rchs sont ceux codés par le gène VH de la lignée germinale 4.22 VH IV.

**TABLEAU 6 : Alignement des séquences d'amino acides ayant conduit au dessin des régions 7C10 V_{H} humaines refaçonnées**

| **Kabat** | **FR ou CDR** | **Chaîne lourde 7C10 de souris** | **Lignée germinale 4.22 VH IV** | **FUR1'CL VH humain** | **7C10 H humain 1 refaçonné** | **7C10 H humain 2 refaçonné** | **7C10 H humain 3 refaçonné** | **Commentaires** |
|---|---|---|---|---|---|---|---|---|
| 1 | FR1 | **D** | **Q** | **Q** | **Q** | **Q** | **Q** | |
| 2 | \| | V | V | V | V | V | V | Zone Vernier |
| 3 | \| | Q | Q | Q | Q | Q | Q | |
| 4 | \| | L | L | L | L | L | L | |
| 5 | \| | Q | Q | Q | Q | Q | Q | |
| 6 | \| | E | E | E | E | E | E | |
| 7 | \| | S | S | S | S | S | S | |
| 8 | \| | G | G | G | G | G | G | |
| 9 | \| | P | P | P | P | P | P | |
| 10 | \| | G | G | G | G | G | G | |
| 11 | \| | L | L | L | L | L | L | |
| 12 | \| | V | V | V | V | V | V | |
| 13 | \| | K | K | K | K | K | K | |
| 14 | \| | P | P | P | P | P | P | |
| 15 | \| | S | S | S | S | S | S | |
| **16** | \| | **Q** | **E** | **E** | **E** | **E** | **E** | |
| 17 | \| | **S** | **T** | **T** | **T** | **T** | **T** | |
| 18 | \| | L | L | L | L | L | L | |
| 19 | \| | S | S | S | S | S | S | |
| 20 | \| | L | L | L | L | L | L | |
| 21 | \| | T | T | T | T | T | T | |
| 22 | \| | C | C | C | C | C | C | |
| 23 | \| | S | **T** | **T** | **T** | **T** | **T** | |
| 24 | \| | V | V | V | V* | V* | V* | H1 2(6) canonique |
| 25 | \| | **T** | **S** | **S** | **S** | **S** | **S** | |
| 26 | \| | G* | G* | G* | G* | G* | G* | H1 2(6) canonique |
| 27 | \| | Y* | Y* | Y* | Y* | Y* | Y* | H1 2(6) canonique Zone Vernier |
| 28 | \| | S | S | S | S | S | S | Zone Vernier |
| 29 | \| | I* | I* | I* | I* | I* | I* | H1 2(6) canonique Zone Vernier |
| 30 | FR1 | **T** | **S** | **S** | **t** | **T** | **S** | Zone Vernier Proche des CDRs |
| 31 | **CDR1** | **G** | **S** | **S** | **g** | **G** | **g** | |
| 32 | \| | **G** | **G** | **G** | **G** | **G** | **G** | |
| 33 | \| | **Y** | **Y** | **Y** | **Y** | **Y** | **Y** | |
| 34 | \| | **L** | **Y** | **Y** | **I** | **L** | **I** | |
| 35 | \| | **W*** | **W*** | **W*** | **W*** | **W*** | **W*** | H1 2(6) canonique VH/VL interface |
| 35A | **CDR1** | **N** | **G** | **G** | **n** | **N** | **n** | |
| 36 | FR2 | W | W | W | W | W | W | |
| 37 | \| | I | I | I | I | I | I | VH/VL interface |
| 38 | \| | R | R | R | R | R | R | |
| 39 | \| | Q | Q | Q | Q | Q | Q | VH/VL interface |
| 40 | \| | **F** | **P** | **P** | **P** | **P** | **P** | |
| 41 | \| | P | P | P | P | P | P | |
| 42 | \| | G | G | G | G | G | G | |
| 43 | \| | **N** | **K** | **K** | **K** | **K** | **K** | |
| 44 | \| | **K** | **G** | **G** | **G** | **G** | **G** | |
| 45 | \| | L | L | L | L | L | L | VH/VL interface (+) |
| 46 | \| | E | E | E | E | E | E | |
| 47 | \| | W | W | W | W | W | w | VH/VL interface Zone Vernier |
| 48 | \| | **M** | **I** | **I** | **m** | **I** | **I** | Zone Vernier Proche des CDRs |
| 49 | FR2 | G | G | G | G | G | G | Zone Vernier |
| 50 | **CDR2** | **Y** | **S** | **s** | **y** | **Y** | **Y** | Zone Vernier |
| 51 | \| | **I** | **I** | **M** | **I** | **I** | **I** | |
| 52 | \| | **S** | **Y** | **F** | **S** | **S** | **S** | |
| 53 | \| | **Y** | **H** | **H** | **y** | **Y** | **y** | |
| 54 | \| | **D** | **S** | **S** | **d** | **D** | **d** | |
| 55 | \| | **G*** | **G*** | **G*** | **G*** | **G*** | **G*** | H2 1(16) canonique |
| 56 | \| | **T** | **S** | **S** | **t** | **T** | **t** | |
| 57 | \| | **N** | **T** | **S** | **n** | **N** | **n** | |
| 58 | \| | **N** | **Y** | **Y** | **n** | **N** | **n** | |
| 59 | \| | **Y** | **Y** | **Y** | **Y** | **Y** | **Y** | |
| 60 | \| | **K** | **N** | **N** | **k** | **K** | **k** | |
| 61 | \| | **P** | **P** | **P** | **P** | **P** | **P** | |
| 62 | \| | **S** | **S** | **S** | **S** | **S** | **S** | |
| 63 | \| | **L** | **L** | **L** | **L** | **L** | **L** | |
| 64 | \| | **K** | **K** | **K** | **K** | **K** | **K** | |
| 65 | **CDR2** | **D** | **S** | **S** | **d** | **d** | **d** | |
| 66 | FR3 | R | R | R | R | R | R | |
| 67 | \| | **I** | **V** | **V** | **i** | **V** | **V** | Zone Vernier Proche des CDRs |
| 68 | \| | **S** | **T** | **T** | **T** | **T** | **T** | |
| 69 | \| | I | I | I | I | I | I | Zone Vernier |
| 70 | \| | **T** | **S** | **S** | **S** | **S** | **S** | |
| 71 | \| | **R*** | **V*** | **V*** | **r*** | **r*** | **V*** | H2 1(16) canonique Zone Vernier |
| 72 | \| | D | D | D | D | D | D | |
| 73 | \| | T | T | T | T | T | T | Zone Vernier |
| 74 | \| | S | S | S | S | S | S | |
| 75 | \| | K | K | K | K | K | K | |
| 76 | \| | N | N | N | N | N | N | |
| 77 | \| | Q | Q | Q | Q | Q | Q | |
| 78 | \| | F | F | F | F | F | F | Zone Vernier |
| 79 | \| | **F** | **S** | **S** | **S** | **S** | **S** | |
| 80 | \| | L | L | L | L | L | L | |
| 81 | \| | K | K | Q | K | K | K | |
| 82 | \| | L | L | L | L | L | L | |
| 82A | \| | **N** | **s** | **R** | **S** | **S** | **S** | |
| 82B | \| | s | S | S | S | S | S | |
| 82C | \| | V | V | V | V | V | V | |
| 83 | \| | T | T | T | T | T | T | |
| 84 | \| | **N** | **A** | **A** | **A** | **A** | **A** | |
| 85 | \| | **E** | **A** | **A** | **A** | **A** | **A** | |
| 86 | \| | D | D | D | D | D | D | |
| 87 | \| | T | T | T | T | T | T | |
| 88 | \| | A | A | A | A | A | A | |
| 89 | \| | **T** | **V** | **V** | **V** | **V** | **V** | |
| 90 | \| | Y | Y | Y | Y | Y | Y | |
| 91 | \| | Y | Y | Y | Y | Y | Y | VH/VL interface |
| 92 | \| | C | C | C | C | C | C | |
| 93 | \| | A | A | A | A | A | A | VH/VL interface Zone Vernier |
| 94 | FR3 | R* | R* | R* | R* | R* | R* | H1 2(6) canonique Zone Vernier |
| 95 | CDR3 | **Y** | | **G** | **Y** | **y** | **y** | VH/VL interface |
| 96 | \| | **G** | | **R** | **g** | **g** | **g** | |
| 97 | \| | **R** | | **Y** | **r** | **r** | **r** | |
| 98 | \| | **V** | | **C** | v | **v** | **v** | |
| 99 | \| | **F** | | **S** | **f** | **f** | **f** | |
| 100 | \| | | | **S** | | | | |
| 100A | \| | | | **T** | | | | |
| 100B | \| | | | **S** | | | | |
| 100C | \| | | | **C** | | | | |
| 100D | \| | | | **N** | | | | |
| 100 E | \| | | | **w** | | | | |
| 100K | \| | **F** | | **F** | **f** | **f** | **f** | VH/VL interface (+) |
| 101 | \| | **D** | | **D** | **d** | **d** | **d** | |
| 102 | **CDR3** | **Y** | | **P** | **y** | **y** | **y** | |
| 103 | FR4 | W | | W | W | W | W | VH/VL interface (+) Zone Vernier |
| 104 | \| | G | | G | G | G | G | |
| 105 | \| | Q | | Q | Q | Q | Q | |
| 106 | \| | G | | G | G | G | G | |
| 107 | \| | T | | **T** | **T** | **T** | T | |
| 108 | \| | **T** | | **L** | **L** | **L** | **L** | |
| 109 | \| | **L** | | **V** | **V** | **V** | V | |
| 110 | \| | T | | T | T | T | T | |
| 111 | \| | V | | V | V | V | V | |
| 112 | \| | S | | S | S | S | S | |
| 113 | FR4 | S | | S | S | S | S | |

| | | | | | | | | |
|---|---|---|---|---|---|---|---|---|
| Légende : La première colonne (Kabat) indique la position du résidu d'acide aminé selon Kabat et al. (1991) ; la deuxième colonne (FR ou CDR) a été réalisée pour identifier facilement les segments du squelette (FR1, FR2, FR3 et FR4) et les segments CDR (CDR1, CDR2 et CDR3) avec les trois CDRs séparant les quatre FRs ; la troisième colonne (Chaîne lourde de souris 7C10) représente la séquence d'amino acides (SEQ ID No. 69) de la région V_{H} de l'anticorps de souris 7C10 ; la quatrième colonne (Lignée germinale 4.22 VH IV) représente la séquence d'amino acides du gène 4.22 VH IV (Sanz et al., 1989) (SEQ ID No. 74) ; la cinquième colonne (FUR1'CL VH humaine, kabat numéro d'accession N020619) représente la séquence d'amino acides (SEQ ID No. 73) IgMK antilamine B d'origine humaine (Mariette et al., 1993) ; les sixième, septième et huitième colonnes (7C10 H humains 1, 2 et 3 refaçonnés) représentent les séquences d'amino acides de la région V_{H} de 7C10 humain refaçonné respectivement pour les versions 1 (SEQ ID No. 75), 2 (SEQ ID No. 79) et 3 (SEQ ID No. 83). "*" indique les parties de la structure canonique de la boucle CDR telle que définie par Chothia et al. (1989). | | | | | | | | |

### Exemple 14. Construction des gènes codant pour les versions humanisées 1 de 7C10 VL et VH par assemblage d'oligonucléotides.

### a) Principe

Les gènes (peptide leader + régions variables VDJ pour VH ou VJ pour VK) codant pour les régions variables humanisées ont été synthétisés par assemblage en phase solide sur billes magnétiques cotées à la streptavidine. Les gènes codant pour 7C10 VH humanisée (445 paires de bases) et 7C10 VL humanisée (433 paires de bases) sont construits en fusionnant deux fragments d'ADN grâce à la présence d'un site de restriction KpnI présent dans les deux séquences et situé à peu près à la moitié du gène (à 200 et 245 nucléotides par rapport à l'extrémité 5' du gène pour VL et VH, respectivement). Les deux fragments qui sont fusionnés ensemble sont eux-mêmes assemblés par une technique d'assemblage qui consiste à utiliser des oligonucléotides phosphorylés (environ 30-35 mer) hybridés deux par deux (un oligo sens et l'autre antisens, sur une homologie d'environ 50 %) de façon à ce qu'ils se chevauchent lors de l'élongation. Un premier oligonucléotide biotinylé en 5' est fixé sur les billes magnétiques puis les paires d'oligonucléotides phosphorylés sont rajoutées une par une. La liaison phosphodiester entre les oligonucléotides phosphorylés juxtaposés est réalisée par l'enzyme T4 DNA ligase.

Les gènes ainsi synthétisés *de novo* peuvent être directement clonés (par digestion avec des enzymes de restriction compatibles avec le vecteur d'expression choisi) ou amplifiés par PCR pour obtenir plus de matériel en préambule au clonage directionnel par digestion enzymatique. La séquence du gène ainsi construit par assemblage *de novo* est alors vérifiée par séquençage automatique de l'ADN.

### b) Protocole expérimental de la technique d'assemblage de novo

Des oligonucléotides phosphorylés en 5' ou biotinylés en 5' dont la concentration a été ajustée à 100 µM ont été commandés chez MWG Biotech (voir les séquences des oligonucléotides utilisés dans le Tableau 7 pour la construction de 7C10 VL humanisée, et le Tableau 8 pour la construction de 7C10 VH humanisée). Les oligonucléotides ont été hybridés par paires (un mélange équimolaire, 500 pmoles, d'un oligo sens et d'un oligo antisens dans le tampon T4 DNA ligase est chauffé à 95°C pendant 5 minutes puis laissé à refroidir sur la paillasse jusqu'à température ambiante) selon un schéma décrit dans le Tableau 9.

Le premier oligonucléotide biotinylé est fixé sur des billes magnétiques cotées à la streptavidine (Dynabeads M-280 Streptavidin, Dynal product no 112-05). Pour cela, à 50 µl de billes décantées (utilisation d'un porte aimant) préalablement lavées deux fois avec 100 µl de tampon TE 1X (tampon Tris-EDTA 100X : 1M Tris-HCl, pH 8, 0,1 M EDTA, Sigma T-9285) on ajoute 500 pmol de l'oligonucléotide biotinylé dans une solution NaCl à 15 mM. Après une incubation à 37°C pendant 15 min, les billes sont lavées deux fois avec le tampon de lavage (10 mM Tris-HCl pH 7,6, 10 mM EDTA et 50 mM NaCl) et les couples d'oligonucléotides hybridés sont alors ajoutés un par un. A chaque rajout d'une paire d'oligonucléotides on chauffe à 95°C pendant 5 min puis on laisse refroidir sur la paillasse jusqu'à température ambiante. Une fois la température ambiante atteinte, on ajoute de 2 µl de T4 DNA ligase à 10 U/µl (Biolabs) et on incube pendant 20 min à 37°C. Les billes sont ensuite lavées (tampon de lavage) et les paires d'oligonucléotides suivantes sont ajoutées ainsi de suite.

Le dernier oligo (antisens) non apparié est assemblé de la façon suivante. 5 µl d'oligo (500 pmol) et 43 µl de tampon T4 DNA ligase sont ajoutés aux billes décantées puis on chauffe le mélange à 95°C pendant 5 min et on le laisse refroidir sur la paillasse jusqu'à température ambiante. Une fois la température ambiante atteinte on ajoute 2 µl de T4 DNA ligase et on incube à 37°C pendant 20 min. Les billes sont ensuite lavées deux fois avec le tampon de lavage puis deux fois avec le tampon TE 1X.

Les billes peuvent alors être conservées à 4°C avant de procéder au clonage et séquençage du gène assemblé *de novo.*

**TABLEAU 7 : Séquence d'ADN des oligonucléotides ayant été utilisés pour la construction de 7C10 VL humanisée 1 par assemblage de novo**

| | | |
|---|---|---|
| LeaderMluI.biotin | 5'-GTCAGAACGCGTGCCGCC | (SEQ ID No. 87) |
| 7C10Lresh.1sens | 5'-ACCATGAAGTTGCCTGTTAGGCTGTTGGTGCT | (SEQ ID No. 88) |
| 7C10Lresh.2sens | 5'-GATGTTCTGGTTTCCTGCTTCCAGCAGTGATG | (SEQ ID No. 89) |
| 7C10Lresh.3sens | 5'-TTGTGATGACTCAGTCTCCACTCTCCCTGCCC | (SEQ ID No. 90) |
| 7C10Lresh.4sens | 5'-GTCACCCCTGGAGAGCCGGCCTCCATCTCCTG | (SEQ ID No. 91) |
| 7C10Lresh.5sens | 5'-CAGGTCTAGTCAGACCATTATACATAGTAATG | (SEQ ID No. 92) |
| 7C10Lresh.6sens | 5'-GAAACACCTATTTGGAATGGTACCTGCAGA | (SEQ ID No. 93) |
| 7C10Lresh.7anti | 5'-GGCAACTTCATGGTGGCGGCACGCGTTCTGAC | (SEQ ID No. 94) |
| 7C10Lresh.8anti | 5'-GAAACCAGAACATCAGCACCAACAGCCTAACA | (SEQ ID No. 95) |
| 7C10Lresh.9anti | 5'-CTGAGTCATCACAACATCACTGCTGGAAGCAG | (SEQ ID No. 96) |
| 7C10Lresh.10anti | 5'-TCTCCAGGGGTGACGGGCAGGGAGAGTGGAGA | (SEQ ID No. 97) |
| 7C10Lresh.11anti | 5'-TCTGACTAGACCTGCAGGAGATGGAGGCCGGC | (SEQ ID No. 98) |
| 7C10Lresh.12anti | 5'-AAATAGGTGTTTCCATTACTATGTACAATGC | (SEQ ID No. 99) |
| 7C10Lresh.13sens | 5'-CAGGGCAGTCTCCACAGCTCCTGATCTATAAA | (SEQ ID No. 100) |
| 7C10Lresh.14sens | 5'-GTTTCTAATCGGCTTTATGGGGTCCCTGACAG | (SEQ ID No. 101) |
| 7C10Lresh.15sens | 5'-GTTCAGTGGCAGTGGATCAGGCACAGATTTTA | (SEQ ID No. 102) |
| 7C10Lresh.16sens | 5'-CACTGAAAATCAGCAGAGTGGAGGCTGAGGAT | (SEQ ID No. 103) |
| 7C10Lresh.17sens | 5'-GTTGGGGTTTATTACTGCTTTCAAGGTTCACA | (SEQ ID No. 104) |
| 7C10Lresh.18sens | 5'-TGTTCCGTGGACGTTCGGCCAAGGGACCAAGG | (SEQ ID No. 105) |
| 7C10Lresh.19sens | 5'-TGGAAATCAAACGTGAGTGGATCCTCTGCG | (SEQ ID No. 106) |
| 7C10Lresh.KpnIREV | 5'-TCTGCAGGTACCATTGC | (SEQ ID No. 107) |
| 7C10Lresh.KpnIbiotin | 5'-TGCAATGGTACCTGCAGAAGC | (SEQ ID No. 108) |
| 7C10Lresh.20anti | 5'-AGACTGCCCTGGCTTCTGCAGGTACCATTGCA | (SEQ ID No. 109) |
| 7C10Lresh.21anti | 5'-CGATTAGAAACTTTATAGATCAGGAGCTGTGG | (SEQ ID No. 110) |
| 7C10Lresh.22anti | 5'-TGCCACTGAACCTGTCAGGGACCCCATAAAGC | (SEQ ID No. 111) |
| 7C10Lresh.23anti | 5'-GATTTTCAGTGTAAAATCTGTGCCTGATCCAC | (SEQ ID No. 112) |
| 7C10Lresh.24anti | 5'-TAAACCCCAACATCCTCAGCCTCCACTCTGCT | (SEQ ID No. 113) |
| 7C10Lresh.25anti | 5'-TCCACGGAACATGTGAACCTTGAAAGCAGTAA | (SEQ ID No. 114) |
| 7C10Lresh.26anti | 5'-TTTGATTTCCACCTTGGTCCCTTGGCCGAAC | (SEQ ID No. 115) |
| 7C10Lresh.BamHIanti sens | 5'-CGCAGAGGATCCACTCACG | (SEQ ID No. 116) |

**TABLEAU 8 : Séquence d'ADN des oligonucléotides ayant été utilisés pour la construction de 7C10 VH humanisée 1 par assemblage de novo**

| | | |
|---|---|---|
| LeaderMluI.biotir | 5'-GTCAGAACGCGTGCCGCC | (SEQ ID No. 117) |
| 7C10Hresh.1sens | 5'-ACCATGAAAGTGTTGAGTCTGTTGTACCTCTTGA | (SEQ ID No. 118) |
| 7C10Hresh.2sens | 5'-CAGCCATTCCTGGTATCCTGTCTCAGGTGCAGCT | (SEQ ID No. 119) |
| 7C10Hresh.3sens | 5'-TCAGGAGTCGGGCCCAGGACTGGTGAAGCCTTCG | (SEQ ID No. 120) |
| 7C10Hresh.4sens | 5'-GAGACCCTGTCCCTCACCTGCACTGTCTCTGGT | (SEQ ID No. 121) |
| 7C10Hresh.5sens | 5'-TACTCCATCACCGGTGGTTATTTATGGAACTGG | (SEQ ID No. 122) |
| 7C10Hresh.6sens | 5'-ATACGGCAGCCCCCAGGGAAGGGACTGGAGTGG | (SEQ ID No. 123) |
| 7C10Hresh.7sens | 5'-ATGGGGTATATCAGCTACGACGGTACCAATAAC | (SEQ ID No. 124) |
| 7C10Hresh.8antisens | 5'-TCAACACTTTCATGGTGGCGGCACGCGTTCTGAC | (SEQ ID No. 125) |
| 7C10Hresh.9antisens | 5'-ATACCAGGAATGGCTGTCAAGAGGTACAACAGAC | (SEQ ID No. 126) |
| 7C10Href.10antisens | 5'-TGGGCCCGACTCCTGAAGCTGCACCTGAGACAGG | (SEQ ID No. 127) |
| 7C10Hresh.11antisens | 5'-TGAGGGACAGGGTCTCCGAAGGCTTCACCAGTCC | (SEQ ID No. 128) |
| 7C10Hresh.12antisens | 5'-CCACCGGTGATGGAGTAACCAGAGACAGTGCAGG | (SEQ ID No. 129) |
| 7C10Hresh.13antisens | 5'-CCCTGGGGGCTGCCGTATCCAGTTCCATAAATAA | (SEQ ID No. 130) |
| 7C10Hresh.14antisens | 5'-TAGCTGATATACCCCATCCACTCCAGTCCCTT | (SEQ ID No. 131) |
| 7C10Hresh.KpnIREV | 5'-GTTATTGGTACCGTCG | (SEQ ID No. 132) |
| 7C10Hresh.KpnIbiotin | 5'-TACGACGGTACCAATAACTAC | (SEQ ID No. 133) |
| 7C10Hresh.15sens | 5'-AAACCCTCCCTCAAGGATCGAATCACCATATC | (SEQ ID No. 134) |
| 7C10Hresh.16sens | 5'-ACGTGACACGTCCAAGAACCAGTTCTCCCTGA | (SEQ ID No. 135) |
| 7C10Hresh.17sens | 5'-AGCTGAGCTCTGTGACCGCTGCGGACACTGCA | (SEQ ID No. 136) |
| 7C10Hresh.18sens | 5'-GTGTATTACTGTGCGAGATACGGTAGGGTCTT | (SEQ ID No. 137) |
| 7C10Hresh.19sens | 5'-CTTTGACTACTGGGGCCAGGGAACCCTGGTCA | (SEQ ID No. 138) |
| 7C10Hresh.20sens | 5'-CCGTCTCCTCAGGTGAGTGGATCCTCTGCG | (SEQ ID No. 139) |
| 7C10Hresh.21antisens | 5'-AGGGAGGGTTTGTAGTTATTGGTACCGTCGTA | (SEQ ID No. 140) |
| 7C10Hresh.22antisens | 5'-ACGTGTCACGTGATATGGTGATTCGATCCTTG | (SEQ ID No. 141) |
| 7C10Hresh.23antisens | 5'-AGAGCTCAGCTTCAGGGAGAACTGGTTCTTGG | (SEQ ID No. 142) |
| 7C10Hresh.24antisens | 5'-CAGTAATACACTGCAGTGTCCGCAGCGGTCAC | (SEQ ID No. 143) |
| 7C10Hresh.25antisens | 5'-AGTAGTCAAAGAAGACCCTACCGTATCTCGCA | (SEQ ID No. 144) |
| 7C10Hresh.26antisens | 5'-CTGAGGAGACGGTGACCAGGGTTCCCTGGCCCC | (SEQ ID No. 145) |
| 7C10Hresh.BamHIantisens | 5'-CGCAGAGGATCCACTCAC | (SEQ ID No. 146) |

**TABLEAU 9 : Protocole d'appariement des oligonucléotides pour l'assemblage de novo des gènes codant pour les formes humanisées de 7C10 VH et VL**

| Assemblage *de novo* du fragment | Assemblage *de novo* du fragment |
|---|---|
| MluI - KpnI de 7C10 VL humanisée 1 | KpnI - BamHI de 7C10 VL humanisée 1 |
| | |
| Oligo leader MluI 7C10 VL Biotinylé | Oligo 7C10 L KpnI Biotinylé |
| Couple d'oligo 1 et 7 | Couple d'oligo 13 et 20 |
| Couple d'oligo 2 et 8 | Couple d'oligo 14 et 21 |
| Couple d'oligo 3 et 9 | Couple d'oligo 15 et 22 |
| Couple d'oligo 4 et 10 | Couple d'oligo 16 et 23 |
| Couple d'oligo 5 et 11 | Couple d'oligo 17 et 24 |
| Couple d'oligo 6 et 12 | Couple d'oligo 18 et 25 |
| Oligo antisens 7C10 VL KpnI | Couple d'oligo 19 et 26 |
| | Oligo antisens 7C10 L BamHI |
| | |

| Assemblage *de novo* du fragment | Assemblage *de novo* du fragments |
|---|---|
| MluI - KpnI de 7C10 VH humanisée 1 | KpnI - BamHI de 7C10 VH humanisée 1 |
| | |
| Oligo leader MluI 7C10 VH Biotinylé | Oligo 7C10 H KpnI Biotinylé |
| Couple d'oligo 1 et 8 | Couple d'oligo 15 et 21 |
| Couple d'oligo 2 et 9 | Couple d'oligo 16 et 22 |
| Couple d'oligo 3 et 10 | Couple d'oligo 17 et 23 |
| Couple d'oligo 4 et 11 | Couple d'oligo 18 et 24 |
| Couple d'oligo 5 et 12 | Couple d'oligo 19 et 25 |
| Couple d'oligo 6 et 13 | Couple d'oligo 20 et 26 |
| Couple d'oligo 7 et 14 | Oligo antisens 7C10 VH BamHI |
| Oligo antisens 7C10 VH KpnI | |

### Exemple 15. Construction des gènes codant pour les versions humanisées 2 de 7C10 VL et 7C10 VH et 3 de 7C10 VH par mutagenèse dirigée.

La version humanisée 2 de 7C10 VH a été obtenue par mutation dirigée des résidus 48 et 67 (selon la nomenclature de Kabat) de la version 1. Cette mutagenèse dirigée a été réalisée à l'aide du système QuikChange™ Site-directed mutagenesis de Stratagene (kit #200518) selon le protocole décrit par le fabricant. La construction s'est faite en deux temps, dans un premier temps le résidu 48 sur la version 1 a été muté à l'aide du couple d'amorces 7C10Hhumanisé1QCM48 sens et antisens (voir Tableau 10) et dans un deuxième temps cette version mutée au résidu 48 a elle-même été mutée au résidu 67 à l'aide du couple d'amorces 7C10Hhumanisé1QCI67 sens et antisens (voir Tableau 10).

La version humanisée 3 de 7C10 VH a été obtenue par mutation dirigée des résidus 30 et 71 (selon la nomenclature de Kabat) de la version 2 en utilisant également le système QuikChange™. Cette construction s'est faite en deux temps. Dans un premier temps, le résidu 30 sur la version 2 a été muté à l'aide des amorces 7C10HhumaniséQCT30 sens et antisens (voir Tableau 10). Dans un deuxième temps, cette version mutée au résidu 30 a elle-même été mutée au résidu 71 en utilisant le couple d'amorces 7C10Hhumanisé1V67QCR71 sens et antisens (voir Tableau 10).

La version humanisée 2 de 7C10 VL a été obtenue par mutation dirigée du résidu 2 (selon la nomenclature de Kabat) de la version 1 en utilisant le système QuikChange™. Le résidu 2 sur la version 1 a été muté en utilisant le couple d'amorces 7C10L humanisélQCV2 sens et antisens (voir Tableau 10).

**TABLEAU 10 : Liste des oligonucléotides utilisés pour la mutagenèse dirigée par le système QuikChange™ de stratagène**

| | | |
|---|---|---|
| 7C10Hhumanisé1QCT30. sens | 5'-CTGGTTACTCCATCAGCGGTGGTTATTTATG | (SEQ ID No. 147) |
| 7C10Hhumanisé1QCT30. antisens | 5'-CATAAATAACCACCGCTGATGGAGTAACCAG | (SEQ ID No. 148) |
| 7C10Hhumanisé1QCM48. sens | 5'-GGGACTGGAGTGGATCGGGTATATCAGCTAC | (SEQ ID No. 149) |
| 7C10Hhumanisé1QCM98. antisens | 5'-GTAGCTGATATACCCGATCCACTCCAGTCCC | (SEQ ID No. 150) |
| 7C10Hhumanisé1QCI67. sens | 5'-TCCCTCAAGGATCGAGTCACCATATCACGTG | (SEQ ID No. 151) |
| 7C10Hhumanisé1QCI67. antisens | 5'-CACGTGATATGGTGACTCGATCCTTGAGGGA | (SEQ ID No. 152) |
| 7C10Hhumanisé1V67QCR71. sens | | (SEQ ID No. 153) |
| 7C10Hhumanisé1V67QCR71. antisens | | (SEQ ID No. 154) |
| 7C10Lhumanisé1QCV2. sens | 5'-GCTTCCAGCAGTGATATTGTGATGACTCAGT | (SEQ ID No. 155) |
| 7C10Lhumanisé1QCV2. antisens | 5'-ACTGAGTCATCACAATATCACTGCTGGAAGC | (SEQ ID No. 156) |

### Exemple 16. Transfection des cellules cos7 par électroporation

Les vecteurs d'expression mammalienne contenant les versions chimériques ou humanisées des chaînes lourdes et légères de l'anticorps 7C10 ont été testés en cellules cos7 pour l'expression transitoire des anticorps recombinants 7C10. L'ADN a été introduit dans les cellules cos par électroporation à l'aide d'un instrument Biorad (Gene Pulsar). L'ADN (10 µg de chaque vecteur) est ajouté à des aliquotes de 0,8 ml de cellules cos à une concentration de 1 x 10⁷ cellules par ml dans du tampon PBS (sans Ca++ et Mg++). Une pulsation de 1,900 volts et d'une capacité de 25 µF a été délivrée. Les cellules cos transfectées sont alors ajoutées à 8 ml de milieu DMEM contenant 5 % de sérum de veau et incubées à 37°C pendant 72 heures. Le surnageant est alors récolté, centrifugé pour éliminer les débris cellulaires et testé par ELISA pour la mesure de sa concentration en anticorps 7C10 recombinant de type IgG1/Kappa humaine.

### Exemple 17. Méthode ELISA pour mesurer les concentrations en anticorps recombinants IgG1/Kappa humain présentes dans le surnageant des transfectants cos

Les surnageants produits par expression transitoire en cellules cos7 ont été testés pour la présence d'anticorps 7C10 de type IgG1/Kappa humaine. Pour la détection de l'immunoglobuline IgG1/Kappa humaine, des plaques ELISA de 96 puits (Maxisorb, Nunc) ont été cotées avec un anticorps polyclonal de chèvre anti-IgG humaine (spécifique pour le fragment Fc gamma, Jackson Immuno-Research Laboratories Inc., #109-005-098). Les surnageants de cellules cos ont été dilués en série et ajoutés aux puits cotés. Après une incubation d'une heure à 37°C et lavage, un anticorps polyclonal de chèvre anti-chaîne légère Kappa humaine conjugué à péroxidase (HRP, Sigma, A-7164) a été ajouté. Après 45 minutes d'incubation à 37°C et lavage, le substrat TMB (KPL #50-76-04) a été ajouté. Après 10 minutes d'incubation la réaction fut stoppée par l'addition d'acide sulfurique 1M et la densité optique lue à 450 nm. Une immunoglobuline humaine IgG1/Kappa humaine purifiée (Sigma, I-3889) de concentration connue a été utilisée comme anticorps de référence standard.

### Exemple 18. Méthode ELISA pour déterminer l'activité de reconnaissance des anticorps recombinants 7C10 de type IgG1/Kappa humains sur le récepteur à l'IGF-1 (IGF-IR)

Les surnageants de culture cos7 ont été testés pour leur capacité à reconnaître l'IGF-1 R par une méthode ELISA. Des plaques ELISA de 96 puits (Dynex Immulon 2HB) ont été cotées avec 100 µl par puit d'une solution de PBS contenant 0,31 ng/µl d'IGF-1 R (Human Insulin-like Growth Factor I soluble Receptor, R & D Systems, #391-GR) par incubation pour une nuit à 4°C. Après lavage avec du PBS contenant 0,05 % Tween 20, les plaques ont été saturées par l'addition d'une solution de PBS contenant 0,5 % Gélatine et incubation à 37°C pendant 1 heure. Après 3 lavages avec du PBS, les échantillons de surnageants cos à tester, préalablement dilués en série dans du PBS contenant 0,1 % Gélatine et 0,05 % Tween 20, ont été ajoutés aux plaques. Après une incubation à 37°C pendant 1 heure suivie de 3 lavages (PBS contenant 0,05 % Tween 20), un anticorps anti-IgG humaine (spécifique pour le fragment Fc) conjugué à la péroxidase (HRP, Jackson Immuno-Research Laboratories Inc., #109-035-098) a été ajouté (dilution au 1/5000 dans du PBS contenant 0,1 % Gélatine et 0,05 % Tween 20). Après 45 minutes d'incubation à 37°C et <<3 lavages (PBS contenant 0,05 % Tween 20), le substrat TMB (KPL #50-76-04) a été ajouté. Après 10 minutes d'incubation la réaction fut stoppée par l'addition d'acide sulfurique 1M et la densité optique lue à 450 nm.

### Exemple 19. Détermination de l'activité de reconnaissance de l'IGF1-R par les différentes versions de l'anticorps 7C10 humanisé par « CDR-grafting »

Dans un premier temps, nous avons comparé l'activité de reconnaissance des formes humanisées 1 des chaînes lourde et légère de 7C10 pour l'IGF-1 récepteur par rapport à la forme chimérique. La figure 28 montre les résultats d'un test ELISA de reconnaissance de l'IGF-1R (voir Exemple 18) à partir de surnageants de cellules cos7 dont la concentration en IgG1/Kappa humaine avait été préalablement déterminée par ELISA (voir Exemple 17). Les courbes de titration des quatre anticorps recombinants testés se chevauchent parfaitement indiquant que leurs affinités relatives pour l'IGF-IR sont très similaires. On en conclut donc que la forme humanisée 1 de 7C10, composée de la chaîne légère humanisée 1 (1 seul résidu de souris présent dans les régions charpentes) en combinaison avec la chaîne lourde humanisée 1 (4 résidus de souris présents dans les régions charpentes), reconnaît spécifiquement l'IGF-1 récepteur et a une affinité très similaire à celle de l'anticorps chimérique (régions variables de souris).

Dans un deuxième temps nous avons regardé l'influence du résidu 2 (selon la nomenclature de Kabat) de la chaîne légère humanisée de 7C10 (version humanisée 1 versus humanisée 2, voir figure 19) sur la reconnaissance de l'IGF-IR. La figure 29 montre les résultats du test ELISA de reconnaissance de l'IGF-IR (voir Exemple 18) à partir de surnageants de cellules cos7 dont la concentration en IgG1/Kappa humaine avait été préalablement déterminée par ELISA (voir Exemple 17). Les deux versions humanisées 1 et 2 de la chaîne légère ont été associées successivement avec 7C10 VH humanisée 1. Les courbes de titration des deux combinaisons se superposent indiquant que la mutation du résidu 2 de la chaîne légère, qui a été changé d'une valine dans la version humanisée 1 pour une isoleucine dans la forme humanisée 2, n'a apparemment aucune influence sur l'affinité relative de reconnaissance de l'IGF1 récepteur. La forme humanisée 2 de la chaîne légère de 7C10 constitue ainsi une version ou aucun résidu de souris (hors CDRs) n'a été conservé. Cette version, totalement humanisée, représente la version préférée de 7C10 VL.

La version totalement humanisée de la chaîne légère 7C10 (version humanisée 2, voir ci-dessus) a été testée en association avec les trois versions humanisées de la chaîne lourde de 7C10. La figure 30 montre les résultats du test ELISA de reconnaissance de l'IGF-1R à partir de surnageants de cellules cos7 dont la concentration en IgG1/Kappa humaine avait été préalablement déterminée par ELISA (voir Exemple 17). Les courbes de titration sont très similaires et se chevauchent pratiquement avec la courbe référence correspondant à l'anticorps chimérique, indiquant que les trois versions humanisées 1, 2 et 3 de 7C10 VH donnent une même affinité relative pour l'IGF-1R quand elles sont associées à 7C10 VL humanisée 2. D'autres tests ELISA menés en parallèle (résultats non montrés) ont toutefois révélé qu'une mutation ponctuelle du résidu 71 (nomenclature de Kabat) d'une arginine (souris) à une valine (humain) entraînait une petite perte d'affinité de l'anticorps correspondant pour l'IGF-1R. Il est ainsi raisonnable de penser que 7C10 VH humanisée 2 a la même affinité relative pour l'IGF-1R que 7C10 VH humanisée 1. Cette forme humanisée 2 sera donc préférée par rapport à la forme 1 puisqu'elle ne comporte que deux acides aminés de souris (résidus 30 et 71, voir figure 24). La forme humanisée 3 qui ne comporte plus aucun résidu de souris (en dehors des CDRs) sera elle aussi préférée puisqu'elle ne semble entraîner qu'une perte minimale d'affinité.

En conclusion, il apparaît que deux formes humanisées de l'anticorps 7C10 selon la présente invention sont particulièrement préférées. Une forme constituée de l'association de 7C10 VH humanisée 2 (2 résidus de souris conservés) avec 7C10 VL humanisée 2 (aucun résidu de souris conservé) et une autre forme constituée de l'association de 7C10 VH humanisée 3 (aucun résidu de souris conservé) avec 7C10 VL humanisée 2 (aucun résidu de souris conservé). Cette dernière forme constitue la version humanisée ultime puisqu'aucun résidu de souris n'est présent à la fois dans les chaînes lourde et légère.

### Exemple 20. Expression de l'EGFR et de l'IGF-IR à la surface des cellules A549

La synergie d'action obtenue par la co-administration de deux ACM respectivement dirigés contre l'IGF-IR et l'EGFR a été étudiée chez des souris nudes porteuses d'une tumeur du poumon non à petites cellules établie par injection sous cutanée (s.c.) de cellules A549 (lignée cellulaire de carcinome de poumon).

Dans un premier temps, et afin de s'assurer de la présence des deux récepteurs IGF-IR et EGFR à la surface de la cellule A549 avant d'injecter celle-ci à la souris, un marquage pour lecture FACS de ces cellules a été réalisé avec respectivement l'ACM murin 7C10 anti-IGF-IR (figure 37B) et l'ACM murin 225 anti-EGFR (figure 37D). Pour ce faire, les cellules ont été saturées 30 min à 4°C avec une solution de PBS 10 % SVF (Sérum de veau foetal), lavées puis incubées 30 min à 4°C avec l'ACM d'intérêt. Après 3 nouveaux lavages, l'anticorps secondaire anti-espèce couplé au FITC (isothiocyanate de fluoroscéïne) est ajouté. Après 30 min d'incubation la lecture au FACS (« Fluorescence Activated Cells Sorter ») est effectuée à 520 nm (excitation 488 nm).

Les résultats présentés dans les figures 37A à 37D montrent que les cellules A549 présentent à leur surface un nombre comparable de récepteurs à l'EGF et à l'IGF1. Dans les deux cas la population est homogène par rapport à la distribution de chacun des récepteurs. La spécificité du marquage est confirmée par l'utilisation d'un contrôle isotypique (figure 37C). Ces résultats valident l'utilisation de la cellule A549 comme modèle pour l'étude d'une synergie d'action sur deux récepteurs IGF-IR et EGFR et pour l'étude d'une collaboration de ces deux récepteurs.

### Exemple 21. Synergie d'action d'un ACM anti-IGF-IR et d'un ACM anti-EGFR coadministrés in vivo, chez la souris nude dans le cadre d'un traitement antitumoral.

Pour cette étude, des souris nudes sont greffées en s.c. avec 5.10⁶ cellules A549. Cinq jours après la greffe de cellules, les tumeurs sont mesurées et un lot homogène de souris en terme de volume tumoral est constitué. A partir de ce lot des groupes de 6 souris sont générés au hasard. Ces souris seront traitées en intra péritonéal (i.p.), 2 fois par semaine avec chacun des ACM 7C10 et 225 individuellement à la dose de 250 µg/souris ou avec les deux ACM en co-administration. L'ACM 9G4 est administré comme contrôle isotypique d'expérience.

Les résultats présentés dans la figure 38 montrent que chacun des anticorps 7C10 et 225 administrés seuls est capable d'induire une diminution significative de la croissance tumorale *in vivo.* On peut noter que les deux ACM testés ont une activité comparable sur la croissance de la tumeur A549. De façon surprenante par rapport à la littérature, une synergie significative est observée lors de l'administration simultanée des deux ACM (p < ou = à 0.01 à chacun des temps de la cinétique dans un test t) suggérant qu'il existe une collaboration des deux récepteurs pour la croissance optimale d'une tumeur *in vivo* et que, contrairement aux données de la littérature, le blocage de l'un des deux axes ne suffit pas à inhiber totalement la croissance médiée par le second.

### Exemple 22. Etude de l'activité antitumorale des anticorps murins 7C10 et 225 coadministrés chez des souris implantées en orthotopique avec des cellules A549.

L'utilisation de modèles orthotopiques pour l'évaluation de l'activité antitumorale présente un intérêt particulier par rapport au processus de dissémination métastatique d'une tumeur. Afin d'évaluer l'activité antitumorale d'un mélange d'anticorps dirigés respectivement contre l'IGF-IR et l'EGFR, 10⁶ cellules A549 (cancer du poumon non à petites cellules) ont été implantées dans la cavité intra-pleurale de souris Nude. Il est à noter que ce type d'implantation tumorale a pour conséquence une dissémination métastatique semblable à celle observée chez l'homme et aboutit à la mort des animaux. La figure 39 montre que l'administration des anticorps 225 et 7C10 seuls permet d'observer un gain comparable et significatif de survie. De façon surprenante, la co-administration de ces deux anticorps augmente de façon considérable la survie des animaux suggérant que ce traitement ait un impact sur la dissémination métastatique des cellules tumorales.

### Exemple 23. 7C10 et 7H2HM inhibe la phosphorylation de la tyrosine de la chaîne β de l'IGF-IR et de l'IRS-I

Des cellules MCF7 sont mises en culture pendant 24 heures à 5.10⁴ cellules/cm² (boîtes de 75 cm², COSTAR) dans 20 ml de RPMI sans rouge de phénol additionné de 5 mM de glutamine, pénicilline/streptomycine (respectivement 100 U / 100 µg/ml) et 10 % de sérum de veau foetal. Après trois lavages en PBS, les cellules ont été incubées pendant 12 heures en milieu (RPMI) sans rouge de phénol, dépourvu de sérum de veau foetal et additionné de 5 mM de glutamine, pénicilline/streptomycine, sérumalbumine bovine à 0,5 µg/ml (Sigma A-8022) et transferrine à 5 µg/ml (Sigma T8158).

Pour l'activation, les cellules ont tout d'abord été incubées à 37°C pendant 2 minutes avec des anticorps bloquant (10 µg/ml) puis l'IGF-I (Sigma 13769, 50 ng/ml) a été ajouté pour deux minutes supplémentaires. La réaction a été stoppée par aspiration du milieu d'incubation et les boites ont été déposées sur de la glace. Les cellules ont été solubilisées par addition de 0,5 ml de tampon de lyse (50 mM tris-HCL pH 7,5, 150 mM NaCl, 1 % Nonidet P40, 0,5 % sodium déoxycholate), additionné d'inhibiteurs de protéase (1 comprimé pour 50 ml, Boehringer Réf.: 1697 498), et d'inhibiteurs de phosphatase (Calbiochem Réf.: 524625 (1/100)). Les cellules ont été raclées et la suspension a été récupérée et placée sur un agitateur à 4°C pendant 1,5 heures. Les solutions ont été centrifugées à 12000 rpm pendant 10 minutes (4°C) et les concentrations en protéines des surnageants ont été quantifiées par BCA.

500 µg de protéines du lysat cellulaire ont été mélangées avec l'anti-IGF-IR (Santa cruz Réf.: sc-713) pour immunoprécipitation et incubées sur agitateur à 4°C pendant 1,5 heures. Les immunoprécipités ont été récupérés par addition de protéine A-agarose (Boehringer Réf.: 1 134 515) et incubés toute la nuit sur agitateur à 4°C. Pour l'immunoprécipitation d'IRS-1, des anticorps anti-IRS-1 couplés à des billes d'agarose (Santa-cruz Réf.: 559Ac) ont été utilisés. Les billes d'agarose ont été lavées deux fois avec 1 ml de tampon de lyse, deux fois avec un tampon de lavage 1 (50 mM Tris-HCL pH 7,5 ; 500 mM NaCl ; 0,1 % Nonidet P40 ; 0,05 % sodium deoxycholate (Boehringer 1 332 597), additionné d'inhibiteurs de protéase et d'inhibiteurs de phosphatase) et une fois avec un tampon de lavage 2 (50 mM Tris-HCL ; 0,1 % Nonidet P40 ; 0,05 % sodium deoxycholate (Boehringer Réf.: 1 332 597), additionné d'inhibiteurs de protéase et d'inhibiteurs de phosphatase 1/100). Les immunoprécipités ont été remis en suspension dans du tampon Laemmli, chauffés à 100°C pendant 5 minutes. Les surnageants ont été analysés par électrophorèse sur gel SDS de polyacrylamide (8 % Novex EC6015). Les protéines ont été transférées sur membrane de nitrocellulose suivi par soit un immunoblot avec des anticorps anti-phosphotyrosine conjugués à HRP (upstate Biotechnology 4G10) ou anti-chaîne béta de l'IGF-IR ou anti-IRS-1 (Santa Cruz Réf.: sc 8038) suivi par un anticorps anti-lapin conjugué à HRP. Les empreintes ont été révélées par chemiluminiscence (Amersham RPN 2209) suivi d'une radioautographie sur films Kodak X-mat AR.

La figure 40A représente des cellules MCF7 non stimulées (0) ou stimulées soit avec IGF-1 (50 ng/ml) seul (0+IGF-1) ou combiné avec des anticorps monoclonaux ou humanisés anti-IGF-IR (10 µg/ml) 7C10, 1H7, 7H2HM. Les anticorps 9G4 ou hIgG1 sont des immunoglobulines murines ou humaines d'isotype IgG1 utilisées comme contrôle négatif de l'expérience. Les chaînes béta de l'IGF-IR ont été immunoprécipitées et blottées avec des anticorps anti-tyrosine phosphorylée. Les résultats obtenus montrent que les anticorps monoclonaux ou humanisés anti-IGF-IR 7C10, 1H7 et 7H2HM inhibent la phosphorylation de la tyrosine de la chaîne béta de l'IGF-IR.

La figure 40B représente des cellules MCF7 non stimulées (0) ou stimulées soit avec IGF-1 (50 ng/ml) seul (0+IGF-1) ou combiné avec des anticorps monoclonaux ou humanisés anti-IGF-IR (10 µg/ml) 7C10, 1H7, 7H2HM. Comme décrit plus haut, les anticorps 9G4 ou hIgG1 sont des immunoglobulines murines ou humaines d'isotype IgG1 utilisées comme contrôle négatif de l'expérience. L'IRS-1 a été immunoprécipité et blotté avec des anticorps anti-tyrosine phosphorylée. Les résultats obtenus montrent que les anticorps monoclonaux 7C10, 7H2HM et 1H7 inhibent la phosphorylation de la tyrosine de l'IRS-1.

### Exemple 24. 7C10 et 7H2HM induit l'internalisation de l'IGF-IR

Des cellules MCF7 et A549 ont été mises en suspension à 1.10⁷ cellules/ml dans du PBS avec 10 % de sérum de veau foetal (tampon FACS). 1.10⁶ cellules ont été incubées 30 minutes à 37°C avec les anticorps monoclonaux à 10 µg/ml (7C10, 7G3, 9G4) ou à 20 µg/ml pour 7H2HM. Après lavage, les cellules ont été marquées à 4°C pendant 30 minutes avec un anti-IGF-IR biotynilé (anticorps monoclonal 12B1) et enfin incubées à 4°C pendant 30 minutes avec un conjugué de streptavidine-488 alexa Fluor^{®}. Les cellules ont été analysées par FACScan (Becton-Dickinson, Enembogegem, Belgique) avec le logiciel Cellquest après élimination des débris.

La figure 41 montre les cellules A549 sans coloration (1^{er} pic), les cellules A549 incubées avec 7C10 ou 7H2HM (2^{ème} pic) et les cellules A549 incubées avec une IgG1 de souris ou de rat irrelevante (3^{ème} pic). On observe une diminution par deux de l'expression de surface de l'IGF-IR par les cellules lorsque les cellules ont été préalablement incubées avec 7C10 ou 7H2HM.

### Exemple 25. 7C10 et 7H2HM induit la dégradation de l'IGF-IR

Des cellules MCF-7 ont été cultivées pendant 24 heures à 10.10⁴ cellules/cm² (75 cm², Costar) dans 15 ml de milieu complet. Ensuite, les cultures ont été lavées trois fois avec du PBS et incubées pendant 12 heures avec du milieu dépourvu de sérum. Ensuite, les cellules ont été incubées avec de la cycloheximide à 25 µg/ml seul ou avec 10 µg/ml d'anticorps monoclonal 7C10, 9G4, 7G3 ou de l'IGF-I (50 ng/ml). Dans certaines expériences, avant incubation avec les anticorps monoclonaux, les cellules ont été traitées pendant 1 heure à 37°C avec du MG-132 (10 µM, Calbiochem 474791) pour inhiber les activités du protéasome. Après l'incubation, les cellules ont été lavées et solubilisées par addition d'un tampon de lyse. 20 µg de protéines ont été analysées par électrophorèse sur gel de polyacrilamide à 8 % de SDS et transférées sur une membrane de nitrocellulose suivie par un immunoblot anti chaîne béta de l'IGF-IR tel que décrit plus haut.

L'analyse par westem-blot (figure 42A) de l'intégrité de l'IGF-IR montre que 7C10 et 7H2HM induisent la dégradation du récepteur alors que le ligand naturel n'entraîne aucune dégradation de ce dernier. Aucune dégradation du récepteur n'est observée avec le 9G4, anticorps irrelevant utilisé comme contrôle isotypique. La figure 42B met, quant à elle, en évidence que la dégradation est inhibée par un inhibiteur de protéasome MG132 (période d'incubation de 2 heures).

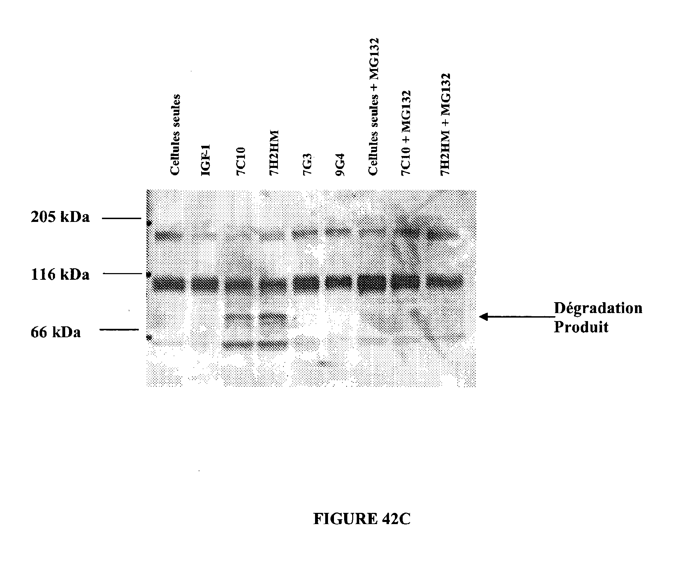

Des résultats comparables ont été obtenus avec l'anticorps humanisé 7H2HM (figure 42C).

## Revendications

1. Utilisation d'un anticorps isolé, ou l'un de ses fragments fonctionnels capable de se lier au récepteur humain du facteur de croissance 1 apparenté à l'insuline IGF-IR, ledit anticorps comprenant une chaîne lourde comprenant les trois CDRs de séquence SEQ ID Nos. 8, 10 et 12, et une chaîne légère comprenant les trois CDRs de séquence SEQ ID Nos. 2, 4 et 6, pour la préparation d'une composition destinée à la prévention ou au traitement du psoriasis.

2. Utilisation selon la revendication 1, **caractérisée en ce que** ledit fragment fonctionnel est choisi parmi les fragments Fv, Fab, (Fab,')₂, Fab', scFv, scFv-Fc et les diabodies, ou une de leurs formes pégylées.

3. Utilisation selon l'une quelconque des revendications précédentes, **caractérisée en ce que** ledit anticorps, ou l'un de ses fragments fonctionnels, est sécrété par l'hybridome murin déposé à la CNCM, Institut Pasteur, Paris, le 19 septembre 2001 sous le numéro I-2717.

4. Utilisation selon l'une quelconque des revendications précédentes, **caractérisée en ce que** ledit anticorps, ou l'un de ses fragments fonctionnels, comprend une chaîne légère de séquence comprenant la séquence d'acide aminé SEQ ID No. 54, et/ou **en ce qu'**il comprend une chaîne lourde de séquence comprenant la séquence d'acide aminé SEQ ID No. 69.

5. Utilisation selon l'une quelconque des revendications précédentes, **caractérisée en ce que** ledit anticorps, ou l'un de ses fragments fonctionnels, est un anticorps chimérique et comprend en outre les régions constantes de chaîne légère et de chaîne lourde dérivées d'un anticorps d'une espèce hétérologue à la souris.

6. Utilisation selon la revendication 5, **caractérisée en ce que** ladite espèce hétérologue est l'Homme.

7. Utilisation selon la revendication 6, **caractérisée en ce que** les régions constantes de chaîne légère et de chaîne lourde dérivées d'un anticorps humain sont respectivement la région kappa et, gamma-1, gamma-2 ou gamma-4.

8. Utilisation selon l'une des revendications 6 ou 7, **caractérisée en ce que** ledit anticorps, ou l'un de ses fragments fonctionnels, comprend une chaîne légère et/ou une chaîne lourde dans lesquelles les segments de squelette FR1 à FR4 de ladite chaîne légère et/ou chaîne lourde sont dérivés respectivement de segments de squelette FR 1 à FR4 de chaîne légère et/ou de chaîne lourde d'anticorps humains.

9. Utilisation selon l'une des revendications 6 à 8, **caractérisée en ce que** ledit anticorps, ou l'un de ses fragments fonctionnels, comprend une chaîne légère comprenant la séquence d'acide aminé SEQ ID No. 61 ou 65, et/ou **en ce qu'**il comprend une chaîne lourde comprenant la séquence d'acide aminé SEQ ID No. 75, 79 ou 83.

10. Utilisation selon la revendication 9, **caractérisée en ce que** ledit anticorps, ou l'un de ses fragments fonctionnels, comprend une chaîne légère comprenant la séquence d'acide aminé SEQ ID No. 65, et **en ce qu'**il comprend une chaîne lourde de séquence comprenant la séquence d'acide aminé SEQ ID No. 83.

11. Utilisation selon l'une quelconque des revendications précédentes, **caractérisée en ce que** ladite composition comprend un deuxième composé choisi parmi les composés capables d'inhiber spécifiquement la fixation de l'EGF sur le récepteur humain du facteur de croissance de l'épiderme EGFR et/ou capable d'inhiber spécifiquement l'activité tyrosine kinase dudit récepteur EGFR.

12. Utilisation selon la revendication 11, **caractérisée en ce que** ledit deuxième composé est choisi parmi les anticorps anti-EGFR isolés, ou leurs fragments fonctionnels, capables d'inhiber par compétition la fixation de l'EGF sur l'EGFR.

13. Utilisation selon la revendication 11 ou 12, **caractérisée en ce que** ledit anticorps anti-EGFR est choisi parmi les anticorps anti-EGFR monoclonaux, chimériques ou humanisés, ou leurs fragments fonctionnels.

14. Utilisation selon l'une quelconque des revendications 12 ou 13, **caractérisée en ce que** lesdits fragments fonctionnels de l'anticorps anti-EGFR sont choisis parmi les fragments Fv, Fab, (Fab')₂, Fab', scFv-Fc et les diabodies, ou une de leurs formes pégylées.

15. Utilisation selon l'une des revendications 12 à 14, **caractérisée en ce que** ledit anticorps anti-EGFR est l'anticorps monoclonal des souris 225, son dérivé chimérique souris-homme C225, ou un anticorps humanisé dérivé de cet anticorps 225.

## Claims

1. Use of an isolated antibody, or one of its functional fragments, capable of binding to the human insulin-like growth factor I receptor IGF-IR, said antibody comprising a heavy chain comprising the three CDRs of sequence SEQ ID Nos 8, 10 and 12, and a light chain comprising the three CDRs of sequence SEQ ID Nos 2, 4 and 6, for the preparation of a composition intended for the prevention or for the treatment of psoriasis.

2. Use according to Claim 1, **characterized in that** said functional fragment is chosen from the fragments Fv, Fab, (Fab')₂, Fab', scFv, scFv-Fc and the diabodies, or one of their pegylated forms.

3. Use according to either one of the preceding claims, **characterized in that** said antibody, or one of its functional fragments, is secreted by the murine hybridoma deposited at the CNCM, Institut Pasteur, Paris, on 19 September 2001 under the number I-2717.

4. Use according to any one of the preceding claims, **characterized in that** said antibody, or one of its functional fragments, comprises a light chain of sequence comprising the amino acid sequence SEQ ID No. 54, and/or **in that** it comprises a heavy chain of sequence comprising the amino acid sequence SEQ ID No. 69.

5. Use according to any one of the preceding claims, **characterized in that** said antibody, or one of its functional fragments, is a chimeric antibody and in addition comprises the light chain and heavy chain constant regions derived from an antibody of a species heterologous to the mouse.

6. Use according to Claim 5, **characterized in that** said heterologous species is man.

7. Use according to Claim 6, **characterized in that** the light chain and heavy chain constant regions derived from a human antibody are respectively the kappa and gamma-1, gamma-2 or gamma-4 region.

8. Use according to either of Claims 6 and 7, **characterized in that** said antibody, or one of its functional fragments, comprises a light chain and/or a heavy chain in which the skeleton segments FR1 to FR4 of said light chain and/or heavy chain are respectively derived from skeleton segments FR1 to FR4 of human antibody light chain and/or heavy chain.

9. Use according to one of Claims 6 to 8, **characterized in that** said antibody, or one of its functional fragments, comprises a light chain comprising the amino acid sequence SEQ ID No. 61 or 65, and/or **in that** it comprises a heavy chain comprising the amino acid sequence SEQ ID No. 75, 79 or 83.

10. Use according to Claim 9, **characterized in that** said antibody, or one of its functional fragments, comprises a light chain comprising the amino acid sequence SEQ ID No. 65, and **in that** it comprises a heavy chain of sequence comprising the amino acid sequence SEQ ID No. 83.

11. Use according to any one of the preceding claims, **characterized in that** said composition comprises a second compound chosen from the compounds capable of specifically inhibiting the binding of EGF to the human epidermal growth factor receptor EGFR and/or capable of specifically inhibiting the tyrosine kinase activity of said EGFR receptor.

12. Use according to Claim 11, **characterized in that** said second compound is chosen from isolated anti-EGFR antibodies, or their functional fragments, capable of competitively inhibiting the binding of EGF to EGFR.

13. Use according to Claim 11 or 12, **characterized in that** said anti-EGFR antibody is chosen from monoclonal, chimeric or humanized anti-EGFR antibodies, or their functional fragments.

14. Use according to either one of Claims 12 and 13, **characterized in that** said functional fragments of the anti-EGFR antibody are chosen from the fragments Fv, Fab, (Fab')₂, Fab', scFv-Fc and the diabodies, or one of their pegylated forms.

15. Use according to one of Claims 12 to 14, **characterized in that** said anti-EGFR antibody is the mouse monoclonal antibody 225, its mouse-human chimeric derivative C225, or a humanized antibody derived from this antibody 225.

## Patentansprüche

1. Verwendung eines isolierten Antikörpers oder von einem von dessen funktionalen Fragmenten, welcher bzw. welches in der Lage ist, sich an den humanen Rezeptor des Insulin-ähnlichen Wachstumsfaktors I IGF-IR zu binden, wobei der Antikörper eine schwere Kette, welche die drei CDRs mit den Sequenzen SEQ ID Nr. 8, 10 und 12 umfasst, und eine leichte Kette, welche die drei CDRs mit den Sequenzen SEQ ID Nr. 2, 4 und 6 umfasst, umfasst, für die Herstellung einer Zusammensetzung, die zur Verhütung oder zur Behandlung von Psoriasis bestimmt ist.

2. Verwendung nach Anspruch 1, **dadurch gekennzeichnet, dass** das funktionale Fragment unter den Fv-, Fab-, (Fab')₂-, Fab'-, scFv-, scFv-Fc-Fragmenten und den Diabodies oder einer von deren pegylierten Formen ausgewählt ist.

3. Verwendung nach einem der vorangegangenen Ansprüche, **dadurch gekennzeichnet, dass** der Antikörper oder das eine von dessen funktionalen Fragmenten durch das Mäuse-Hybridom, welches bei der CNCM, Institut Pasteur, Paris, am 19. September 2001 unter der Nr. I-2717 hinterlegt worden ist, sekretiert wird.

4. Verwendung nach einem der vorangegangenen Ansprüche, **dadurch gekennzeichnet, dass** der Antikörper oder das eine von dessen funktionalen Fragmenten eine leichte Kette mit einer Sequenz, welche die Aminosäuresequenz SEQ ID Nr. 54 umfasst, umfasst und/oder dass er bzw. es eine schwere Kette mit einer Sequenz, welche die Aminosäuresequenz SEQ ID Nr. 69 umfasst, umfasst.

5. Verwendung nach einem der vorangegangenen Ansprüche, **dadurch gekennzeichnet, dass** der Antikörper oder das eine von dessen funktionalen Fragmenten ein chimärer Antikörper ist und außerdem die konstanten Regionen der leichten Kette und der schweren Kette, welche von einem Antikörper von einer zu der Maus heterologen Spezies abgeleitet sind, umfasst.

6. Verwendung nach Anspruch 5, **dadurch gekennzeichnet, dass** die heterologe Spezies der Mensch ist.

7. Verwendung nach Anspruch 6, **dadurch gekennzeichnet, dass** die konstanten Regionen der leichten Kette und der schweren Kette, die von einem humanen Antikörper abgeleitet sind, die Region kappa bzw. gamma-1, gamma-2 oder gamma-4 sind.

8. Verwendung nach einem der Ansprüche 6 oder 7, **dadurch gekennzeichnet, dass** der Antikörper oder das eine von dessen funktionalen Fragmenten eine leichte Kette und/oder eine schwere Kette umfasst, in welchen die Gerüstsegmente FR1 bis FR4 der leichten Kette und/oder schweren Kette jeweils von Gerüstsegmenten FR1 bis FR4 der leichten Kette und/oder schweren Kette von humanen Antikörpern abgeleitet sind.

9. Verwendung nach einem der Ansprüche 6 bis 8, **dadurch gekennzeichnet, dass** der Antikörper oder das eine von dessen funktionalen Fragmenten eine leichte Kette, welche die Aminosäuresequenz SEQ ID Nr. 61 oder 65 umfasst, umfasst und/oder dass er bzw. es eine schwere Kette, welche die Aminosäuresequenz SEQ ID Nr. 75, 79 oder 83 umfasst, umfasst.

10. Verwendung nach Anspruch 9, **dadurch gekennzeichnet, dass** der Antikörper oder das eine von dessen funktionalen Fragmenten eine leichte Kette, welche die Aminosäuresequenz SEQ ID Nr. 65 umfasst, umfasst und dass er bzw. es eine schwere Kette mit einer Sequenz, welche die Aminosäuresequenz SEQ ID Nr. 83 umfasst, umfasst.

11. Verwendung nach einem der vorangegangenen Ansprüche, **dadurch gekennzeichnet, dass** die Zusammensetzung eine zweite Verbindung umfasst, die unter den Verbindungen, die in der Lage sind, spezifisch die Bindung von EGF an den humanen Rezeptor des epidermalen Wachstumsfaktors EGFR zu inhibieren, und/oder in der Lage sind, spezifisch die Tyrosinkinaseaktivität des Rezeptors EGFR zu inhibieren, ausgewählt ist.

12. Verwendung nach Anspruch 11, **dadurch gekennzeichnet, dass** die zweite Verbindung unter den isolierten anti-EGFR-Antikörpern oder deren funktionalen Fragmenten, welche in der Lage sind, die Bindung des EGF an den EGFR durch Kompetition zu inhibieren, ausgewählt ist.

13. Verwendung nach Anspruch 11 oder 12, **dadurch gekennzeichnet, dass** der anti-EGFR-Antikörper unter den monoklonalen, chimären oder humanisierten anti-EGFR-Antikörpem oder deren funktionalen Fragmenten ausgewählt ist.

14. Verwendung nach einem der Ansprüche 12 oder 13, **dadurch gekennzeichnet, dass** die funktionalen Fragmente des anti-EGFR-Antikörpers unter den Fv-, Fab-, (Fab')₂-, Fab'-, scFv-Fc-Fragmenten und den Diabodies oder einer von deren pegylierten Formen ausgewählt sind.

15. Verwendung nach einem der Ansprüche 12 bis 14, **dadurch gekennzeichnet, dass** der anti-EGFR-Antikörper der monoklonale Antikörper 225 von der Maus, dessen chimäres Maus-Mensch-Derivat C225 oder ein von diesem Antikörper 225 abgeleiteter humanisierter Antikörper ist.
